# EUROPEAN PATENT SPECIFICATION

(11) **EP 2 831 236 B1**
(45) Date of publication and mention of the grant of the patent: **10.10.2018**
(21) Application number: 13718611.0
(22) Date of filing: 28.03.2013
(51) Int. Cl.: C12N 15/63

(54) **BACTERIAL EXPRESSION SYSTEM**
BAKTERIELLES EXPRESSIONSSYSTEM
SYSTÈME BACTÉRIEN D'EXPRESSION

(30) Priority: 30.03.2012 GB 201205796
(43) Date of publication of application: 04.02.2015
(73) Proprietor: The University of Nottingham, Nottingham NG7 2RD (GB)
(72) Inventor: MINTON, Nigel, Peter, Nottingham NG7 2RD (GB); ZHANG, Ying, Nottingham NG7 2RD (GB); SCHWARZ, Katrin, Nottingham NG7 2RD (GB); HEAP, John, Nottingham NG7 2RD (GB)
(74) Representative: Barker Brettell LLP
(86) International application number: PCT/GB2013/050836
(87) International publication number: WO 2013/144647

(56) References cited:
- BERTRAM RALPH ET AL: "The application of Tet repressor in prokaryotic gene regulation and expression", MICROBIAL BIOTECHNOLOGY, vol. 1, no. 1, January 2008 (2008-01), pages 2-16, XP002703937, ISSN: 1751-7907
- DUPUY B ET AL: "Clostridium difficile toxin synthesis is negatively regulated by TcdC", JOURNAL OF MEDICAL MICROBIOLOGY, vol. 57, no. 6, June 2008 (2008-06), pages 685-689, XP002703938, ISSN: 0022-2615
- EHRT SABINE ET AL: "Controlling gene expression in mycobacteria with anhydrotetracycline and Tet repressor", NUCLEIC ACIDS RESEARCH, vol. 33, no. 2, 2005, XP002703939, ISSN: 0305-1048
- ZHANG ZHEN ET AL: "Two-Component Signal Transduction System CBO0787/CBO0786 Represses Transcription from Botulinum Neurotoxin Promoters in Clostridium botulinum ATCC 3502", PLOS PATHOGENS, vol. 9, no. 3, March 2013 (2013-03), XP002703941,
- LOVULLO ERIC D ET AL: "TetR-Based Gene Regulation Systems for Francisella tularensis", APPLIED AND ENVIRONMENTAL MICROBIOLOGY, vol. 78, no. 19, October 2012 (2012-10), pages 6883-6889, XP002703940,

## Description

The present invention relates to an expression system for the transcriptional control of the expression of a nucleic acid in a bacterial host. The expression system may be located in the chromosome or localised to an extrachromosomal element/vector or a combination thereof. More specifically, an expression system of the present invention may be used to control the expression of genes involved in anabolism, catabolism, DNA modification or transposition.

The concept of 'orthogonal' expression systems is being explored by researchers in synthetic biology, and potentially offers high level expression as discussed in An and Chin, 2009, Proc Natl Sci USA 106: 8477-82. Orthogonal systems are uncoupled from evolutionary constraints, and selectively abstracted from cellular regulation, making use of heterologous elements, such as phage T7 polymerase. The advantage of using such polymerases resides in the fact that they are specifically targeted to the promoter employed in front of the transgene.

The present invention provides an orthogonal expression system based on the properties of a unique class of sigma factor found in toxinogenic species.

The production of major extracellular toxins by pathogenic strains of *Clostridium botulinum, Clostridium tetani* and *Clostridium difficile,* and a bacteriocin by *Clostridium perfringens,* is dependent on a related group of RNA polymerase sigma-factors assigned to group 5 of the sigma (70) family (Dupuy and Matamouros, 2006, Research Microbiology, 157: 201-205). They recognise highly specific promoter elements which uniquely precede the toxin/bacteriocin genes of these bacteria. No other genes in the genome are known to be under their transcriptional control.

It has been further shown that the group 5 RNA polymerase sigma factors which include BotR, TetR, TcdR and UviA are sufficiently similar that they are interchangeable (Dupuy et al., 2006, Molecular Microbiology, 60(4): 1044-1057; Dupuy and Matamouros, 2006, Research Microbiology, 157: 201-205).

Such functional interchangeability of these sigma factors has been attributed to the strong conservation of the subregion 4.2 sequences and the conserved -35 sequences of their target promoters.

In a first aspect of the invention there is provided a bacterial expression system for expressing a nucleic acid in accordance with claim 1 herein.

In one embodiment, the expression system of the present invention utilises the *Clostridium difficile* TcdR sigma factor (TcdR encoded by *tcdR*) responsible for the expression of the two toxins, TcdA and TcdB, encoded by *tcdA* and *tcdB,* respectively. The *tcdA* and *tcdB* promoters responsible for the expression of the toxin genes *tcdA* and *tcdB* respectively are the only transcriptional signals in the *C*. *difficile* genome recognised by TcdR. However, the expression system of the invention could equally involve the use of homologous sequences derived from *Clostridium botulinum, Clostridium tetani* and *Clostridium perfringens,* or indeed any genes/sigma factors that belong to the group 5 RNA polymerase sigma factor family. That is BotR/*botR* (*C. botulinum*)*,* TetR/*tetR* (*C*. *tetani*) and UviA/u*viA* (*C. perfringens*) are equivalent to TcdR/*tcdR*.

One of the advantages of the expression system of the invention, which uses, for example, the *tcdA* and *tcdB* promoters is that these promoters are poorly recognised by the *E.coli* RNA polymerase (RNP). This allows vectors carrying the promoters to be easily manipulated in *E. coli* without any potential toxic or other growth side effects arising from unwanted expression of the nucleic acid operably linked to the promoter. This can be a problem with using strong vegetative promoters derived from *Clostridium* which are invariably also efficiently recognised by *E. coli's* RNP, for example, the fdx promoter of the ferredoxin gene of *Clostridium sporogenes* (Takamizawa et al., 2004, Protein Expression and Purification 36: 70-75), the promoter of the thiolase gene (*thl)* of *Clostridium acetobutylicum* (Girbal L, et al., 2003, Applied and Environmental Microbiology 69: 4985-4988) and the promoter of the beta-2 toxin gene (cpb2) (Chen Y, et al., 2005, Applied and Environmental Microbiology, 71: 7542-7547) or the *cpe* gene (Chen Y, et al., 2004, Virology. 329: 226-33) of *Clostridium perfringens.*

In a preferred embodiment of the invention, there is provided a bacterial expression system for expressing a nucleic acid comprising:
(a) DNA encoding the TcdR sigma factor; and
(b) an expression cassette comprising a *tcdA* and/or *tcdB* promoter operably linked to a heterologous nucleic acid; wherein (a) and (b) are located on the same expression vector, separate expression vectors or are integrated into the bacterial host genome.

A group 5 RNA polymerase sigma factor may be BotR, TetR, TcdR or UviA. Preferably, the group 5 RNA polymerase sigma factor is BotR. Preferably, the group 5 RNA polymerase sigma factor is TetR. Preferably, the group 5 RNA polymerase sigma factor is TcdR. Preferably, the group 5 RNA polymerase sigma factor is UviA.

The group 5 RNA polymerase sigma factor may have a sequence identity or sequence homology of at least 60%, 65%, 70%, 75%, 80%, 85%, 90%, 95%, 98%, 99% or is identical to one or more of BotR (SEQ ID NO:1), TetR (SEQ ID NO: 2), TcdR (SEQ ID NO: 3) or UviA (SEQ ID NO: 4).

The BotR sigma factor may be from any toxigenic strain of *Clostridium botulinum.* BotR is a positive regulator of the botulism toxin genes, *ntnh* and ha33 (Jacobson, M.J., et al., 2008, Applied and Environmental Microbiology, 74: 2778-2786) and typically comprises 178 amino acids which may comprise the following sequence, SEQ ID NO:1. A typical representative is that of GenBank Accession Number YP_001253340 as defined in SEQ ID NO: 1 below:

MNKLFLQIKMLKNDNREFQEIFKHFEKTINIFTRKYNIYDNYNDILYHLWYTLKKV
DLSNFNTQNDLERYISRTLKRYCLDICNKRKIDKKIIYNSEIVDKKLSLIANSYSSY
LEFEFNDLISILPDDQKKIIYMKFVEDIKEIDIAKKLNISRQSVYKNKIMALERLEPIL
KKLINM

The TetR sigma factor may be from any toxigenic strain of *Clostridium tetani.* TetR is a positive regulator of the tetanus toxin gene (tetX) in *Clostridium tetani* and is homologous to BotR (Marvaud et al., 1998, Infection and Immunity, 66: 5698-5702). TetR typically comprises 177 amino acids which many comprise the following sequence, SEQ ID NO:2. A typical representative is that of GenBank Accession Number NP_780796 as defined in SEQ ID NO: 2 below:

MNKTKKLIFKAAIKIFSQNGYNGTTMDDIAKEANVAKGTLYYHFKSKEEIFKFIINE
GMNIISEKLDDISKQDSDSISKLKAVCKAQLSVVYENRDFFKVIMSQLWGSEIRQ
SEIREKIEKYIKDIEKYIKDAVDEGGIKKGETYFMAYAVFGMLCSASIYELINEDKE
KIDEVAESLMDYILKGIES

The TcdR sigma factor may be from any toxinogenic strain of *Clostridium difficile,* such as strain 630 (Genbank Accession Number AM180355). TcdR (formerly called TxeR, Mani and Dupuy, 2001, Proc Natl Acad Sci U S A. 98: 5844-5849 or TcdD, Rupnik M, et al., 2005, Journal of Medical Microbiology 54: 113-117) comprises 184 amino acids which may comprise the following sequence, SEQ ID NO:3. A typical representative is that of GenBank Accession Number CAJ67491 as defined in SEQ ID NO: 3 below:

MQKSFYELIVLARNNSVDDLQEILFMFKPLVKKLSRVLHYEEGETDLIIFFIELIKNI
KLSSFSEKSDAIIVKYIHKSLLNKTFELSRRYSKMKFNFVEFDENILNMKNNYQSK
SVFEEDICFFEYILKELSGIQRKVIFYKYLKGYSDREISVKLKISRQAVNKAKNRAF
KKIKKDYENYFNL

The TcdR sigma factor is approximately 22-kDa in size and contains a potential C-terminal helix-turn-helix DNA-binding motif. The TcdR sigma factor shows sequence similarities to TetR, a positive regulator of the tetanus toxin gene in *Clostridium tetani,* BotR, a positive regulator of the botulism toxin genes in *Clostridium botulinum* and UviA, a putative positive regulator of the UV-inducible bacteriocin (bcn) gene of *Clostridium perfringens.*

The UviA sigma factor may be from any toxigenic strain of *Clostridium perfringens.* UviA is a positive regulator of the UV-inducible bacteriocin gene (Dupuy B, et al., 2005, Molecular Microbiology, 55: 1196-206.) and typically comprises 188 amino acids in which may comprise the following sequence, SEQ ID NO: 4. A typical representative is that of GenBank Accession Number ABG87874 as defined in SEQ ID NO: 4 below:

MQELYEKIKLCKLGNKEALQEVVNIFEKTIMKELFKFKKENSTIFMNEYDFQDKK
SEITLNVLNAIKNMPIESFEYKTDYSVKKYIRKTILNKLNEIKTKEFNKTENEYKYE
FDFSFFKSSDFNEPNTDIFVHDLIDKLSEKERKVIKYKYIYGKSDVEIGELLNCSR
QYVNKIKNRALKNIKKFLDD

An expression vector of the invention may be a self-replicating extrachromosomal vector or a vector that facilitates the integration of the expression vector into a bacterial host chromosome. The expression vector may be a plasmid, phage or phasmid or conjugative element, such as a conjugative transposon.

The expression vector is capable of being chromosomally integrated and maintained in a bacterial cell or is capable of being maintained extrachromosomally in a bacterial cell.

The expression vector may comprise an expression cassette. The expression cassette may comprise a promoter recognised by group 5 RNA polymerase sigma factor operably linked to a heterologous nucleic acid.

The expression cassette may also include a selectable marker gene to allow the selection of transformed host cells. Selectable marker genes are well known in the art and will vary with the host cell used. Preferred selectable marker genes are those specifying resistance to antibiotics, nucleoside and amino acid analogues and heavy metals, as well as markers complementing auxotrophic phenotypes. Antibiotic resistance markers include those specifying resistance to tetracycline (such as *tetM* and *tetA*)*,* erythromycin and lincomyin (such as *ermB*) ampicillin (such as *bla*)*,* penicillin (such as *penP*) chloramphenicol and thiamphenicol (such as *catP*)*,* kanamycin (such as *kan*)*,* spectinomycin (such as *aad9*) and streptomycin. Examples of nucleoside analogues include fluoroorotic acid, and 5'-fluorocytosine.

The expression cassette may include one or more other regulatory components in addition to said promoter. Typically the one or more regulatory components may include but are not limited to, nucleotide sequences corresponding to recombination sites, leader or signal sequences, ribosomal binding sites, transcriptional start and termination sequences, and enhancer or activator sequences.

According to another aspect of the invention, there is provided an expression vector which comprises an expression cassette comprising a promoter recognised by the group 5 RNA polymerase sigma factor operably linked to a heterologous nucleic acid encoding a CAZyme. Preferably, the expression vector comprises an expression cassette comprising a *tcdA* and/or *tcdB* promoter operably linked to the heterologous nucleic acid.

Preferably, the expression vector comprises the TcdR sigma factor and a *tcdA* and/or *tcdB* promoter operably linked to the heterologous nucleic acid.

The promoter recognised by the group 5 RNA polymerase sigma factor may be that of the *C*. *botulinum ntnh* or *ha33* gene, the *C*. *tetani tetX* gene, the *tcdA* and *tcd* genes of *C*. *difficile and*/*or* the *bcn* gene of *C*. *perfringens.* Preferably the promoter is *tcdA and*/*or tcdB.* The promoters *ntnh, ha33, tetX, tcdA, tcdB and*/*or bcn* may be interchangeable.

The *ntnh* or *ha33* promoters may be from any toxinogenic strain of *Clostridium* botulinum such as ATCC 3502, NCTC 2916, Hall A, Kyoto, Loch Maree or Eklund. The promoter may comprise the following sequence: aaaattttagg**tttaca**aaaaatagtgtggctat**gttata**tataaatgataagaatatactgaaaaa. (SEQ ID NO: 5). The bold sequences **tttaca** and **gttata** represent the promoter -35 and -10 regions, respectively.

The *tetX* promoter may be from any toxinogenic strain of *Clostridium* tetani, such as *Clostridium tetani* strain E88 or CN655. The promoter may comprise the following sequence, SEQ ID NO:6:
taaattttcag**tttaca**aaaaataacctgattat**gttata**tgtaattgtaaaaaacatataaaaaat

The bold sequences **tttaca** and **gttata** represent the promoter -35 and -10 regions, respectively.

The *tcdA* promoter may be from any toxinogenic strain of *Clostridium difficile,* such as strain 630. The promoter may comprise the following sequence, SEQ ID NO: 7:
tataagatatg**tttaca**aattactatcagacaat**ctcctt**atctaataGaagagtcaattaactaat. The bold sequences **tttaca** and **ctcctt** represent the promoter -35 and -10 regions, respectively. The upper case letter in the site sequence represents position +1 of mRNA.

The *tcdB* promoter may be from any toxinogenic strain of *Clostridium difficile,* such as strain 630. The promoter may comprise the following sequence, SEQ ID NO: 8:
atctaagaatatcttaatttttatattttatatagaacaaagtttacatatttatttcagacaacgtctttattcaatcga aga, which contains two overlapping promoter sequences comprising ptcdB1 and ptcdB2:
ptcdB1 (SEQ ID NO: 9): gaacaaag**tttaca**tatttatttcagacaac**gtcttt**attcaatcGaaga ptcdB2 (SEQ ID NO:10): a**tctaag**aatatcttaatttt**tatatttt**atatagaacaaagtttAcata The bold sequences **tctaag** and **tatttt** represent the promoter -35 and -10 regions, respectively.

Preferably, the *tcdA* or *tcdB* promoter is derived from a regulatory component of a *tcdA* or *tcdB* gene, respectively.

The *bcn* promoter may be from any bacteriocinogenic strain of *Clostridium* perfringens, such as encoded by the plasmid plP404 as found in strain SM101. The promoter may comprise the following sequence: tataaatttag**tttaca**aaattgaagtcaaatta**cttttt**atattatgtaaaacaaattcaagcttg. (SEQ ID NO: 11). The bold sequences **tttaca** and **cttttt** represent the promoter -35 and -10 regions, respectively.

The heterologous nucleic acid may be any nucleic acid wherein the nucleic acid is not the natural nucleic acid encoded by the promoter recognised by the group 5 RNA polymerase sigma factor. The heterologous nucleic acid includes a synthetic nucleic acid.

The heterologous nucleic acid may be a transposase-encoding nucleic acid. The heterologous nucleic acid may be a nucleic acid encoding at least one enzyme involved in butanol production, isopropanol production or acetone production.

Described is the use of a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a heterologous nucleic acid; wherein (a) and (b) are located on the same expression vector, separate expression vectors or are integrated into the bacterial host genome.

Also described is the use of a bacterial expression system for expressing a nucleic acid comprising:
(a) DNA encoding the TcdR sigma factor; and
(b) an expression cassette comprising a *tcdA* and/or *tcdB* promoter operably linked to a heterologous nucleic acid; wherein (a) and (b) are located on the same expression vector, separate expression vectors or are integrated into the bacterial host genome.

According to another aspect of the invention, there is provided a method for expressing a nucleic acid in a non-pathogenic bacterial cell comprising:
(1) introducing a bacterial expression system according to the invention into the bacterial host; wherein the bacterial expression system comprises:
   (a) DNA encoding a group 5 RNA polymerase sigma factor; and
   (b) an expression cassette comprising a promoter recognised by the group 5 RNA polymerase sigma factor operably linked to a heterologous nucleic acid encoding CAZyme;
   wherein (a) and (b) are located on the same expression vector, separate expression vectors or are integrated into the non-pathogenic bacterial host genome.

In a preferred embodiment, the method for expressing a nucleic acid in a bacterial cell comprises:
(1) introducing the bacterial expression system into the bacterial host genome; wherein the bacterial expression system comprises:
   (a) DNA encoding the TcdR sigma factor; and
   (b) an expression cassette comprising a *tcdA* and/or *tcdB* promoter operably linked to the heterologous nucleic acid; wherein (a) and (b) are located on the same expression vector, separate expression vectors or are integrated into the bacterial host genome.
   (a) and (b) as described above may be integrated into the bacterial genome in a single event or in two separate events.

The expression vector and or expression system are preferably for use in a bacterial cell, such as a bacterial cell from the class Clostridia, including the genus *Clostridium.* Preferably, the expression system is for use in the species *Clostridium acetobutylicum.*

The bacterial cell may be any bacterial species, but preferably members of the bacterial phylum Firmicutes composed of the class Clostridia (orders Clostridiales, Halanaerobiales, Natranaerobiales and Thermoanaerobacterales), the class Bacilli (orders Bacillales and Lactobacillales) and the class Mollicutes (orders Acholeplasmatales, Anaeroplasmatales, Entomoplasmatales, Haloplasmatales and Mycoplasmatales). Within the order Clostridiales is the genus, *Clostridium.* Preferred species are *C*. *acetobutylicum, C. aerotolerans, C. baratii, C. beijerinckii, C. bifermentans, C. botulinum, C. butyricum, C. cadaveris, C. cellulolyticum, C. chauvoei, C. clostridioforme, C. colicanis, C. difficile, C. estertheticum,C. fallax, C. feseri, C. formicaceticum, C. histolyticum, C. innocuum, C. kluyveri, C. Ijungdahlii, C. lavalense, C. novyi, C. oedematiens, C. paraputrificum, C.pasteurianum, C. perfringens, C. phytofermentans, C. piliforme, C. ragsdalei, C. ramosum, C. scatologenes, C. septicum, C. sordellii, C. sporogenes, C. sticklandii, C. tertium, C. tetani, C. thermocellum, C. thermosaccharolyticum, C*. *tyrobutyricum, C.paprosolvens, C*. *saccharobutylicum, C*. *carboxidovorans, C*. *scindens,* and *C*. *autoethanogenum.* Within the order Bacillales are Bacillaceae which include the genera *Bacillus* and *Geobacillus,* and Staphylococcaceae, which include the genus *Staphylococcus.* Preferred *Bacillus* species are: *B. alcalophilus, B. aminovorans, B. amyloliquefaciens, B. anthracis, B. caldolyticus, B. circulans, B. coagulans, Bglobigii, B. licheniformis, B. natto, B. polymyxa, B. phaericus, B. stearothermophilus, B. subtilis, B. thermoglucosidasius, B. thuringiensis* and *B*. *vulgatis.* Preferred *Geobacillus* species are: *G*. *debilis, G. stearothermophilus, G. thermocatenulatus, G. thermoleovorans, G. kaustophilus, G. thermoglucosidasius, G. thermodenitrificans, G. gargensis, G. jurassicus, G. lituanicus, G. pallidus, G. subterraneus, G. tepidamans, G. thermodenitrificans, G. thermoglucosidasius, G. thermoleovorans, G. toebii, G. uzenensis* and *G*. *vulcani.* Preferred *Staphylococcus* species include: *S*. *arlettae, S. aureus, S. auricularis, S. capitis, S. caprae, S. carnosus, S. chromogenes, S. cohnii, S. condimenti, S. delphini, S. devriesei, S. epidermidis, S. equorum, S. felis, S. fleurettii, S. gallinarum, S. haemolyticus, S. hominis, S. hyicus, S. intermedius, S. kloosii, S. leei, S. lentus, S. lugdunensis, S. lutrae, S. lyticans, S. massiliensis, S. microti, S. muscae, S. nepalensis, S. pasteuri, S. pettenkoferi,S. piscifermentans, S*. *pseudintermedius, S. pulvereri, S. rostri, S. saccharolyticus, S. saprophyticus, S. schleiferi, S. sciuri, S. simiae, S. simulans, S. stepanovicii, S. succinus, S. vitulinus, S. warneri* and *S*. *xylosus.*

Preferably, the bacterial cell is *C*. *acetobutylicum, C. difficile, C. beijerinckii, C. Ijungdahlii, C. kluyveri, C. botulinum, C. autoethanogenum, C.pasteurianum, C*. *saccharobutylicum, C*. *carboxidovorans, C*. *sporogenes, C*. *phytofermentans, C. ragsdalei, C. tyrobutyricum, C. perfringens, C. butyricum, C. cellulolyticum, C. formicaceticum, C. novyi, C. scatologenes, C. septicum, C*. *sordellii, C*. *sticklandii, C*. *tetani, C*. *thermocellum, C*. *thermosaccharolyticum, C.paprosolvens, C. scindens,* or *C*. *bifermentans.*

Preferably the bacterial cell is *C.ljungdahlii.* Preferably, the bacterial cell is *C*. *acetobutylicum.* Preferably the bacterial cell is *C.autoethanogenum.* Preferably, the bacterial cell is *C.carboxidovorans.* Preferably, the bacterial cell is *C.ragsdalei.* Preferably, the bacterial cell is *C.scatologenes.* Preferably, the bacterial cell is *C.scindens.* Preferably, the bacterial cell is *C.pasteuranium.* Preferably, the bacterial cell is *C.phytofermentans.* Preferably, the bacterial cell is *C.beijerinckii.*

### Use of the expression system for expressing CAZyme-encoding nucleic acids

Described is the use of the expression system for producing carbohydrate-active enzymes and binding proteins involved in the synthesis and degradation of complex carbohydrates, referred to as "CAZymes"(Cantarel BL, et al., 2009, The Carbohydrate-Active EnZymes database (CAZy): an expert resource for Glycogenomics, Nucleic Acids Research 37:D233-238: http://www.cazypedia.org/).

The bacterial expression system for expressing a nucleic acid may comprise:
(a) DNA encoding a TcdR sigma factor; and
(b) an expression cassette comprising a *tcdA* and/or *tcdB* promoter operably linked to at least one CAZyme-encoding nucleic acid; and wherein (a) and (b) are located on the same expression vector, separate expression vectors or are integrated into the bacterial host genome.

Described is the use of a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to least one CAZyme-encoding nucleic acid; wherein (a) and (b) are located on the same expression vector, separate expression vectors or are integrated into the bacterial host genome.

Also described is the use of a bacterial expression system for expressing a nucleic acid comprising:
(a) DNA encoding the TcdR sigma factor; and
(b) an expression cassette comprising a *tcdA* and/or *tcdB* promoter operably linked to least one CAZyme-encoding nucleic acid; wherein (a) and (b) are located on the same expression vector, separate expression vectors or are integrated into the bacterial host genome.

The CAZyme-encoding nucleic acid may encode enzymes involved in cellulose degradation, such as Cel48F of *Clostridium cellulolyticum* (Blouzard J. C. et al., 2007, J. Bacteriol. 189: 2300-2309) and CelA of *Clostridium thermocellum* (Beguin P. et al., 1985, J. Bacteriol. 162: 102-105), or the degradation of xylanase, such as Xyn10A of *Clostridium cellulolyticum* (Blouzard J.C. et al., 2007, J. Bacteriol. 189: 2300-2309). These enzymes may be composed entirely of endogenous encoded amino acid sequences, or consist of hybrid chimeric enzymes in which the hydrolytic domain of one enzyme is combined with the dockerin domain of another enzyme able to bind to a heterologous cohesion domain present on a heterologous scafoldin protein. Thus, the hydrolytic domain of a hydrolase from a *Clostridium cellulolyticum* enzyme (eg. Cel48F) may be combined with the dockerin domain of the *Clostridium acetobutylicum* Cel9C, or the Xyn10A hydrolytic domain may be combined with the dockerin domain of the *Clostridium thermocellum* dockerin domain of CelS. Alternatively, an enzyme may be used which encodes the natural hydrolytic domain and natural dockerin domain, such as the CelA protein of *Clostridium thermocellum.* Preferably, the bacterial cell is a *Clostridium* bacterial cell. More preferably, the bacterial cell is a solvent-producing species such as *C*. *acetobutylicum.*

Preferably, the CAZyme-encoding nucleic acid encodes Cel48F, CelA and/or Xyn10A.

### Use of the expression system for the development of industrial strains of C.acetobutylicum for the production of useful chemical commodities and fuels.

There is currently great interest in harnessing the metabolism of prokaryotic species to generate chemical commodities and fuels through their growth on all manner of cost effective feedstocks. Metabolism is the sum of the biochemical reactions required for energy generation and the use of energy to synthesize essential cell material from small molecules. Accordingly, there is an energy-generating component, called catabolism, and an energy-consuming, biosynthetic component, called anabolism. Catabolic reactions lead to energy generation in the form of nucleoside triphosphates like e.g. ATP and reducing equivalents like e.g. NADH and NADPH, which can then be used in anabolic reactions to build cell material from nutrients in the environment. The specific metabolic properties of a microbe determine its fundamental lifestyle and its potential use in industrial processes.

All microbial metabolisms can be divided according to: (1) How carbon is captured (either derived from carbon dioxide - autotrophic, from organic compounds - heterotrophic, or: from CO₂ and organic acids - mixotrophic); (2) How the organism obtains the reducing equivalents needed either in energy conservation or in biosynthetic reactions (either from inorganic compounds - lithotrophic, or organic compounds organotrophic), and (3) How the organism obtains energy for living and growth (energy is obtained from external chemical compounds - chemotrophic, or from light - phototrophic).

Fermentation is a specific type of heterotrophic metabolism that uses organic carbon instead of oxygen as a terminal electron acceptor. As oxygen is not required, fermentative metabolism takes place under anaerobic conditions. Many organisms can use fermentation under anaerobic conditions and aerobic respiration when oxygen is present. Instead of using an ATPase as in respiration, ATP during fermentative metabolism is produced by substrate-level phosphorylation where a phosphate group is transferred from a high-energy organic compound to ADP to form ATP. As a result of the need to produce high energy phosphate-containing organic compounds (generally in the form of CoA-esters) fermentative organisms use NADH and other cofactors to produce many different reduced metabolic by-products, often including hydrogen gas (H₂). These reduced organic compounds are generally small organic acids and alcohols derived from pyruvate, the end product of glycolysis. Examples include alcohols, such as ethanol and butanol, and organic acids, such as acetate, lactate, and butyrate. Fermentative organisms are very important industrially and may be used to make many different types of chemical commodities and fuels.

One such organism is *Clostridium acetobutyicum,* which produces acetic acid and butyric acid during exponential growth, and then undergoes a profound physiological/metabolic 'switch' as the cells enter stationary phase, whereupon the solvents acetone, butanol and ethanol are produced. The conditions for the 'switch' are poorly-defined, but include extracellular acid accumulation and low pH. The stage of acid production is known as 'acidogenic' phase, the stage of solvent production as 'solventogenic' phase. Within the solventogenic phase acids, produced during the early acidogenic phase, are re-absorbed and converted into solvents.

Several problems arise, the first of which occurring during solventogenic fermentation, where acid re-uptake leads to the loss of carbon as acetone, a low-value by-product, and as CO₂. The switch to solvent production occurs at late exponential/early stationary phase in laboratory and industrial cultures. A limit is therefore imposed on the maximum theoretical yield of butanol, as carbon must be lost as acetone and CO₂ to recover (and convert to butanol) the organic acids produced during the first phase of the fermentation. Secondly, the regulatory mechanism(s) of the switch to solventogenesis are not well understood, and the switch is not very robust under known conditions. Some cultures fail to switch, and these are completely unproductive. Solvent production is typically achieved during rapid growth in sugar-rich medium, very different conditions than would be experienced by a strain growing on a renewable substrate, such as lignocellulose. Thirdly, *C*. *acetobutylicum* is known to 'degenerate', that is, to permanently lose the capacity for solventogenesis, among other phenotypes, especially during serial or continuous culturing. At least one mechanism for this degeneration has been identified as the loss of the large native plasmid pSOL1, which includes genes essential for solventogenesis.

Use of the expression system allows the development of strains that (1) produce butanol and isopropanol continuously, including during exponential growth, (2) express the desired fermentation enzymes constitutively, not subject to native regulation, and (3) have the necessary genes stably located on the chromosome, where they are not at risk from the plasmid-loss mode of degeneration. Isopropanol is not naturally produced by *C*. *acetobutylicum,* but its production through genetic manipulation would allow the transformation of the low-value product acetone to a higher value product.

The expression system may be utilised for the development of industrial strains of *C.acetobutylicum* for the production of useful chemical commodities and fuels, such as succinate, isoprenes, alcohols (such as ethanol, isopropanol, 2,3-butanediol, *iso*-butanol and *n*-butanol) and solvents, such as acetone.

Of particular interest are those chemicals that can be produced biologically from sugars. These include 1-carbon chemicals (eg., formic acid, methanol, carbon monoxide), 2-carbon chemicals (e.g., acetaldehyde, acetic acid and anhydride, ethanol, glycine, oxalic acid, ethylene gycol, ethylene oxide), 3-carbon chemicals (e.g., alanine, glycerol, 3-hydroxypropionic acid, isopropanol, lactic acid, malonic acid, serine, proprionic acid, acetone), 4-carbon chemicals (e.g., acetoin, aspartic acid, butadiene, butanediol, butanol, fumaric acid, 3-Hydroxybutyrolactone, malic acid, succinic acid, threonine), 5-carbon chemicals (arabinitol, furfural, glutamic acid, glutaric acid, isoprene, Itaconic acid, levulinic acid, proline, xylitol, xylonic acid) and 6-carbon chemicals (, aconitic acid, adipic acid, ascorbic acid, caproic acid, citric acid, fructose, 2,5 furan dicarboxylic acid, glucaric acid, gluconic acid, Kojic and comeric acid, lysine and soribitol). These building blocks may be subsequently converted into high-value bio-based chemicals or materials, such as acrylic acid, 1,3-propanediol, methyl acrylate, acrylamide, 1,4-butanediol, Butyrolactone, tetrahydrofuran, 2-pyrrolidone, malonic acid, caprolactam, hexamethylenediamine, 6-hydroxycaproic acid and 6-aminocaproic acid, isoprene, isoprenoids, carotenoids, quinones, squalene and various alkanes and alkenes.

Preferably, the 2-carbon chemical is ethylene. Preferably, the 3-carbon chemical is isopropanol. Preferably, the 4-carbon chemical is butanol. Preferably, the 4-carbon chemical is succinate. Preferably, the 4-carbon chemical is butadiene. Preferably, the 4-carbon chemical is butanediol. Preferably, the 5-carbon chemical is isoprene. Preferably, the 6-carbon chemical is adipic acid. Preferably, the 6-carbon chemical is caproic acid.

Described is a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a nucleic acid encoding one or more 1-carbon chemical, 2-carbon chemical, 3-carbon chemical, 4-carbon chemical, 5-carbon chemical, and/or 6-carbon chemical; wherein (a) and (b) are located on the same expression vector, separate expression vectors or are integrated into the bacterial host genome.

Described is a bacterial expression system for expressing a nucleic acid comprising:
(a) DNA encoding a TcdR sigma factor; and
(b) an expression cassette comprising a *tcdA* and/or *tcdB* promoter operably linked to a nucleic acid encoding one or more 1-carbon chemical, 2-carbon chemical, 3-carbon chemical, 4-carbon chemical, 5-carbon chemical, and/or 6-carbon chemical; wherein (a) and (b) are located on the same expression vector, separate expression vectors or are integrated into the bacterial host genome.

Described is the use of a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a nucleic acid encoding one or more 1-carbon chemical, 2-carbon chemical, 3-carbon chemical, 4-carbon chemical, 5-carbon chemical, and/or 6-carbon chemical; wherein (a) and (b) are located on the same expression vector, separate expression vectors or are integrated into the bacterial host genome.

Also described is the use of a bacterial expression system for expressing a nucleic acid comprising:
(a) DNA encoding the TcdR sigma factor; and
(b) an expression cassette comprising a *tcdA* and/or *tcdB* promoter operably linked to a nucleic acid encoding one or more 1-carbon chemical, 2-carbon chemical, 3-carbon chemical, 4-carbon chemical, 5-carbon chemical, and/or 6-carbon chemical; wherein (a) and (b) are located on the same expression vector, separate expression vectors or are integrated into the bacterial host genome.

Described is a method for expressing a nucleic acid in a bacterial cell comprising:
(1) introducing a bacterial expression system into the bacterial host genome; wherein the bacterial expression system comprises:
   (a) DNA encoding a group 5 RNA polymerase sigma factor; and
   (b) an expression cassette comprising a promoter recognised by the group 5 RNA polymerase sigma factor operably linked to a nucleic acid encoding one or more 1-carbon chemical, 2-carbon chemical, 3-carbon chemical, 4-carbon chemical, 5-carbon chemical, and/or 6-carbon chemical; wherein (a) and (b) are located on the same expression vector, separate expression vectors or are integrated into the bacterial host genome.

Also described is a method for expressing a nucleic acid in a bacterial cell comprising:
(1) introducing a bacterial expression system into the bacterial host genome; wherein the bacterial expression system comprises:
   (a) DNA encoding the TcdR sigma factor; and
   (b) an expression cassette comprising a *tcdA* and/or *tcdB* promoter operably linked to a nucleic acid encoding one or more 1-carbon chemical, 2-carbon chemical, 3-carbon chemical, 4-carbon chemical, 5-carbon chemical, and/or 6-carbon chemical; wherein (a) and (b) are located on the same expression vector, separate expression vectors or are integrated into the bacterial host genome.

Described is is provided a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a nucleic acid encoding at least one enzyme involved in butanol production, isopropanol production and/or acetone production; wherein (a) and (b) are located on the same expression vector, separate expression vectors or are integrated into the bacterial host genome.

Described is is provided a bacterial expression system for expressing a nucleic acid comprising:
(a) DNA encoding a TcdR sigma factor; and
(b) an expression cassette comprising a *tcdA* and/or *tcdB* promoter operably linked to a nucleic acid encoding at least one enzyme involved in butanol production, isopropanol production and/or acetone production; wherein (a) and (b) are located on the same expression vector, separate expression vectors or are integrated into the bacterial host genome.

Described is the use of a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to at least one enzyme involved in butanol production, isopropanol production and/or acetone production; wherein (a) and (b) are located on the same expression vector, separate expression vectors or are integrated into the bacterial host genome.

Also described is the use of a bacterial expression system for expressing a nucleic acid comprising:
(a) DNA encoding the TcdR sigma factor; and
(b) an expression cassette comprising a *tcdA* and/or *tcdB* promoter operably linked to at least one enzyme involved in butanol production, isopropanol production and/or acetone production; wherein (a) and (b) are located on the same expression vector, separate expression vectors or are integrated into the bacterial host genome.

Preferably, the enzyme involved in butanol production is *ald* and/or *bdhB* (derived from *Clostridium beijerinckii* and encoding coenzyme A-acylating aldehyde dehydrogenase and NADH-dependent butanol dehydrogenase, respectively). Preferably, the enzyme involved in isopropanol production is adh (derived from Clostridium beijerinckii and encoding alcohol dehydrogenase). Preferably, the enzyme involved in acetone production is *adc* (encoding acetoacetate decarboxylase), *ctfA* (encoding acetoacetyl-CoA transferase subunit A) and *ctfB* (encoding acetoacetyl-CoA transferase subunit B).

### Use of the expression system for expressing a transposase

Use of the expression system for the expression of a transposase has the advantage that the promoter does not function in the donor *E.coli* host, as it is only recognised by a specific *C*. *difficile* sigma factor. As a consequence, transposition does not take place in the *E.coli* donor prior to transfer to the clostridial host, an undesirable destabilising trait, and expression of the transposase (and transposition) is therefore confined to the eventual clostridial host.

Described is a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a transposase-encoding nucleic acid; wherein (a) and (b) are located on the same expression vector, separate expression vectors or are integrated into the bacterial host genome.

Described is a bacterial expression system for expressing a nucleic acid comprising:
(a) DNA encoding a TcdR sigma factor; and
(b) an expression cassette comprising a *tcdA* and/or *tcdB* promoter operably linked to a transposase-encoding nucleic acid; wherein (a) and (b) are located on the same expression vector, separate expression vectors or are integrated into the bacterial host genome.

Described is the use of a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a transposase-encoding nucleic acid; wherein (a) and (b) are located on the same expression vector, separate expression vectors or are integrated into the bacterial host genome.

Also described is the use of a bacterial expression system for expressing a nucleic acid comprising:
(a) DNA encoding the TcdR sigma factor; and
(b) an expression cassette comprising a *tcdA* and/or *tcdB* promoter operably linked to a transposase-encoding nucleic acid; wherein (a) and (b) are located on the same expression vector, separate expression vectors or are integrated into the bacterial host genome.

Described is a method for expressing a nucleic acid in a bacterial cell comprising:
(1) introducing a bacterial expression system into the bacterial host genome; wherein the bacterial expression system comprises:
   (a) DNA encoding a group 5 RNA polymerase sigma factor; and
   (b) an expression cassette comprising a promoter recognised by the group 5 RNA polymerase sigma factor operably linked to a transposase-encoding nucleic acid; wherein (a) and (b) are located on the same expression vector, separate expression vectors or are integrated into the bacterial host genome.

Also described is a method for expressing a nucleic acid in a bacterial cell comprising:
(1) introducing a bacterial expression system into the bacterial host genome; wherein the bacterial expression system comprises:
   (a) DNA encoding the TcdR sigma factor; and
   (b) an expression cassette comprising a *tcdA* and/or *tcdB* promoter operably linked to a transposase-encoding nucleic acid; wherein (a) and (b) are located on the same expression vector, separate expression vectors or are integrated into the bacterial host genome.

The transposase-encoding nucleic acid may encode for *Himar1 C9.*

### Use of the expression system in generating transposon libraries and screening for mutants of a bacterial strain affected in solvent production, tolerance or substrate utilisation.

Use of the expression system described herein may be used to generate transposon libraries. Such libraries may be screened for mutants affected in solvent production, solvent tolerance, or in substrate utilisation. Two fundamentally different approaches could be adopted:-
(i) a pool of transposon mutants could be generated to allow both the direct selection of mutants able to tolerate higher concentrations of solvent and to determine the identity of non-essential genes
(ii) a library could be created of specific transposon mutants in all non-essential genes that can subsequently be tested using BioLog approaches that are affected in solvent production and substrate utilisation

Described is a method of identifying a mutant of a bacterial strain affected in solvent production, tolerance or substrate utilisation, comprising:
(i) providing a library of transposon mutants of the bacterial strain generated through the use of a bacterial expression system described herein; and
(ii) selecting for mutants that have altered solvent production, tolerance or substrate utilisation.

The solvent may be acetone, butanol, ethanol, isopropanol, ethylene, butadiene, butanediol or isoprene.

Described is a mutant bacterial strain affected in solvent production, tolerance or substrate utilisation identified by the method described above.

The expression system, described herein, may also be used in conjunction with a recently described high throughput random approach, transposon directed insertion-site sequencing (TraDIS), which utilizes nucleotide sequencing to prime from the transposon and sequence into the adjacent target DNA, simultaneously mapping the site of insertion of every transposon in a mutant pool (Langridge et al., 2009. Genome Res. 19: 2308-16). TraDIS has previously been used to map 370,000 unique transposon insertion sites to the *Salmonella* Typhi chromosome. The density and resolution of mapped insertion sites (one every 13 bp) allowed the identification of every essential gene in the genome. Moreover, following growth of the mutant pool in the presence or absence of ox bile, the semi-quantitative nature of the assay led to the identification of genes that contributed to bile tolerance, a trait required for carriage of S*.Typhi.* Thus, the method can be used to simultaneously assay every gene in the genome to identify niche-specific essential genes. One such niche-specific condition is growth in the presence of butanol.

ABI SOLiD 3+ sequencing libraries could be prepared from DNA flanking transposon insertion sites from a solventogenic *Clostridium* species, such as *C. acetobutylicum,* grown in different selective conditions (eg. standard media as well as media supplemented with butanol). Sequence reads could then be matched back to the *Clostridium* genome to identify genes that are non-essential. Genes that are not represented or highly under-represented in each sample of sequences may be candidate essential genes. A total of 20 million mapped sequence tags of 40-50 bp may be generated, representing approximately 15 bp of transposon sequence and 25-35bp DNA flanking Himar1 C9 insertion sites. The observed distribution and frequency of insertion sites across the genome may be used to identify a list of potentially essential genes and sites under each condition.

The outcome may be two-fold. In the first instance it may allow the identification of genes that cannot be inactivated. This is extremely important as it may identify those genes for which time and effort using directed methods (TargeTron and ClosTron) would be wasted. Secondly, it may identify genes which contribute to solvent tolerance, providing valuable information on the mechanisms currently employed to confer resistance and may provide the basis of rational approaches in enhancing solvent resistance. To identify solvent resistance, the high density library generated through the use of the conditional vector described herein may be employed to directly select for mutants that have become more tolerant to solvents (for example, acetone, butanol, ethanol, isopropanol, ethylene, butadiene, butanediol or isoprene), an important goal in the drive to improve solvent production. Sequencing of the site of insertion of the transposon may further provide valuable information on the mechanisms currently employed to confer resistance and again may provide the basis of rational approaches in enhance solvent resistance.

Another valuable resource is the acquisition of a library of individual mutants comprising individual clones inactivated in every possible gene. Such a library may be generated using the conditional vector described herein to express a transposase-encoding nucleic acid. The generated library may then be screened, in a BioLog microtitre format, for those mutations that are affected in such properties as solvent production, solvent tolerance, and substrate utilization. Such information may be extremely valuable in considerably increasing the understanding of the metabolic processes responsible for solvent formation and sugar utilization, particularly in terms of regulation.

The following terms, unless otherwise indicated, shall be understood to have the following meanings:
The term 'CAZyme' as used herein refers to carbohydrate-active enzymes which build and breakdown complex carbohydrates and glycoconjugates. These are principally based on a family of enzymes termed glycoside hydrolases (EC 3.2.1.-), commonly abbreviated to GH. These are a widespread group of enzymes which hydrolyse the glycosidic bond between two or more carbohydrates or between a carbohydrate and a non-carbohydrate moiety. Trivial names for their members include cellulases, xylanases, chitinases, glucanases, arabinofuranosidase, xylosidase and cellobiohydrolases. Other CAZyme family members include: Glycosyltransferases (GTs), which undertake the formation of glycosidic bonds; polysaccharide lyases (PLs), which catalyse the non-hydrolytic cleavage of glycosidic bonds; carbohydrate esterases (CEs), which hydrolyse carbohydrate esters. These enzymes can incorporate an carbohydrate binding module (CBM). CAZymes which form part of a cellulosome also incorporate dockerin module essential to the formation of the cellulosome structure. The cellulosome is a large, secreted, multi-enzyme complex, or nanomachine responsible for the degradation of lignocellulose. It has two major components: (i) the core of the complex is a modular, non-catalytic scaffoldin protein which serves to bring enzymes into close proximity, and (ii) catalytic enzymatic subunits, which when anchored to scaffold via cohesin-dockerin interactions, enhanced enzyme activity. The cohesin domain forms part of the scaffoldin, whereas the corresponding dockerin domain forms part of the CAZyme. Only those CAZymes that are associated with a cellulosome possess a dockerin domain.

### Use of the expression system for expressing a prodrug converting enzyme (PCE).

A fundamental requirement of any new anti-cancer therapy is the facility to subject tumour cells to a toxic agent, while at the same time excluding normal healthy tissues from such exposure. Despite the conceptual simplicity, the derivation of such therapies has proven challenging. Attempts have been made to localise protein-based anti-cancer agents at the tumour using 'specific' antibodies or viral vectors. The former is disadvantaged by antigen heterogeneity and the absence of suitable antigen targets in many tumour types. Viral vectors suffer from a lack of tumour specificity, poor levels of transgene expression and inefficient distribution of the vector throughout the tumour.

An alternative approach which overcomes the above deficiencies has been devised based on the use of non-pathogenic clostridial spores. Whilst intravenously injected clostridial spores are dispersed throughout the body, only those that encounter the hypoxic environment of a solid tumour go on to germinate and multiply. The potential therapeutic efficacy is due not only to the fact that clostridia show a high selectivity for hypoxic tumour areas, but also because these hypoxic areas are considered one of the most important barriers to current cancer therapy. Delivery is thus exquisitely selective. This has led to the concept of engineering clostridia to produce anticancer agents, and in particular prodrug converting enzymes (PCEs), through the incorporation of the gene encoding the PCE into the genome of the clostridial cell. This approach is called Clostridial-Directed-Enzyme Prodrug Therapy (CDEPT). In CDEPT (Minton NP, 2003, Nature Reviews Microbiology, 1(3): 237-42) the anti-cancer drug is introduced into the bloodstream as harmless "prodrug", and is subsequently converted into the active drug by an "enzyme" that has been specifically targeted ("directed") to tumour cells through the colonisation of the tumour with clostridial cells producing the enzyme, prior to injection of the prodrug. As the enzyme is specifically produced within the tumour, high therapeutic doses are achieved only within the vicinity of the tumour, and nowhere else within the body. Moreover, not every tumour cell needs to be targeted as a single enzyme molecule can catalyse the generation of large quantities of therapeutic drug. Thus, there is no necessity to target every tumour cell, i.e., the so-called 'bystander effect'.

The successful use of CDEPT in human therapy requires that the gene encoding the PCE is introduced into the chromosome of the clostridial delivery vehicle used. A preferred embodiment of the expression system is therefore its use to bring about the expression of the chromosomally located PCE gene.

Described is a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a PCE-encoding nucleic acid; wherein (a) and (b) are located in the bacterial host genome.

Also described is a bacterial expression system for expressing a nucleic acid comprising:
(a) DNA encoding a TcdR sigma factor; and
(b) an expression cassette comprising a *tcdA* and/or *tcdB* promoter operably linked to a PCE-encoding nucleic acid; wherein (a) and (b) are located in the bacterial host genome.

Described is the use of a bacterial expression system for expressing a nucleic acid comprising:
(a) DNA encoding a group 5 RNA polymerase sigma factor; and
(b) an expression cassette comprising a promoter recognised by the group 5 RNA polymerase sigma factor operably linked to a PCE-encoding nucleic acid; wherein (a) and (b) are located in the bacterial host genome.

Described is a method for expressing a nucleic acid in a bacterial cell comprising:
(1) introducing a bacterial expression system into the bacterial host genome; wherein the bacterial expression system comprises:
   (a) DNA encoding a group 5 RNA polymerase sigma factor; and
   (b) an expression cassette comprising a promoter recognised by the group 5 RNA polymerase sigma factor operably linked to a PCE-encoding nucleic acid;
   wherein (a) and (b) are located in the bacterial host genome.

Also described is a method for expressing a nucleic acid in a bacterial cell comprising:
(1) introducing a bacterial expression system into the bacterial host genome; wherein the bacterial expression system comprises:
   (a) DNA encoding the TcdR sigma factor; and
   (b) an expression cassette comprising a *tcdA* and/or *tcdB* promoter operably linked to a PCE-encoding nucleic acid; wherein (a) and (b) are located in the bacterial host genome.

Described is a method for treating cancer, which method comprises administering to a subject, a bacterial cell comprising an expression vector which comprises (a) DNA encoding a group 5 RNA polymerase sigma factor; and (b) an expression cassette comprising a promoter recognised by the group 5 RNA polymerase sigma factor operably linked to a PCE-encoding nucleic acid; wherein (a) and (b) are located in the bacterial host genome.

Described is the use of a bacterial cell comprising an expression vector which comprises (a) DNA encoding a group 5 RNA polymerase sigma factor; and (b) an expression cassette comprising a promoter recognised by the group 5 RNA polymerase sigma factor operably linked to a PCE-encoding nucleic acid; wherein (a) and (b) are located in the bacterial host genome for the manufacture of a medicament for the treatment of cancer.

Described is a bacterial cell comprising an expression vector which comprises (a) DNA encoding a group 5 RNA polymerase sigma factor; and (b) an expression cassette comprising a promoter recognised by the group 5 RNA polymerase sigma factor operably linked to a PCE-encoding nucleic
(1) introducing a bacterial expression system into the bacterial host genome; wherein the bacterial expression system comprises:
   (a) DNA encoding a group 5 RNA polymerase sigma factor; and
   (b) an expression cassette comprising a promoter recognised by the group 5 RNA polymerase sigma factor operably linked to a PCE-encoding nucleic acid;
   wherein (a) and (b) are located in the bacterial host genome.

In a preferred embodiment of the invention, there is provided a method for expressing a nucleic acid in a bacterial cell comprising:
(1) introducing a bacterial expression system into the bacterial host genome; wherein the bacterial expression system comprises:
   (a) DNA encoding the TcdR sigma factor; and
   (b) an expression cassette comprising a *tcdA* and/or *tcdB* promoter operably linked to a PCE-encoding nucleic acid; wherein (a) and (b) are located in the bacterial host genome.

The invention further provides a method for treating cancer, which method comprises administering to a subject, a bacterial cell comprising an expression vector which comprises (a) DNA encoding a group 5 RNA polymerase sigma factor; and (b) an expression cassette comprising a promoter recognised by the group 5 RNA polymerase sigma factor operably linked to a PCE-encoding nucleic acid; wherein (a) and (b) are located in the bacterial host genome.

The invention further provides the use of a bacterial cell comprising an expression vector which comprises (a) DNA encoding a group 5 RNA polymerase sigma factor; and (b) an expression cassette comprising a promoter recognised by the group 5 RNA polymerase sigma factor operably linked to a PCE-encoding nucleic acid; wherein (a) and (b) are located in the bacterial host genome for the manufacture of a medicament for the treatment of cancer.

The invention further provides a bacterial cell comprising an expression vector which comprises (a) DNA encoding a group 5 RNA polymerase sigma factor; and (b) an expression cassette comprising a promoter recognised by the group 5 RNA polymerase sigma factor operably linked to a PCE-encoding nucleic acid; wherein (a) and (b) are located in the bacterial host genome for use in the treatment of cancer.

The PCE-encoding nucleic acid may encode for cytosine deaminase (CD), nitroreductase (NTR) and carboxypeptidase G2 (CPG2). CD mediates the conversion of 5-FC into 5-fluorouracil (5-FU). The differential toxicity between 5-FC and 5-FU is large (10⁴). This is because 5-FU is further metabolised into two inhibitors of DNA and RNA synthesis, 5-fluorouridine-5'-triphosphate and 5-fluoro-2'-deoxyuridine 5'-monophophate. NTR reduces the 4-nitro group of the prodrug CB1954 (5-(aziridin-1-yl)-2,4-dinitrobenzamide) to produce a cytotoxic hydroxylamine derivative that causes DNA interstrand crosslinking²⁰. On a dose by dose basis, the 4-hydroxylamine species is 10⁴- to 10⁵-fold more cytotoxic than the CB 1954 progenitor. CPG2 catalyses the cleavage of amidic, urethanic and ureidic bonds between an aromatic nucleus and L-glutamic acid. These include the prodrug CMDA, a benzoylglutamate mustard, which is derivatised to produce a benzoyl mustard that causes DNA-DNA crosslinking. The drug is up to 100-fold more toxic than its corresponding prodrug (Minton NP, 2003, Nature Reviews Microbiology, 1(3): 237-42)

Preferably, the PCE-encoding nucleic acid nucleic acid encodes for a nitroreductase, cytosine deaminase or carboxypeptidase G2.

The term 'nucleic acid' as used herein includes single or double-stranded mRNA, RNA, cRNA and DNA, said DNA inclusive of cDNA and genomic DNA.

A 'polynucleotide' is a nucleic acid having eighty or more contiguous nucleotides, while an 'oligonucleotide' has less than eighty nucleotides.

The term 'group 5 RNA polymerase sigma factor' as used herein refers to the RNA sigma factors BotR, TetR, TcdR and UviA which have been assigned to 'group 5' of the sigma 70 family. (Dupuy and Matamouros, 2006, Research Microbiology, 157: 201-205). The term also refers to a group 5 RNA polymerase sigma factor which has a sequence identity or homology of at least 60, 65%, 70%, 75%, 80%, 85%, 90%, 95%, 98%, 99% or 99.9% to BotR, TetR, TcdR and/or UviA.

A nucleic acid has 'identity', 'homology' or is 'homologous' to a second nucleic acid if the first nucleic acid sequence has a similar sequence to the second nucleic acid sequence. In a preferred embodiment, a homologous nucleic acid is one that exhibits at least 65% sequence homology to the plasmid replication region, more preferred is at least 70% sequence homology. Even more preferred are homologous nucleic acids that exhibit at least 75%, 80%, 85%, or 90% sequence homology to the plasmid replication region. In a yet more preferred embodiment a homologous nucleic acid exhibits at least 95%, 98%, 99% or 99.9% sequence identity. As used herein, homology between two regions of nucleic acid sequence (especially with respect to predicted structural similarities) is interpreted as implying similarity in function. The term 'homology' is synonymous with the term 'identity.'

By 'operably linked' it is meant that said promoter(s) is/are positioned relative to the endogenous, heterologous or synthetic nucleic acid to initiate, regulate or otherwise control transcription.

By 'heterologous nucleic acid' it is meant a nucleic acid distinct from that normally linked to a specific promoter. Preferably it means a nucleic acid distinct from a nucleic acid encoding for example, a *tcdA* or *tcdB* toxin-encoding gene. The heterologous nucleic may be a synthetic nucleic acid. The heterologous nucleic acid preferably encodes a polypeptide which is to be expressed in bacteria. The polypeptide can be a CAZyme enzyme such as the glycoside hydrolases Cel48F, Xyn10A *or* CelA. Other polypeptides of interest include transposase enzymes.

By 'endogenous nucleic acid' it is meant that the coding sequence faithfully corresponds to that found in the organism in which it is found.

By 'synthetic nucleic acid' it is meant that the coding sequence has been changed/synthesised such that it differs from the naturally occurring sequence, but still encodes the same protein sequence.

A 'transposase' is an enzyme that binds to the ends of a transposon and catalyzes the movement of the transposon to another part of the genome by a variety of mechanisms, including a cut and paste mechanism or a replicative transposition mechanism.

*'Himar1 C9'* refers to a mini-transposon in which the selectable marker, such as *catP* (encoding chlorampheniciol acetyltransferase and responsible for resistance to thiamphenicol or chloramphenicol), is flanked by inverted repeat regions (ITR1 and ITR2), proceeded by the transposase gene.

The term 'promoter' as used herein refers to a sequence of DNA, usually upstream (5') to the coding sequence of a structural gene, which controls the expression of the coding region by providing the recognition for RNA polymerase and/or other factors required for transcription to initiate at the correct site.

The skilled person in the art would appreciate that any of the preferred features according to any aspect of the invention may be applied to any other aspect of the invention described.

Preferred embodiments of the present invention will now be described, merely by way of example, with reference to the following drawings and examples.

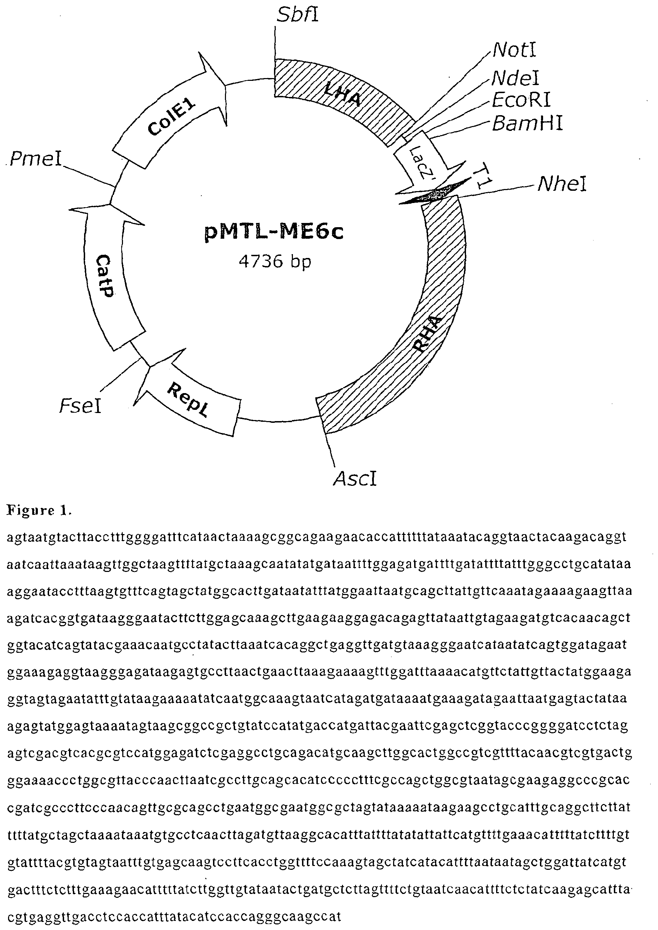

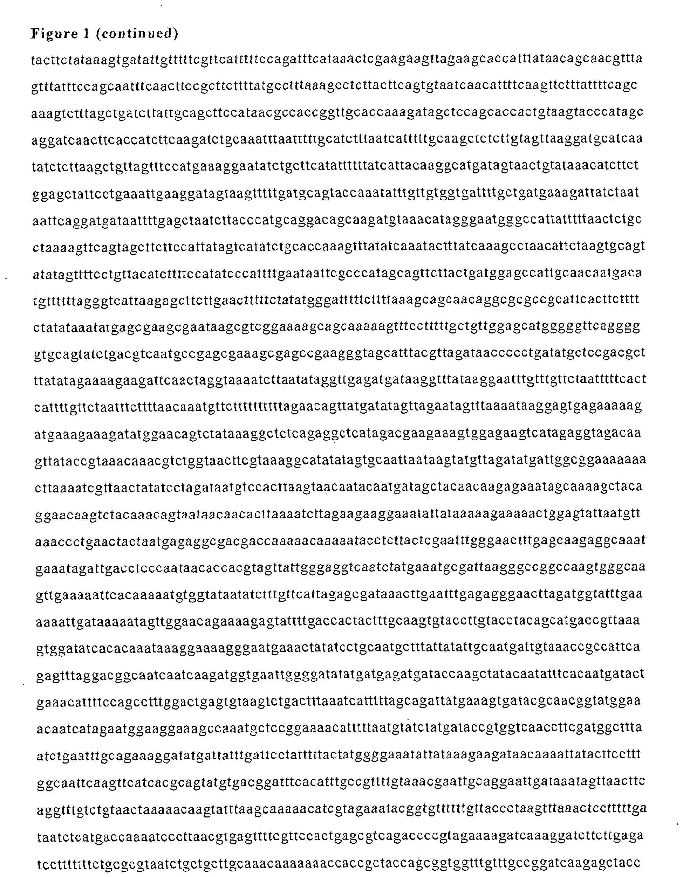

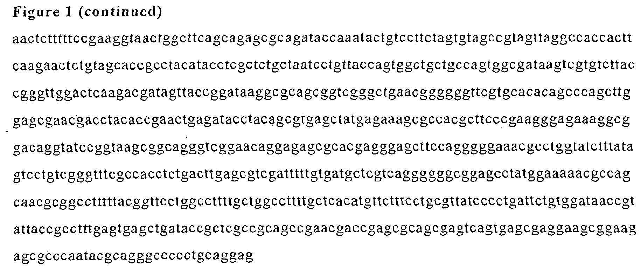

**Figure 1** - shows a schematic view of the plasmid pMTL-ME6c
   Key: LHA, left hand homology arm encompassing a foreshortened *Clostridium acetobutylicum pyrE* gene lacking its first 13 codons; LacZ', incorporating multiple cloning sites; T1, transcriptional terminator of the *Clostridium pasteurianum* ferredoxin gene; RHA, right hand homology arm composed of 1200 bp from immediately downstream of the *Clostridium acetobutylicum pyrE* gene, encompassing the *hydA* gene, CAC0028; RepL, the replication protein of plasmid plM13; *catP,* a *Clostridium perfringens-derived* gene encoding chloramphenicol acetyltransferase, and; ColE1, the replication origin of plasmid ColE1.

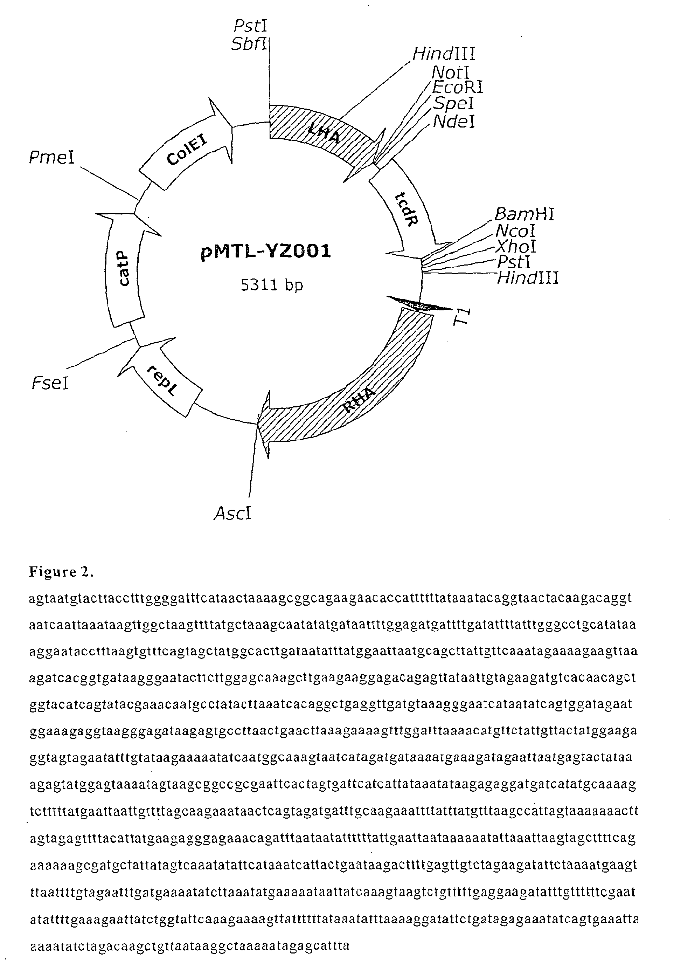

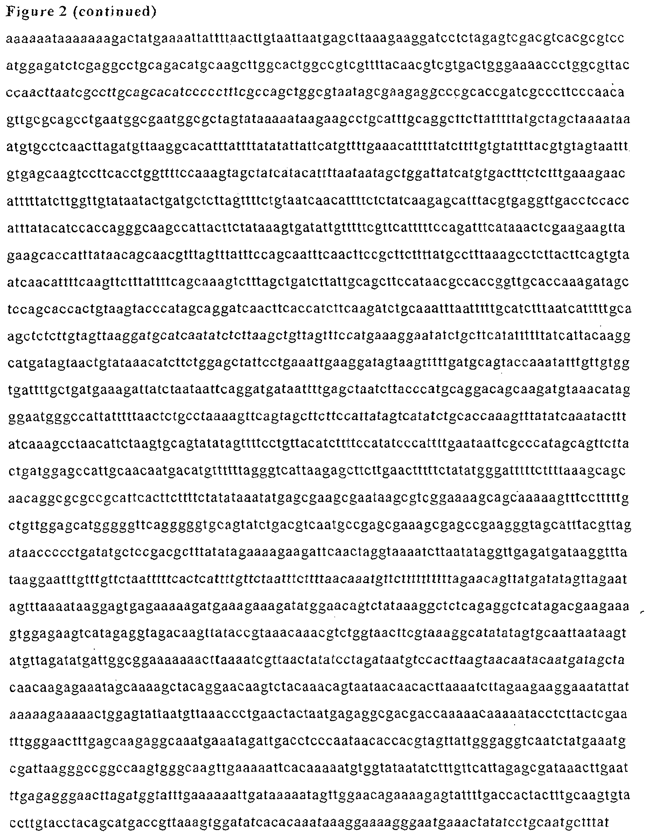

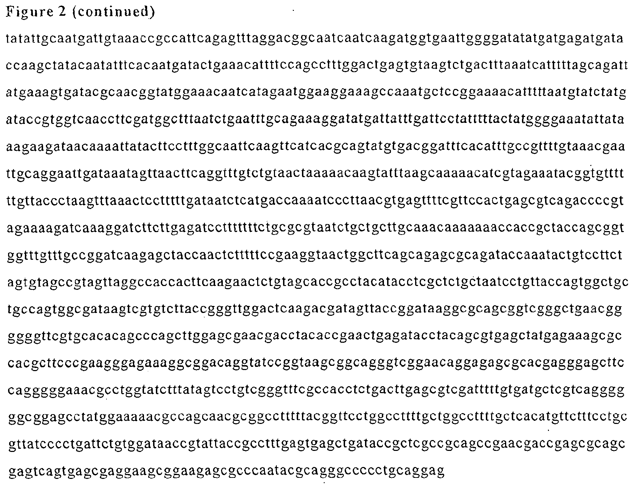

**Figure 2** - shows a schematic view of the plasmid pMTL-YZ001
   Key: LHA, left hand homology arm encompassing a foreshortened *Clostridium acetobutylicum pyrE* gene lacking its first 13 codons; *tcdR* , derived from *Clostridium difficile* strain 630 and encoding an alternative RNA polymerase sigma-factor assigned to group 5 of the sigma (70) family; T1, the transcriptional terminator of the *Clostridium pasteurianum* ferredoxin gene; RHA, right hand homology arm composed of 1200 bp from immediately downstream of the *Clostridium acetobutylicum pyrE* gene, encompassing the *hydA* gene, CAC0028; RepL, the replication protein of plasmid plM13; *catP,* a *Clostridium perfringens-derived* gene encoding chloramphenicol acetyltransferase, and; ColE1, the replication origin of plasmid ColE1.

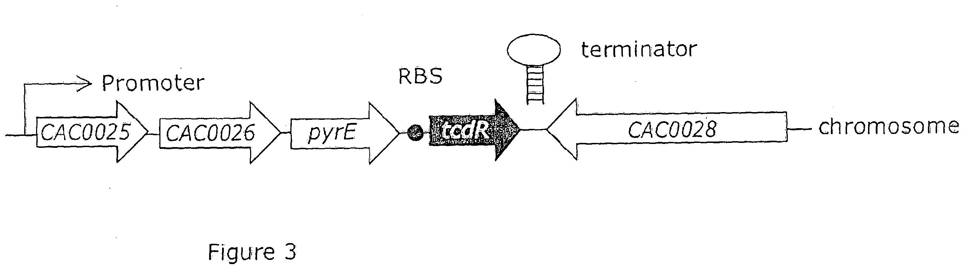

**Figure 3** - shows a schematic representation of the genome of strain CRG3011. A promoter-less copy of the *tcdR* gene (including its ribosome binding site, RBS) of *Clostridium difficile* strain 630 has been inserted into the genome using ACE technology immediately downstream of the pyrE gene (CAC0027). Illustrated are the surrounding genes, and the position of the promoter responsible for expression of *tcdR,* and the position of the *tcdR RBS.* The illustrated terminator is the T1 transcriptional terminator of the *Clostridium pasteurianum* ferredoxin gene.

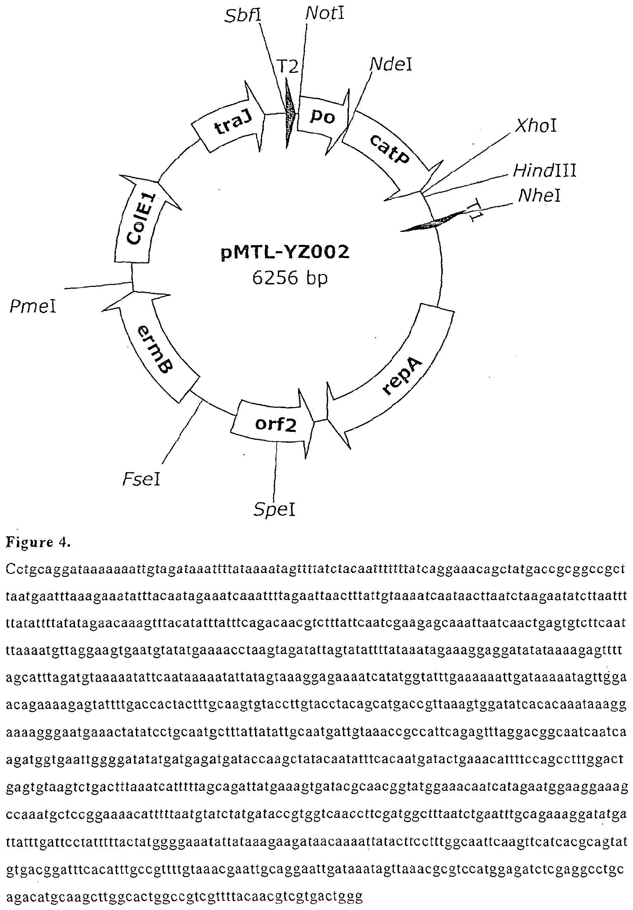

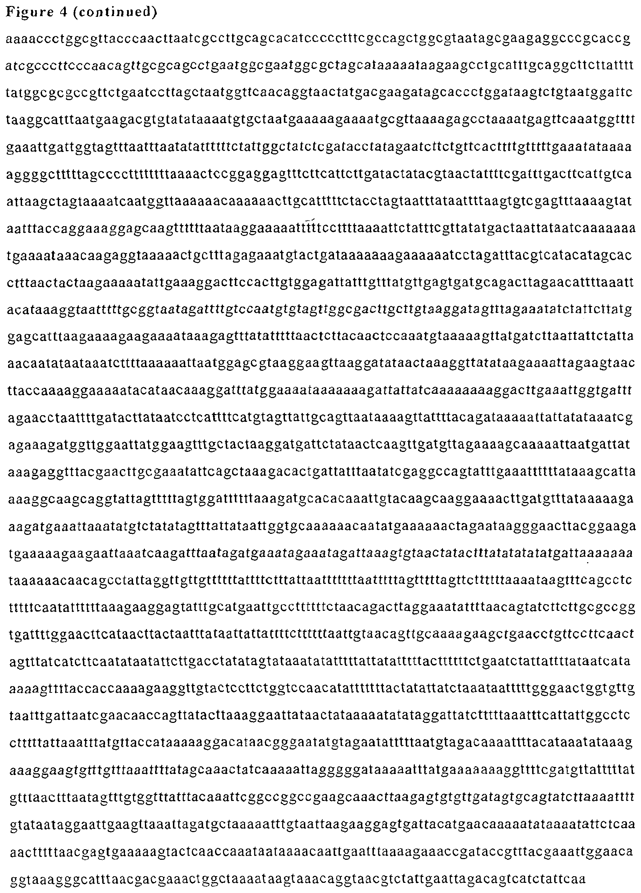

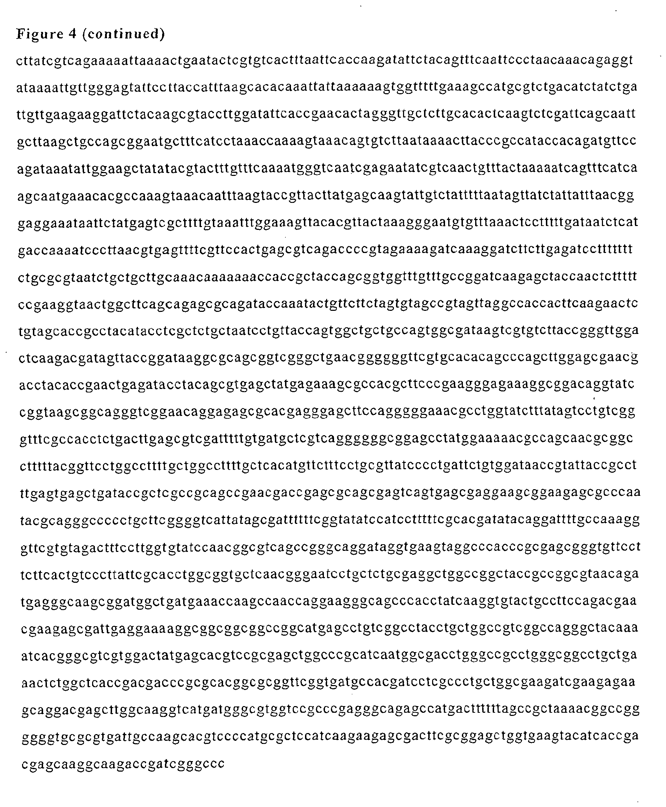

**Figure 4** - shows a schematic view of the plasmid pMTL-YZ002.
   Key: T2, transcriptional terminator descented downstream of the *Clostridium difficile* strain 630 CD0164 gene; po, the promoter region of the *Clostridium difficile tcdB* gene; *catP,* a *Clostridium perfringens-derived* gene encoding chloramphenicol acetyltransferase; T1, transcriptional terminator of the *Clostridium pasteurianum* ferredoxin gene; repA and orf2, replication region of the *Clostridium botulinum* plasmid pBP1; *ermB,* the macrolidelincosamide-streptogramin B antibiotic resistance gene of plasmid pAMβ1; ColE1, the replication origin of plasmid ColE1, and; *traJ,* transfer function of the RP4 *oriT* region.

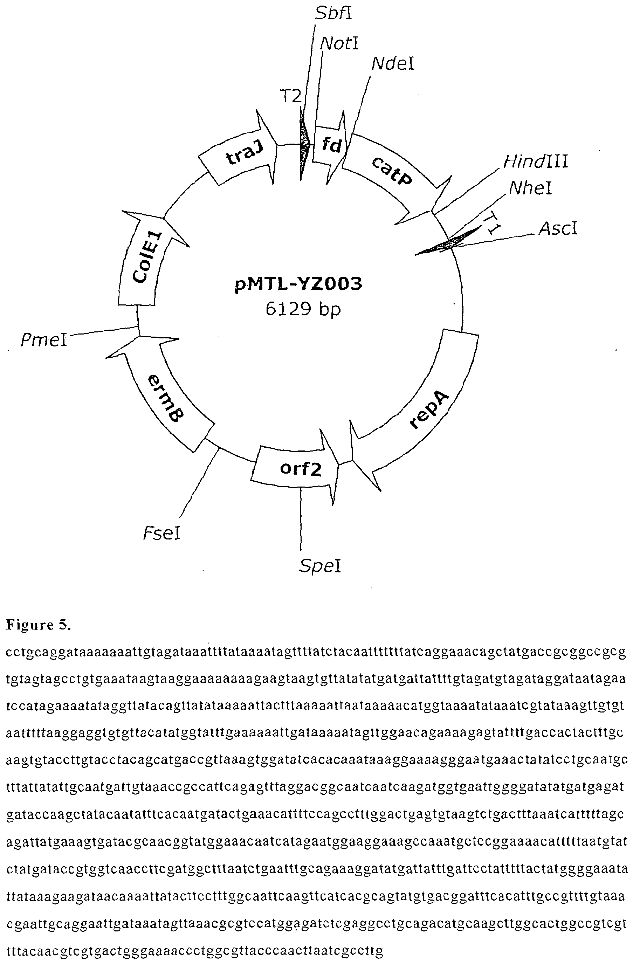

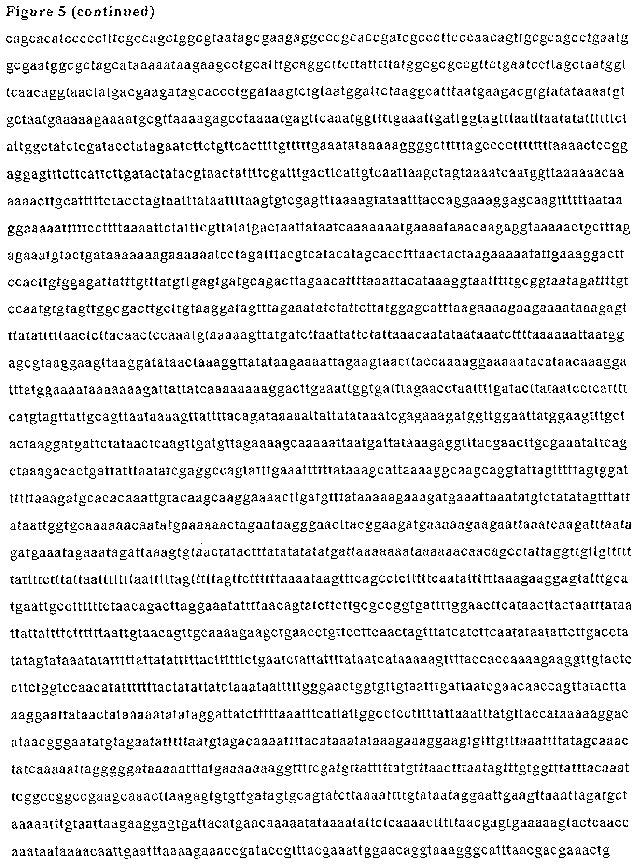

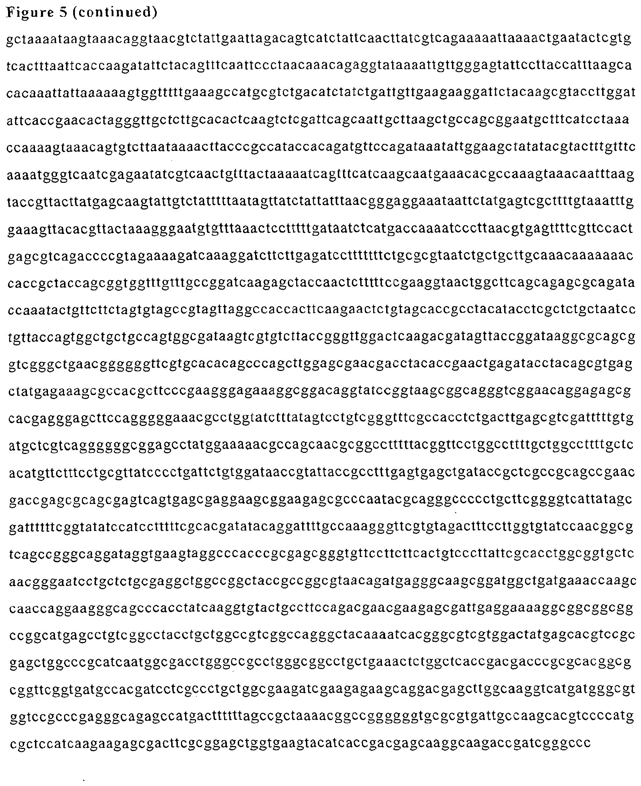

**Figure 5** - shows a schematic view of the plasmid pMTL-YZ003.
   Key: T2, a transcriptional terminator isolated from downstream of the *Clostridium difficile* strain 630 CD0164 gene; fd, the promoter region of the *Clostridium pasteurianum* ferredoxin gene, derivatised to include an operator sequence from the *E.coli* lac operon and designated fac ; the catP, a Clostridium perfringens-derived gene encoding chloramphenicol acetyltransferase; T1 the transcriptional terminator of the *Clostridium pasteurianum* ferredoxin gene; repA and orf2, replication region of the *Clostridium botulinum* plasmid pBP1; ermB, the macrolide-lincosamide-streptogramin B antibiotic resistance gene of plasmid pAMβ1; ColE1, the replication origin of plasmid ColE1, and; traJ, transfer function of the RP4 oriT region.
**Figure 6** - shows CAT activity of either *Clostridium acetobutylicum* ATCC 824 wildtype or strain CRG3011 (carrying *tcdR*) carrying plasmids pMTL-YZ002 (P*_{tcdB}*) and pMTL-YZ003 (P*_{fdx}*).
**Figure 7** - shows a schematic representation of strains generated to produce a recombinant CAZyme through TcdR-mediated expression from the P*_{tcdA}* promoter.
   **[A]** A promoter-less copy of the *tcdR* gene of *Clostridium difficile* strain 630 has been inserted immediately downstream of the *pyrE* gene (CAC0027) of *C*. *acetobutylicum* ATCC 824 wildtype strain by ACE technology resulting in the strain CRG3011. Illustrated are the surrounding genes, and the position of the promoter responsible for expression of *tcdR,* and the position of the *tcdR* RBS. The illustrated terminator is the T1 transcriptional terminator of the *Clostridium pasteurianum* ferredoxin gene.
   **[B]** In the same cell, CAZymes encoding genes were inserted downstream of the thiolase gene (*thl,* CAC2873), together with the promoter of the *tcdA* gene and the *ermB* gene.

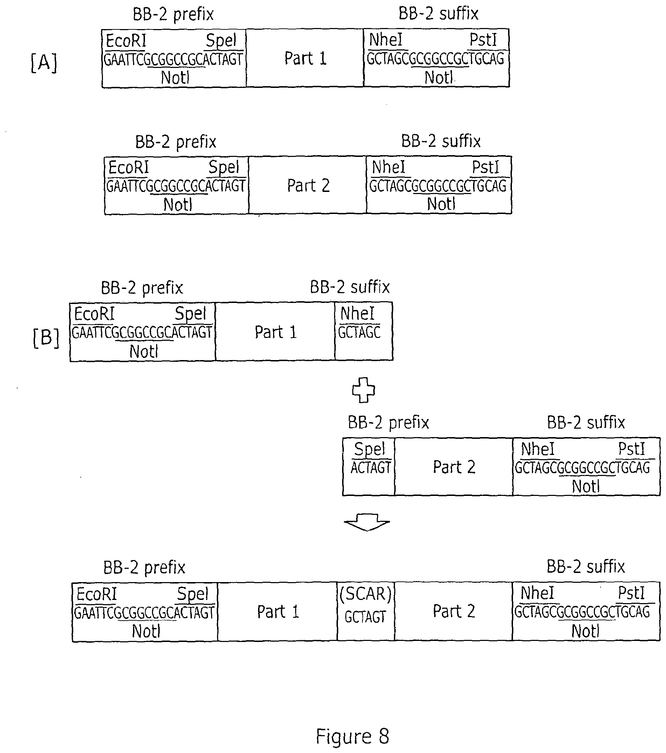

**Figure 8** - shows Assembly of parts in BioBrick-2 standard format.
   Each 'part' is preceded by a nucleotide sequence (prefix) encompassing the restriction enzyme sites EcoRI, NotI and Spel, and followed by a nucleotide sequence (suffix) encompassing the restriction sites Nhel, Notl and PstI. Parts are joined through cleavage of the Part 1 suffix with Nhel and the Part 2 prefix with Spel and the subsequent ligation of the compatible sticky ends generated. Fusion of the two sticky ends, and the joining of part 1 to part 2, results in the 'SCAR' sequence GCTAGT, which can no longer be cleaved by either Nhel or Spel.

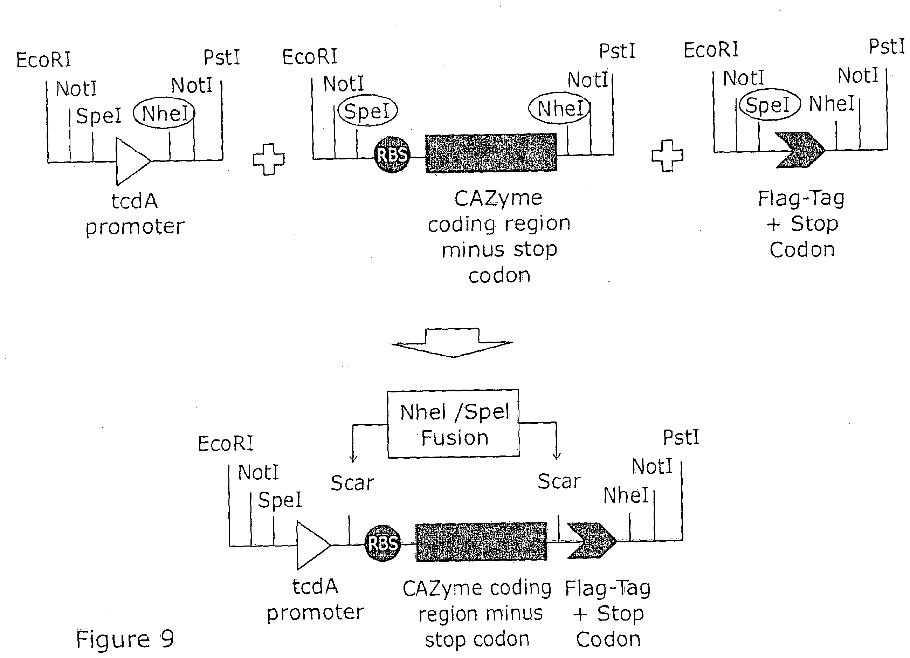

**Figure 9** - shows a schematic representation of the assembly of natural CAZyme encoding modules from 3 BioBrick-2 parts encompassing the *tcdA* promoter, CAZyme coding region, and Flag-Tag and stop codon.
   The three parts are joined using the compatible sticky ends generated by the cleavage of the part 1 (*tcdA* promoter) suffix sequence with Nhel, the part 2 (CAZyme coding region) prefix and suffix sequence with Spel and Nhel, respectively, and the part 3 (Flag-Tag and stop codon) prefix sequence with Spel. Ligation of the 3 generated restriction fragments into one contiguous sequence results in a Nhel/Spel 'SCAR' sequence (GCTAGT) between part 1 and 2, and between part 2 and 3.

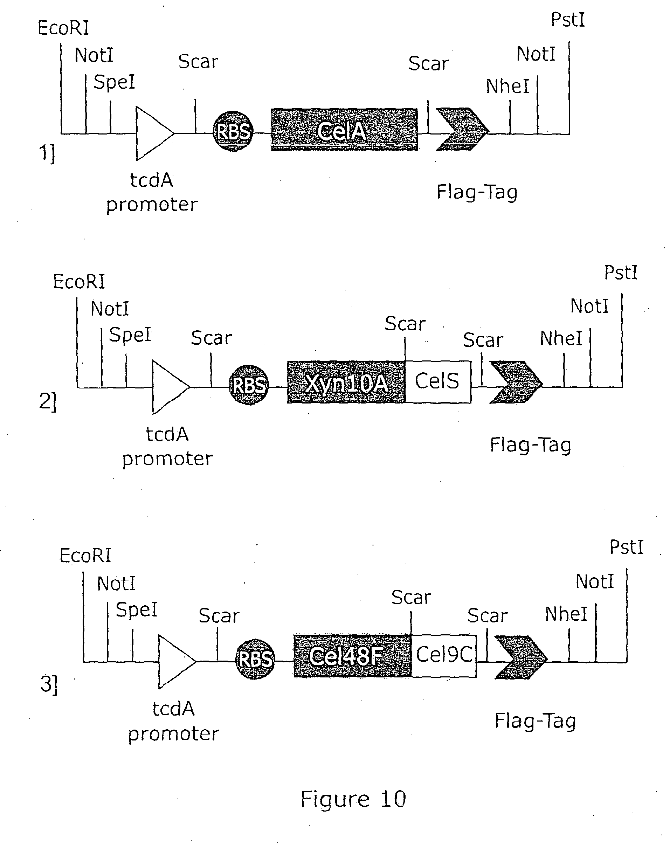

**Figure 10** - shows a schematic representation of the assembly of chimeric CAZyme encoding modules from 2 BioBrick-2 parts, specifying a CAZyme catalytic coding region and a cellulosome dockerin domain coding region. DNA constructs were assembled from re-usable modular parts in a standard format (BioBrick assembly standard 12.

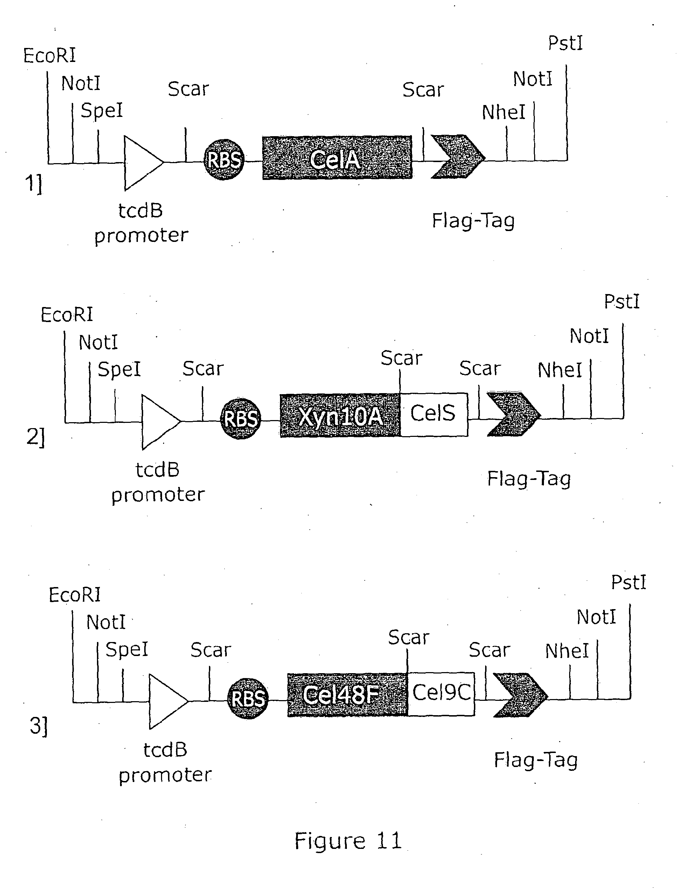

**Figure 11** - shows a schematic representation of the assembled three CAZymes tested. DNA constructs were assembled from re-usable modular parts in a standard format (BioBrick assembly standard 12.

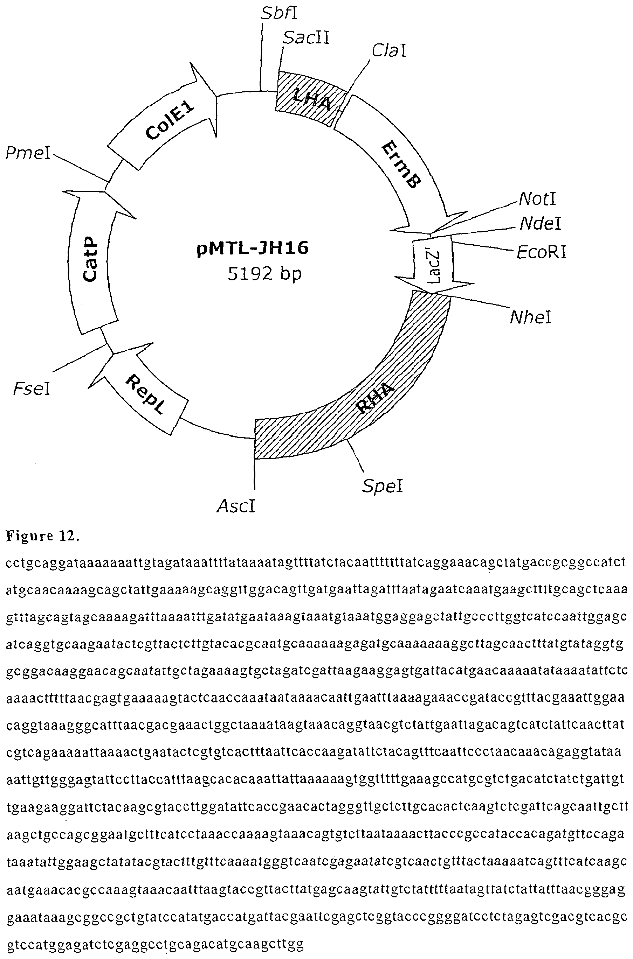

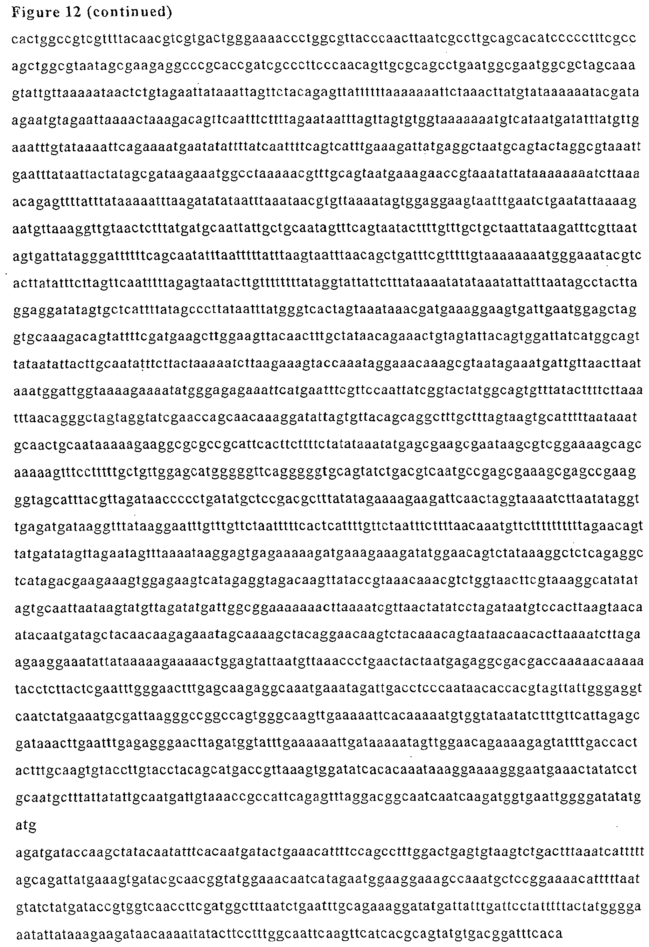

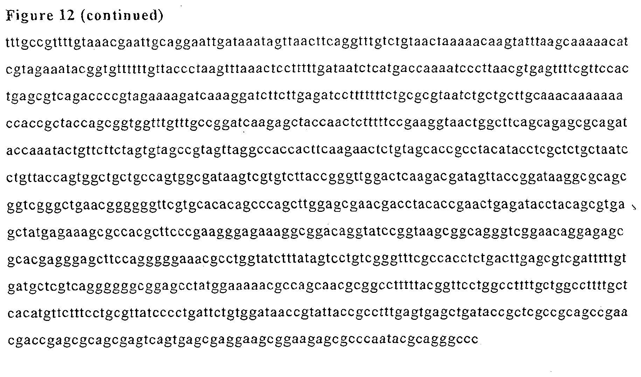

**Figure 12** - shows a schematic representation of the ACE vector PMTL-JH16.
   Key: LHA, left hand homology arm encompassing a foreshortened *Clostridium acetobutylicum* pyrE gene lacking its first 13 codons; LacZ', incorporating multiple cloning sites; T1, transcriptional terminator of the *Clostridium pasteurianum* ferredoxin gene; RHA, right hand homology arm composed of 1200 bp from immediately downstream of the *Clostridium acetobutylicum* pyrE gene, encompassing the hydA gene, CAC0028; RepL, the replication protein of plasmid plM13; CatP, a *Clostridium perfringens-derived* gene encoding chloramphenicol acetyltransferase, and; ColE1, the replication origin of plasmid ColE1.

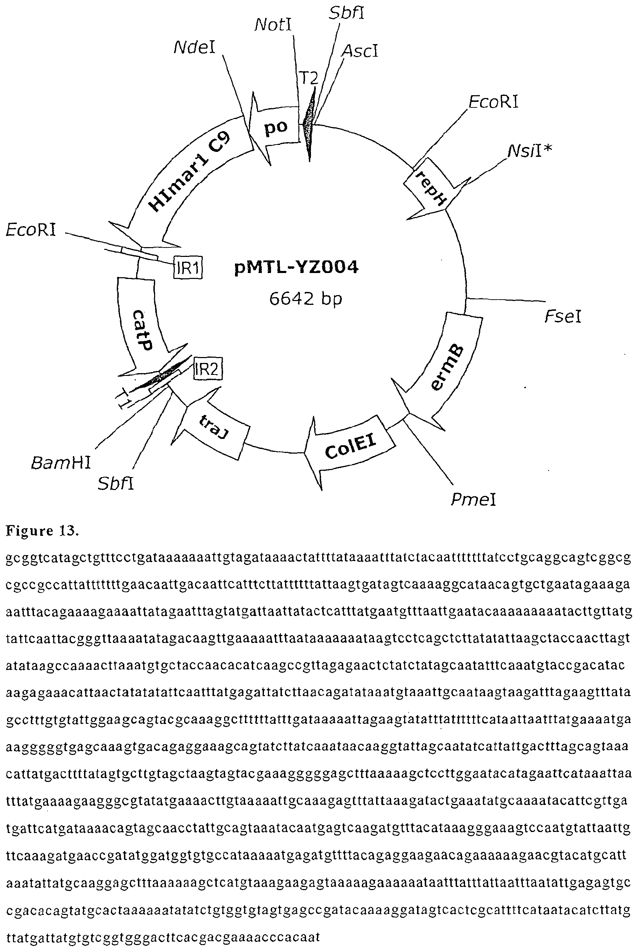

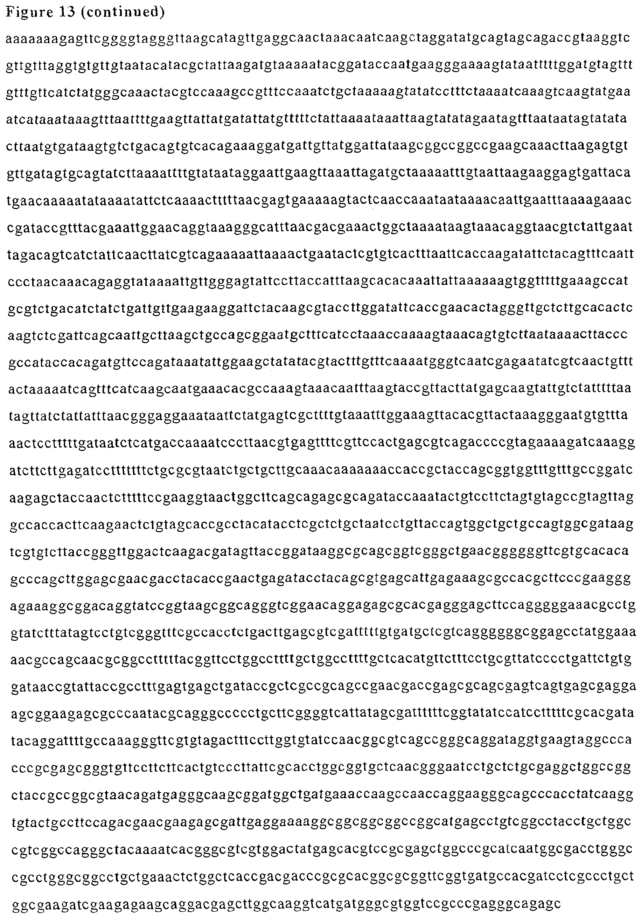

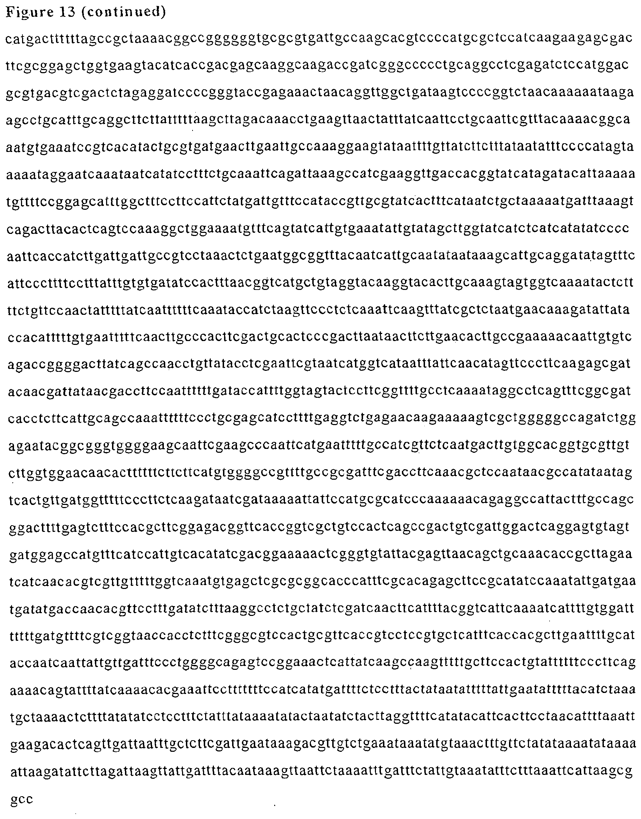

**Figure 13** - shows a schematic representation of the *mariner* transposon plasmid pMTL-YZ004 & pMTL-YZ005
   Plasmid pMTL-YZ004 was made by swopping the region between the AscI and Fsel sites of plasmid pMTL-SC1 which carries the pBP1 replicon with a 1626 bp AscI-FseI fragment from the modular plasmid pMTL83251 which encompasses the replication region of plasmid pCB102. Thereafter, plasmid pMTL-YZ004 was cleaved with Nsil (*), the sticky ends generated blunt-ended by treatment with T4 polymerase, and the linear fragment self-ligated to yield plasmid pMTL-YZ005.
   Key: T2, a transcriptional terminator isolated from downstream of the *Clostridium difficile* strain 630 CD0164 gene; repH, replication region of the *Clostridium butyricum* plasmid pCB102; *, the NsiI site that was blunt-ended to yield plasmid pMTL-YZ005; ermB, the macrolide-lincosamide-streptogramin B antibiotic resistance gene of plasmid pAMβ1; ColE1, the replication origin of plasmid ColE1; traJ, transfer function of the RP4 *oriT* region; IR1 & IR2, inverted repeat regions flanking the mini-transposon element; T1 the transcriptional terminator of the *Clostridium pasteurianum* ferredoxin gene; catP, a *Clostridium perfringens-derived* gene encoding chloramphenicol acetyltransferase, HImar1 C9, mariner transposase gene, and; po, the promoter region of the *Clostridium difficile tcdB* gene.

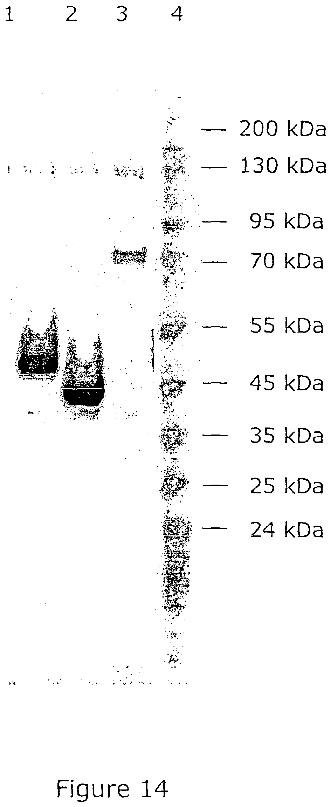

**Figure 14** - shows Western-Blot analysis on native CelA and chimeric Cel48F and Xyn10A expressing *C*. *acetobutylicum* CRG3011 strains.
   Lanes: 1, CelA; 2, Xyn10A; 3, Cel48F, and; 4. Molecular weight marker (P7710S, NEB).

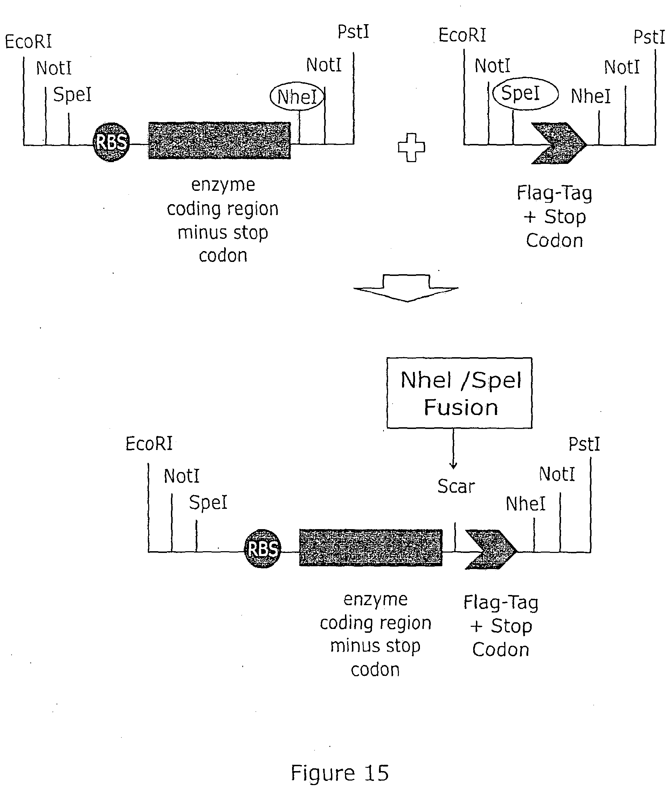

**Figure 15** - shows the assembly of structural genes encoding enzymes involved in solvent production derivatised to include a COOH-terminal Flag-Tag sequence. Following fusion of the two illustrated BioBrick-2 parts (through ligation of the Nhel and Spel sticky ends) the modified gene was inserted into pMTL-JH16 as a Notl-Nhel fragment between the equivalent sites present in the vector. As pMTL-JH16 carries a transcriptional terminator sequence immediately after the Nhel site (at the proximal end of the RHA), every construct is followed by a transcriptional termination signal.

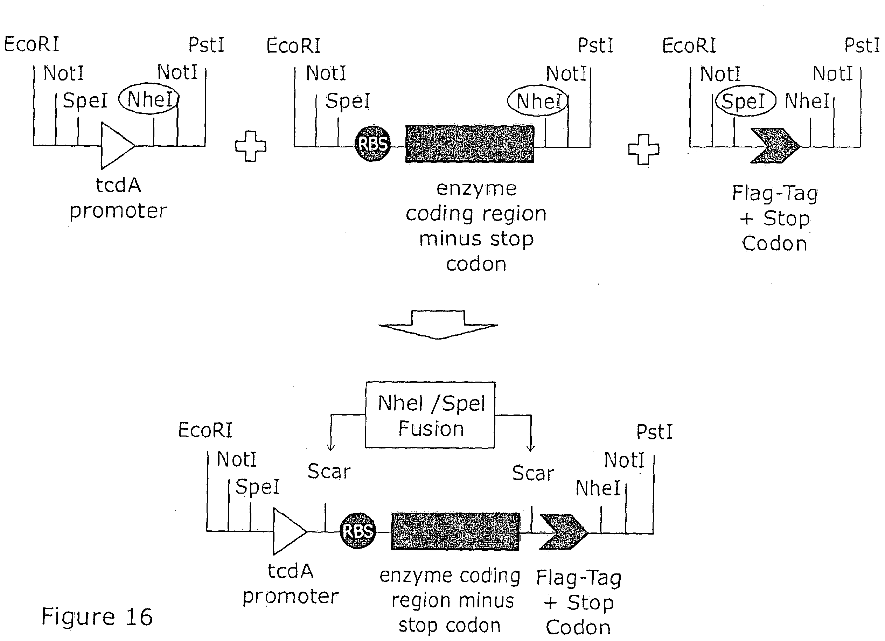

**Figure 16** - shows assembly of structural genes encoding enzymes involved in solvent production together with a Flag-Tag and *tcdA* promoter.

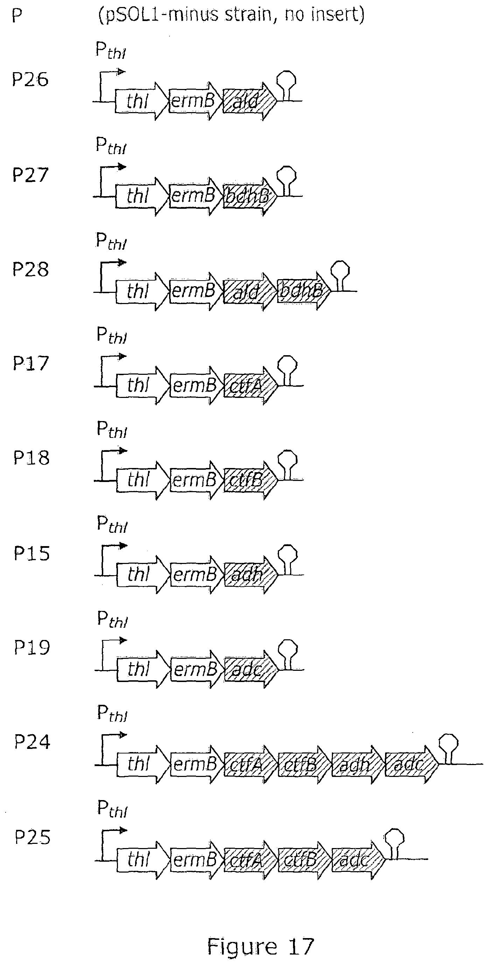

**Figure 17** - shows a schematic representation of the strains generated carrying the various genes encoding enzymes involved in solvent production at the thiolase locus of the genome. Each gene was derivatised to include a COOH-terminal Flag-Tag. The position of the thiolase gene promoter *(Pₜₕₗ)* is indicated, as is the position of the transcriptional terminator at the end of each operon present in the genome immediately 5' to the downstream CAC2872 gene. Genes are:- *thl,* thiolase; *ermB,* erythromycin ribosomal methylase B; aid, coenzyme A-acylating aldehyde dehydrogenase; *bdhB,* NADH-dependent butanol dehydrogenase B; *ctfA,* acetoacetyl-CoA transferase subunit A; *ctfB_{.}* acetoacetyl-CoA transferase subunit B; adc, acetoacetate decarboxylase, and; *adh*, alcohol dehydrogenase.

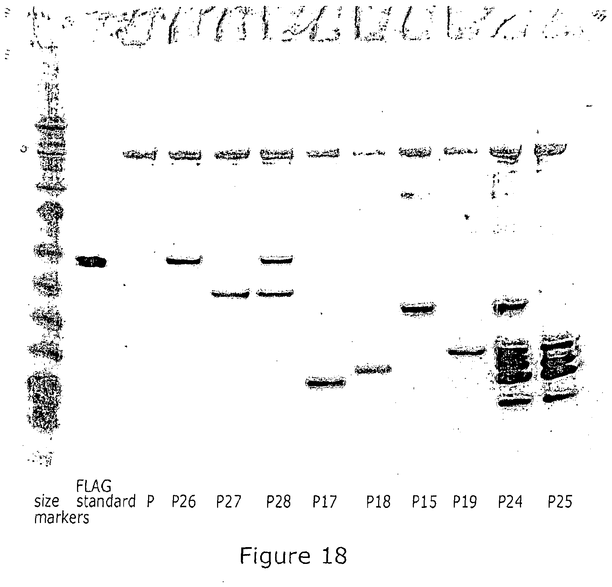

**Figure 18** - shows Western Blots of the Various Synthetic Operon ACE insertion strains.

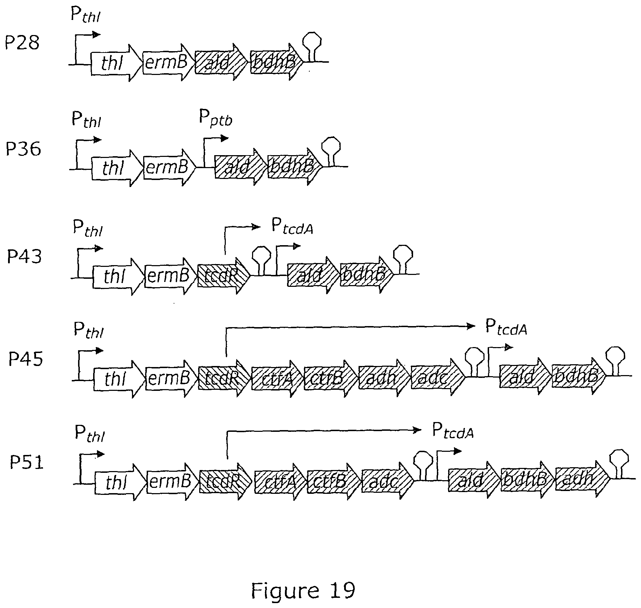

**Figure 19** - shows a schematic representation of additional synthetic operons inserted into the *Clostridium acetobutylicum* chromosome. Each gene was derivatised to include a COOH-terminal Flag-Tag. The position of the thiolase gene promoter (P*ₜₕₗ*) is indicated, as is the position of the transcriptional terminator at the end of each operon present in the genome immediately 3' to the downstream CAC0028 gene (encoding hydrogenase, *hydA*)*.* The position of the promoter (Pt*cdA*) of the *tcdA* gene is also shown. Genes are:- *thl,* thiolase; *ermB,* erythromycin ribosomal methylase B, *ald*, coenzyme A-acylating aldehyde dehydrogenase; *bdhB,* NADH-dependent butanol dehydrogenase B; *ctfA,* acetoacetyl-CoA transferase subunit A; *ctfB,* acetoacetyl-CoA transferase subunit B; *adc*, acetoacetate decarboxylase; adh, alcohol dehydrogenase, and; *tcdR,* RNA Polymerase Sigma factor of the *Clostridium difficile* σ70 family.

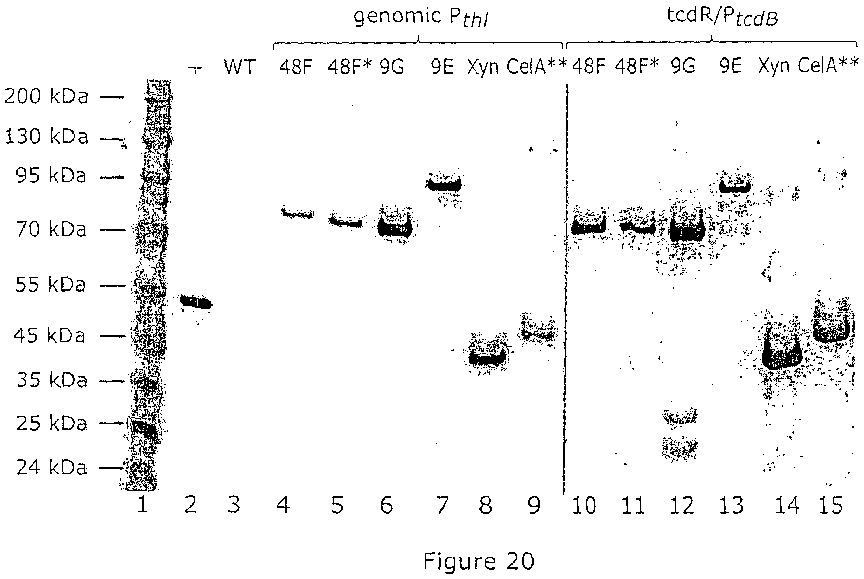

**Figure 20** - shows Western-Blot analysis on native and chimeric CAZymes expressing wild type *C*. *acetobutylicum* and CRG3011 strains.
   Lanes: 1, Molecular weight marker (P7710S, NEB); 2, Flag-tag positive control; 3, wild type *C*. *acetobutylicum* ; 4, wild type *C*. *acetobutylicum* expressing Cel48F with dockerin domain CelS under the transcriptional control of *thl* promoter; 5, wild type *C*. *acetobutylicum* expressing Cel48F with dockerin domain Cel9C under the transcriptional control of thl promoter; 6, wild type *C*. *acetobutylicum* expressing Cel9G with dockerin domain CelS under the transcriptional control of *thl* promoter; 7, wild type *C*. *acetobutylicum* expressing Cel9E with dockerin domain CelS under the transcriptional control of thl promoter; 8, wild type *C*. *acetobutylicum* expressing Xyn10A with dockerin domain CelS under the transcriptional control of *thl* promoter; 9, wild type *C*. *acetobutylicum* expressing CelA with dockerin domain CelA under the transcriptional control of *thl* promoter; 10, *C*. *acetobutylicum* CRG3011 strain expressing Cel48F with dockerin domain CelS under the transcriptional control of *tcdB* promoter; 11, *C*. *acetobutylicum* CRG3011 strain expressing Cel48F with dockerin domain Cel9C under the transcriptional control of *tcdB* promoter; 12, *C*. *acetobutylicum* CRG3011 strain expressing Cel9G with dockerin domain CelS under the transcriptional control of *tcdB* promoter; 13, *C*. *acetobutylicum* CRG3011 strain expressing Cel9E with dockerin domain CelS under the transcriptional control of *tcdB* promoter; 14, *C*. *acetobutylicum* CRG3011 strain expressing Xyn10A with dockerin domain CelS under the transcriptional control of *tcdB* promoter; 15, *C*. *acetobutylicum* CRG3011 strain expressing CelA with dockerin domain CelA under the transcriptional control of *tcdB* promoter.

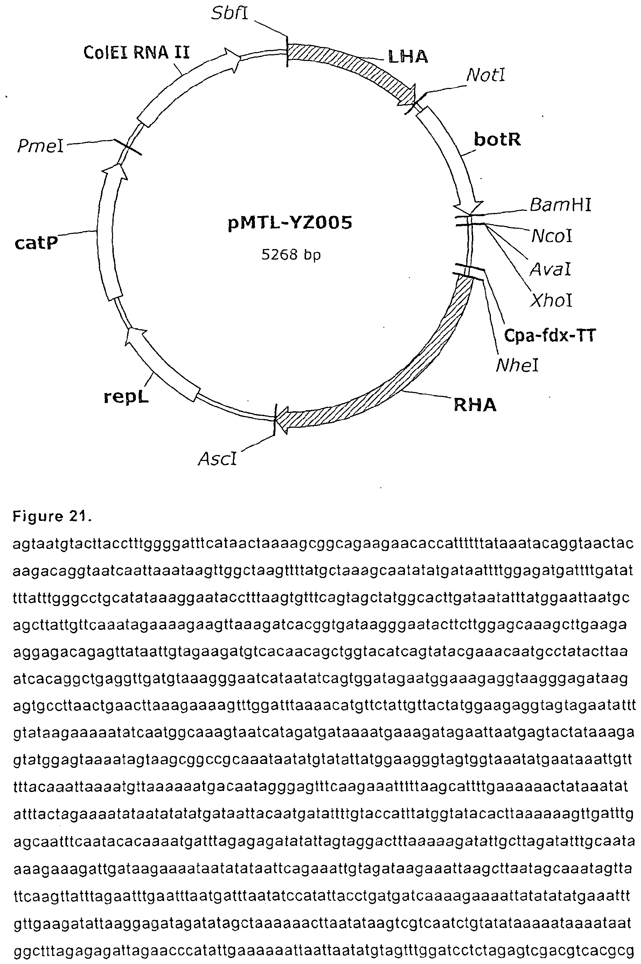

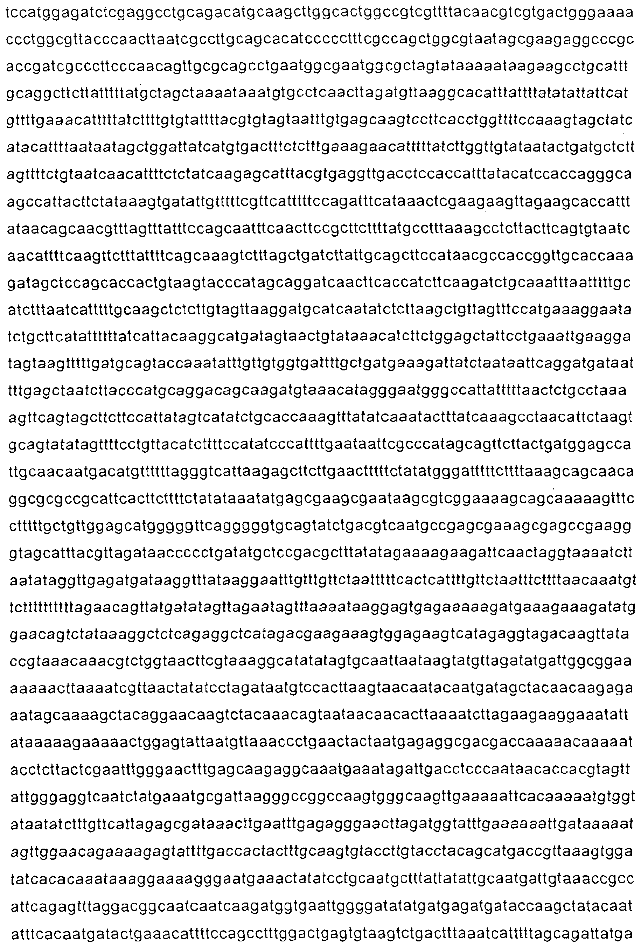

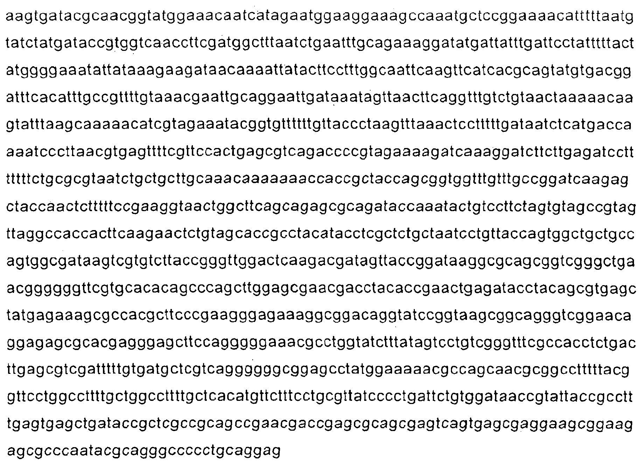

**Figure 21** - shows a schematic view of the plasmid pMTL-YZ005
   Key: LHA, left hand homology arm encompassing a foreshortened *Clostridium acetobutylicum pyrE* gene lacking its first 13 codons; *botR,* derived from *Clostridium botulinum* strain ATCC 3502 and encoding an alternative RNA polymerase sigma-factor assigned to group 5 of the sigma (70) family; Cpa fdx TT, transcriptional terminator of the *Clostridium pasteurianum* ferredoxin gene; RHA, right hand homology arm composed of 1200 bp from immediately downstream of the *Clostridium acetobutylicum pyrE* gene, encompassing the *hydA* gene, CAC0028; RepL, the replication protein of plasmid plM13; *catP,* a *Clostridium perfringens-derived* gene encoding chloramphenicol acetyltransferase, and; ColE1, the replication origin of plasmid ColE1.

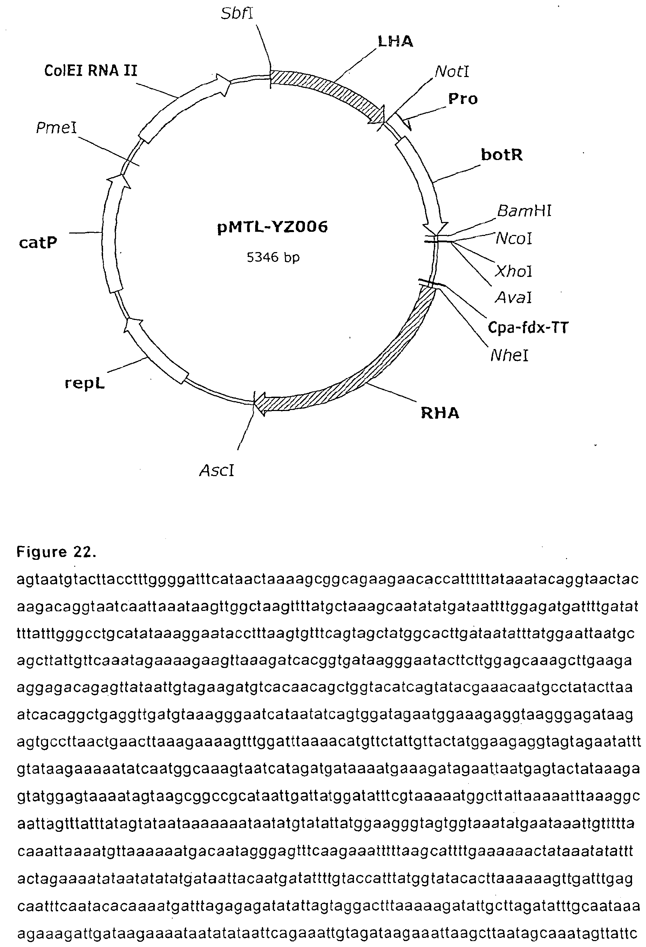

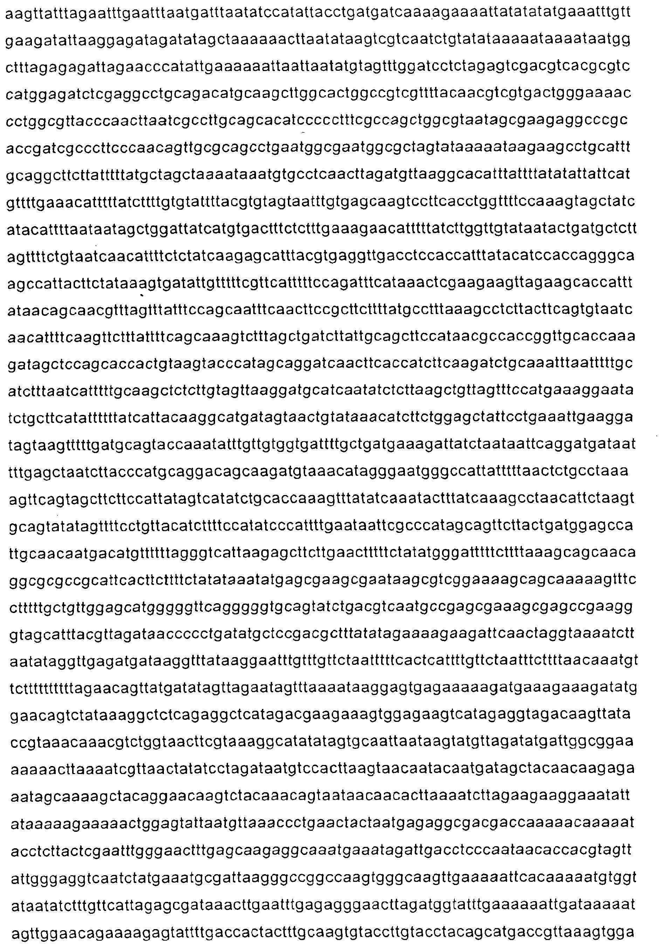

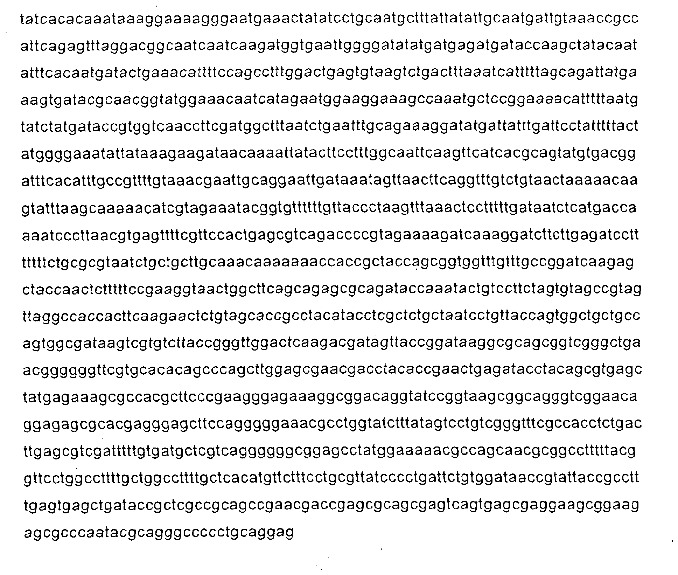

**Figure 22** - shows a schematic view of the plasmid pMTL-YZ006
   Key: LHA, left hand homology arm encompassing a foreshortened *Clostridium acetobutylicum pyrE* gene lacking its first 13 codons; Pro, native promoter of *botR*; *botR ,* derived from *Clostridium botulinum strain ATCC 3502* and encoding an alternative RNA polymerase sigma-factor assigned to group 5 of the sigma (70) family; Cpa fdx TT, the transcriptional terminator of the *Clostridium pasteurianum* ferredoxin gene; RHA, right hand homology arm composed of 1200 bp from immediately downstream of the *Clostridium acetobutylicum pyrE* gene, encompassing the *hydA* gene, CAC0028; RepL, the replication protein of plasmid plM13; *catP,* a *Clostridium perfringens-derived* gene encoding chloramphenicol acetyltransferase, and; ColE1, the replication origin of plasmid ColE1.

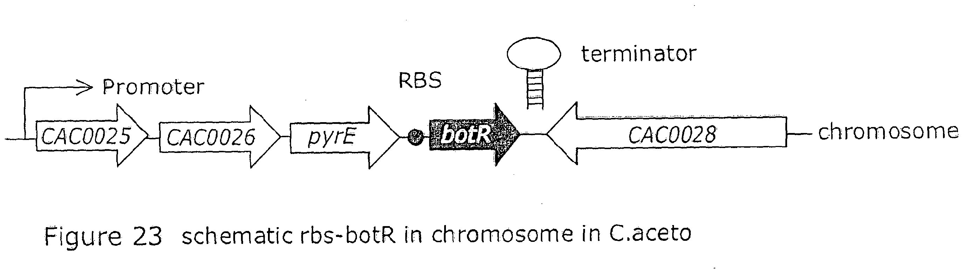

**Figure 23** - shows a schematic representation of the genome of strain CRG3755. A promoter-less copy of the *botR* gene (including its ribosome binding site, RBS) of *Clostridium botulinum* strain ATCC 3502 has been inserted into the genome using ACE technology immediately downstream of the *pyrE* gene (CAC0027). Illustrated are the surrounding genes, and the position of the promoter responsible for expression of *botR,* and the position of the *botR* RBS. The illustrated terminator is the transcriptional terminator of the pasteurianum ferredoxin gene.

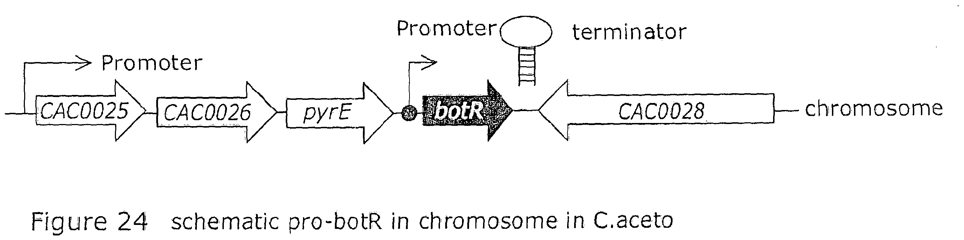

**Figure 24** - shows a schematic representation of the genome of strain CRG3756. The botR gene including its ribosome binding site, RBS, and promoter, of *Clostridium botulinum* strain ATCC 3502 has been inserted into the genome using ACE technology immediately downstream of the *pyrE* gene (CAC0027). Illustrated are the surrounding genes, and the position of the promoter responsible for expression of *botR,* and the position of the botR RBS. The illustrated terminator is the transcriptional terminator of the *Clostridium pasteurianum* ferredoxin gene.

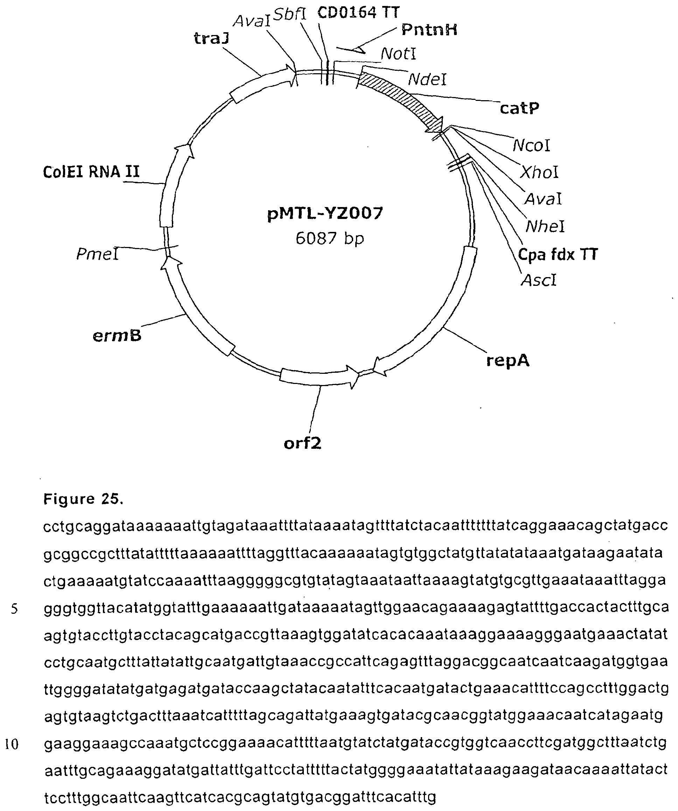

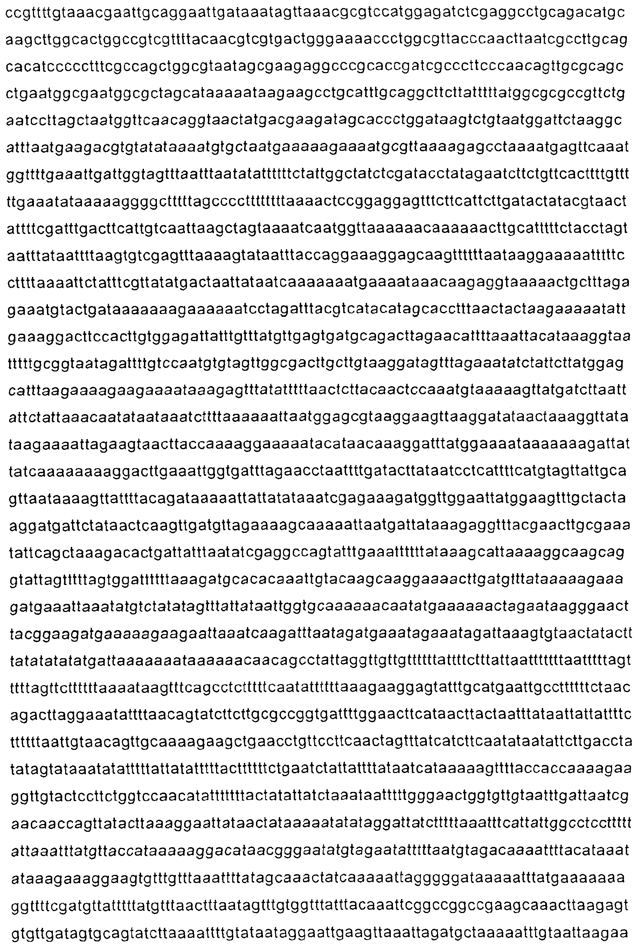

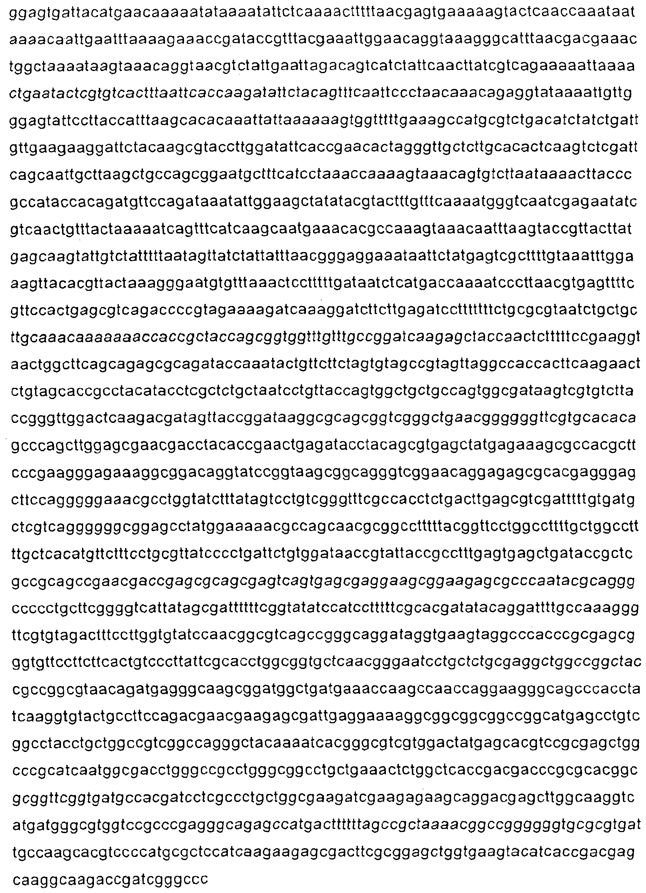

**Figure 25** - shows a schematic view of the plasmid pMTL-YZ007.
   Key: CD0164 TT, transcriptional terminator descented downstream of the *Clostridium difficile* strain 630 CD0164 gene; P*_{ntnH}*, the promoter region of the *Clostridium botulinum ntnH* gene; *catP,* a *Clostridium perfringens-derived* gene encoding chloramphenicol acetyltransferase; Cpa fdx TT, the transcriptional terminator of the *Clostridium pasteurianum* ferredoxin gene; *repA* and orf2, replication region of the *Clostridium botulinum* plasmid pBP1; *ermB,* the macrolidelincosamide-streptogramin B antibiotic resistance gene of plasmid pAMβ1; ColE1, the replication origin of plasmid ColE1, and; *traJ,* transfer function of the RP4 *oriT* region.

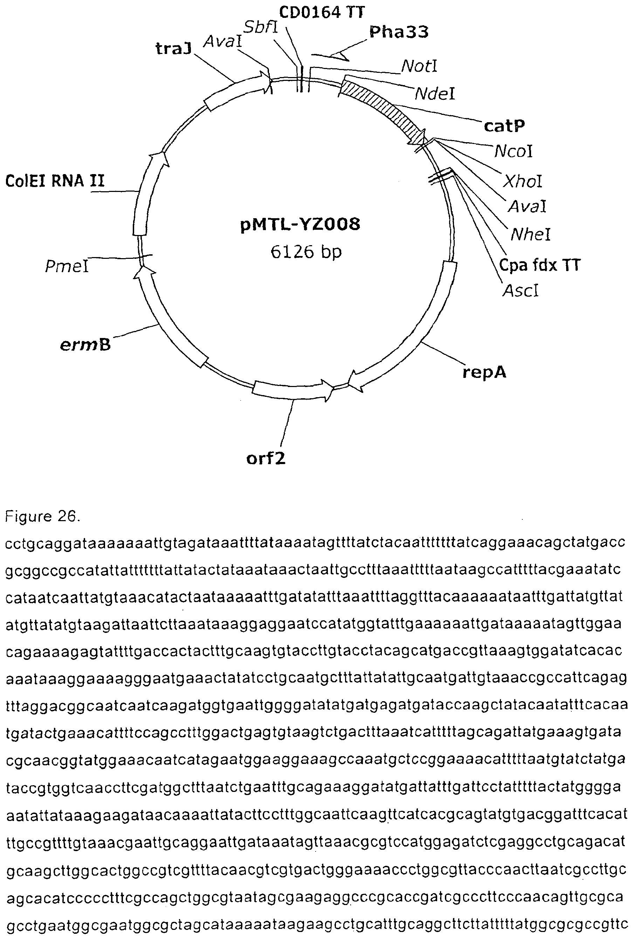

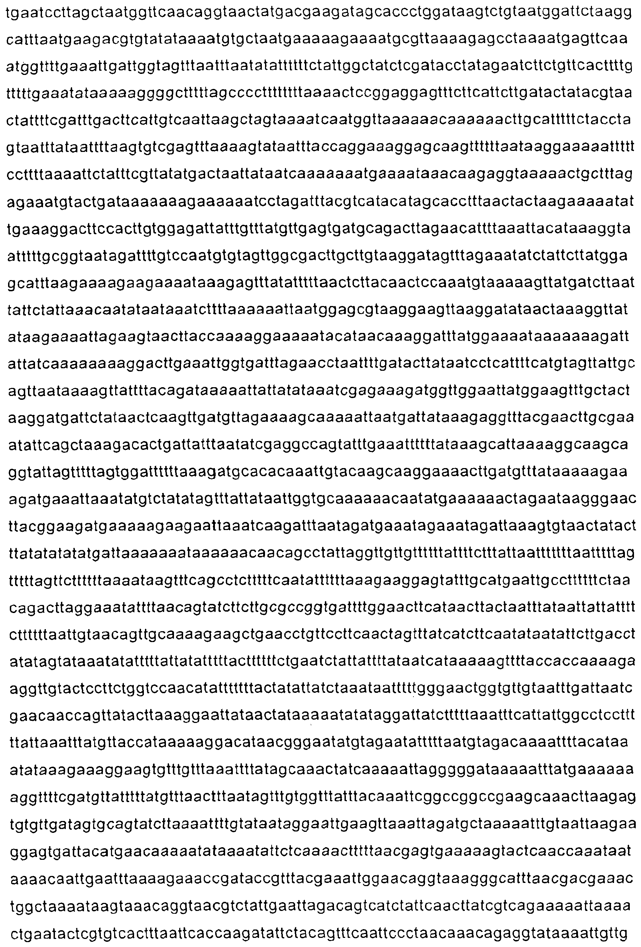

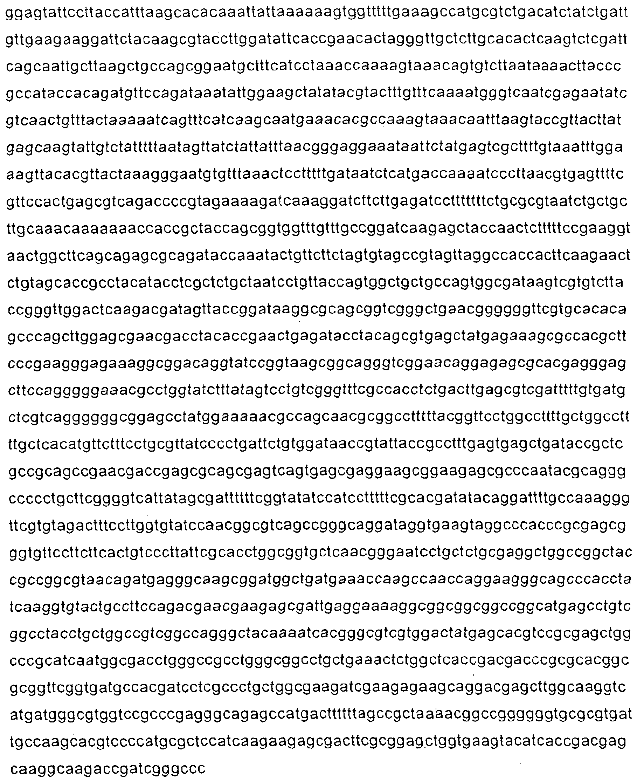

**Figure 26** - shows a schematic view of the plasmid pMTL-YZ008.
   Key: CD0164 TT, a transcriptional terminator isolated from downstream of the Clostridium difficile strain 630 CD0164 gene; P*ₕₐ₃₄*, the promoter region of the *Clostridium botulinum ha34* gene; the *catP,* a *Clostridium perfringens-derived* gene encoding chloramphenicol acetyltransferase; Cpa fdx TT, the transcriptional terminator of the *Clostridium pasteurianum* ferredoxin gene; *repA* and orf2, replication region of the *Clostridium botulinum* plasmid pBP1; *ermB,* the macrolide-lincosamide-streptogramin B antibiotic resistance gene of plasmid pAMβ1; ColE1, the replication origin of plasmid ColE1, and; *traJ,* transfer function of the RP4 oriT region.

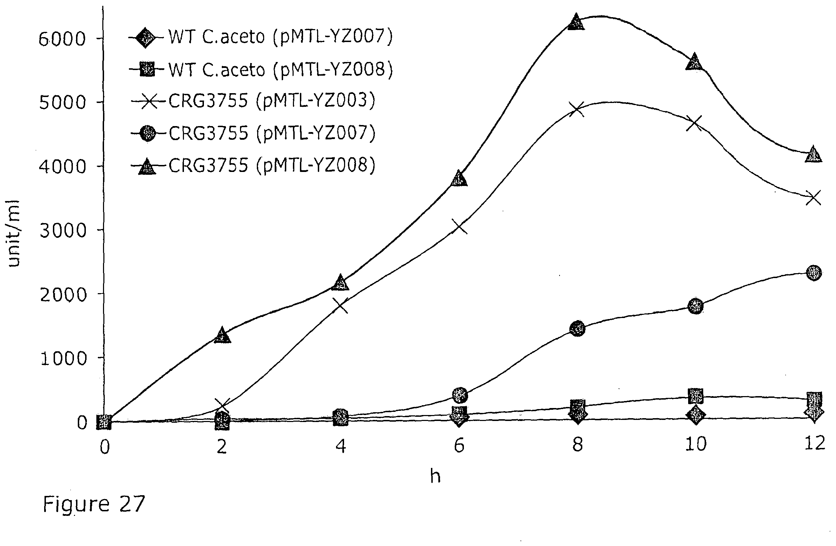

**Figure 27** - shows CAT activity of either *Clostridium acetobutylicum* ATCC 824 wildtype or strain CRG3755 (carrying *botR*) carrying plasmids pMTL-YZ007 (P*_{ntnH}*), pMTL-YZ008 (P*ₕₐ₃₄*), and pMTL-YZ003 (P*_{fdx}*).

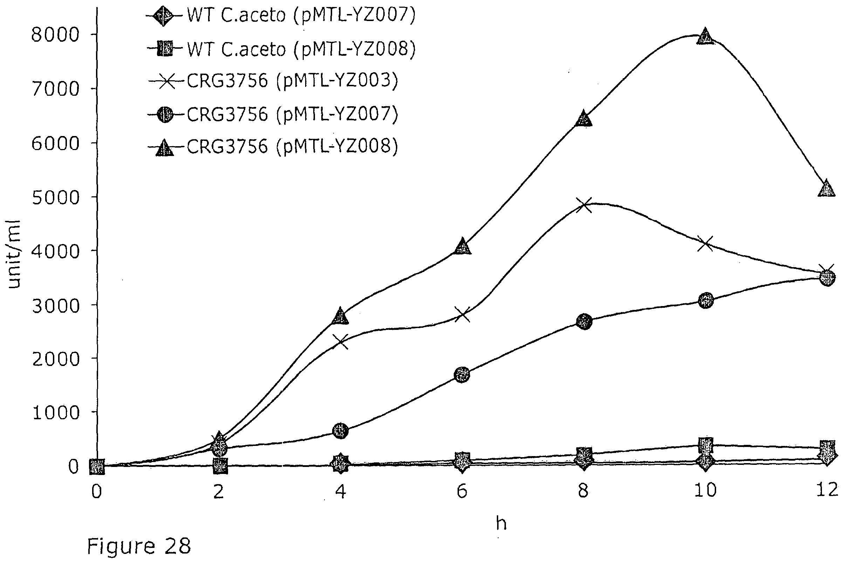

**Figure 28** - shows CAT activity of either *Clostridium acetobutylicum* ATCC 824 wildtype or strain CRG3756 (carrying *botR* and its native promoter) carrying plasmids pMTL-YZ007 (P*_{ntnH}*), pMTL-YZ008 (P*ₕₐ₃₃*), and pMTL-YZ003 (P*_{fdx}*).

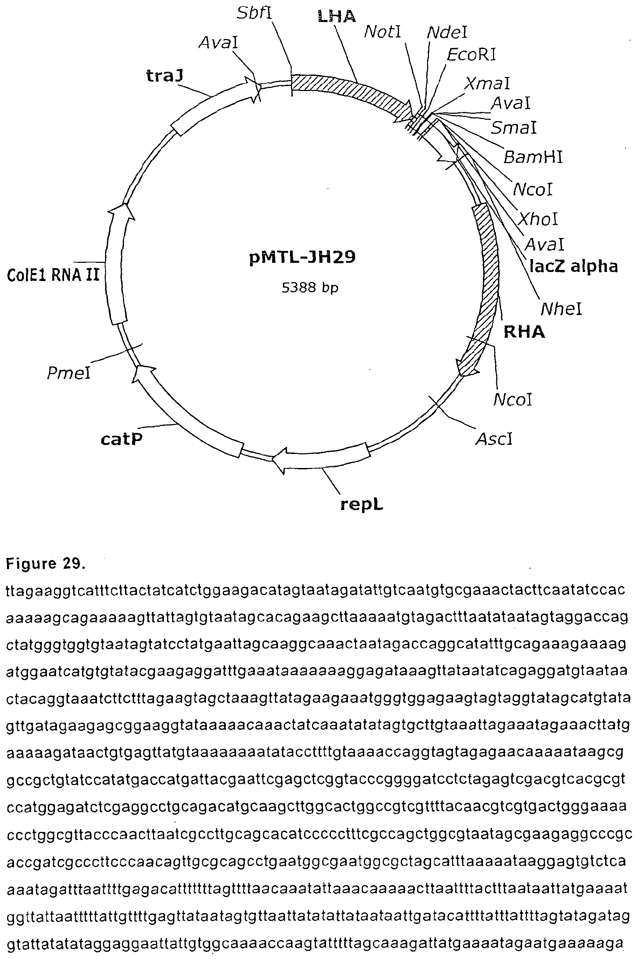

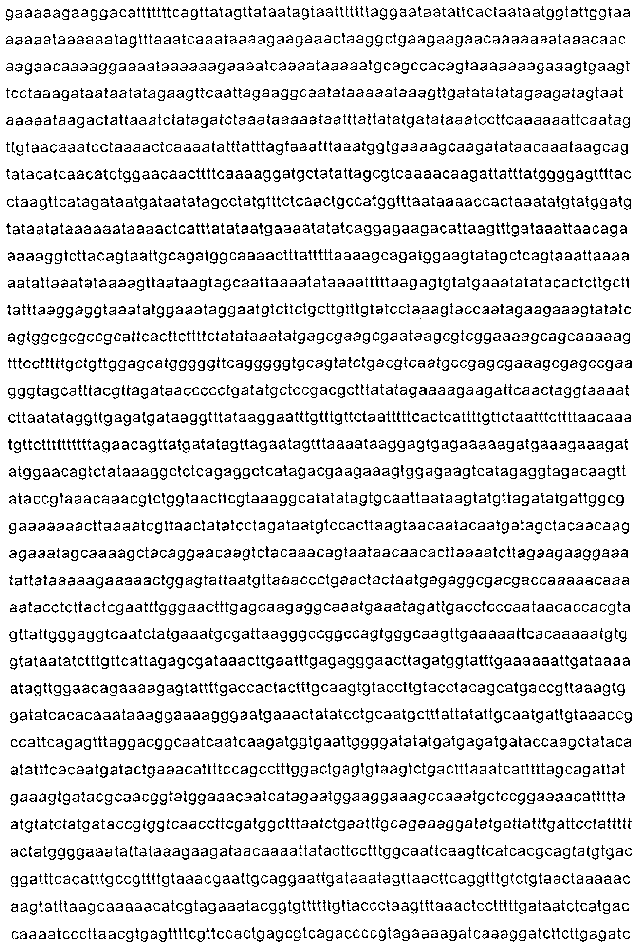

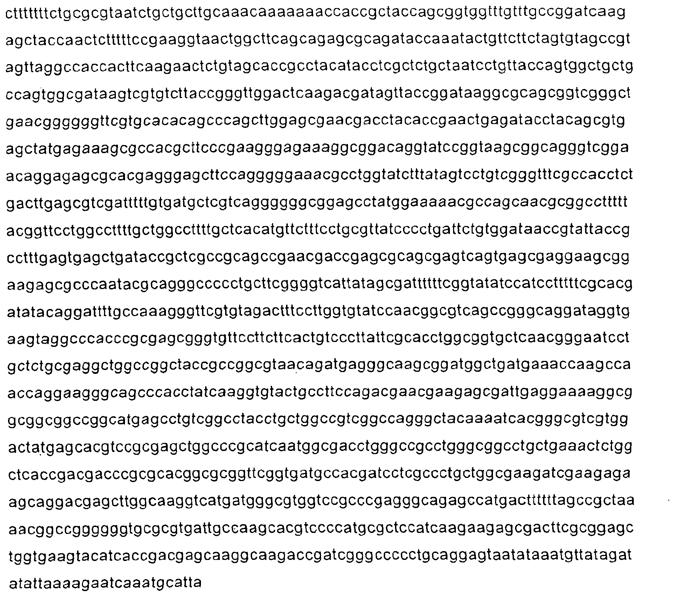

**Figure 29** - shows a schematic representation of the ACE vector PMTL-JH29.
   Key: LHA, left hand homology arm encompassing a foreshortened *Clostridium sporogenes* pyrE gene lacking its first 13 codons; LacZ', incorporating multiple cloning sites; T1, transcriptional terminator of the *Clostridium pasteurianum* ferredoxin gene; RHA, right hand homology arm composed of 1200 bp from immediately downstream of the *Clostridium sporogenes pyrE* gene, encompassing the CS3234 gene; RepL, the replication protein of plasmid pIM13; CatP, a *Clostridium perfringens-derived* gene encoding chloramphenicol acetyltransferase, and; ColE1, the replication origin of plasmid ColE1, and; *traJ,* transfer function of the RP4 *oriT* region.

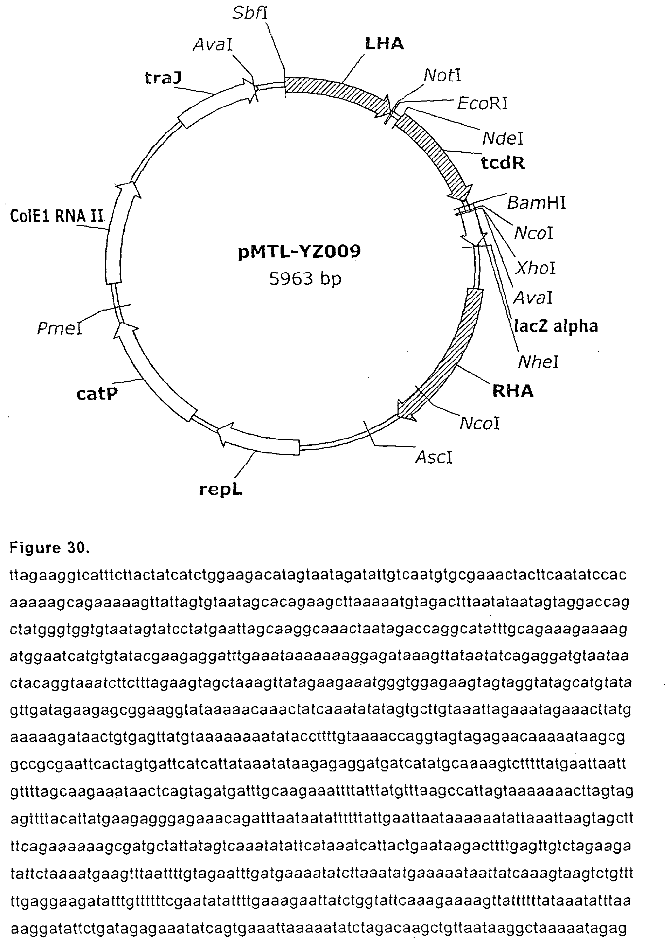

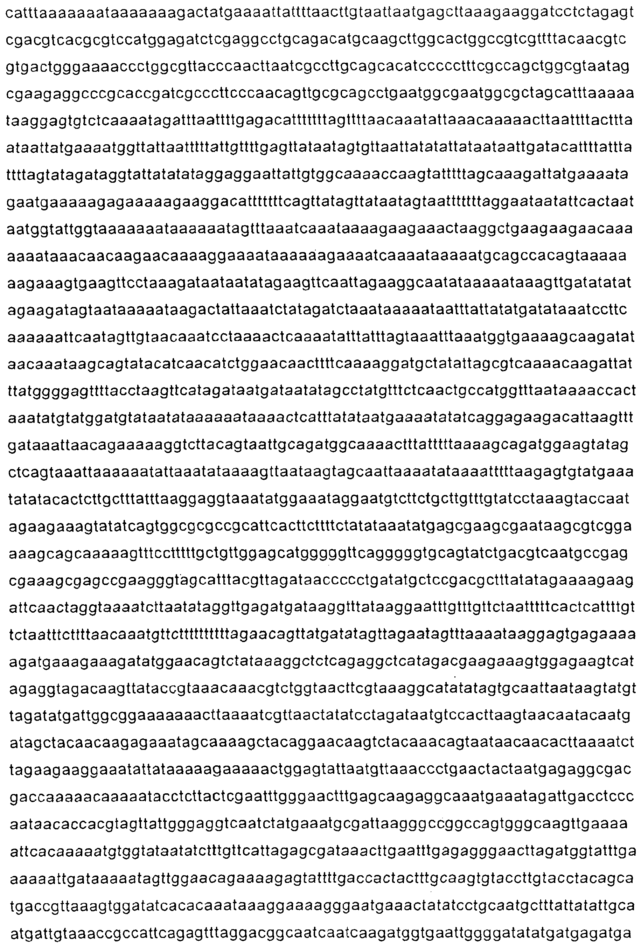

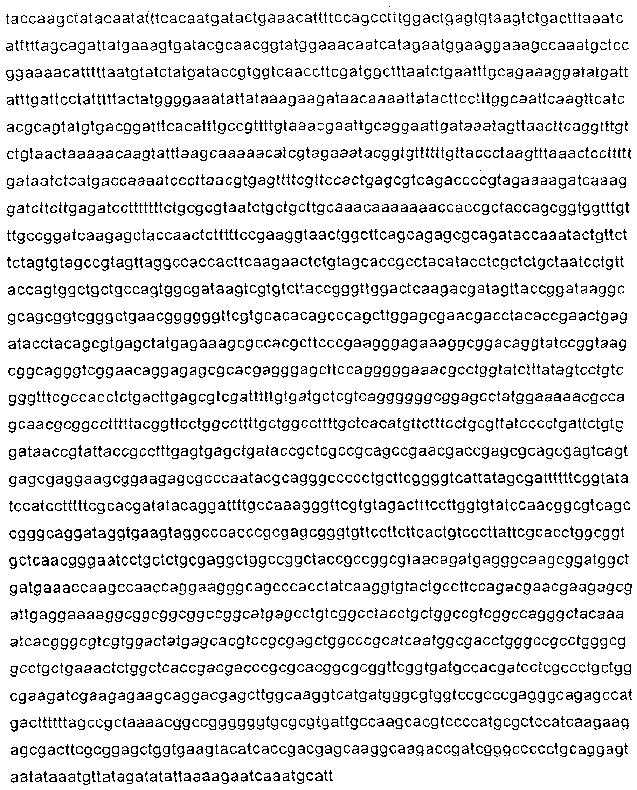

**Figure 30****-** shows a schematic view of the plasmid PMTL-YZ009.
   Key: LHA, left hand homology arm encompassing a foreshortened *Clostridium sporogenes* pyrE gene lacking its first 13 codons; *tcdR,* derived from *Clostridium difficile* strain 630 and encoding an alternative RNA polymerase sigma-factor assigned to group 5 of the sigma (70) family; T1, transcriptional terminator of the *Clostridium pasteurianum* ferredoxin gene; RHA, right hand homology arm composed of 1200 bp from immediately downstream of the *Clostridium sporogenes* pyrE gene, encompassing the CS3234 gene; RepL, the replication protein of plasmid plM13; CatP, a *Clostridium perfringens-derived* gene encoding chloramphenicol acetyltransferase, and; ColE1, the replication origin of plasmid ColE1, and; *traJ,* transfer function of the RP4 oriT region.

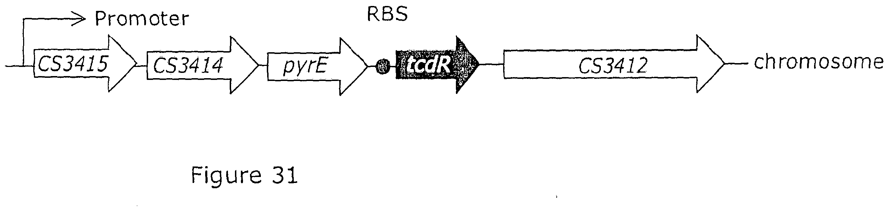

**Figure 31** - shows a schematic representation of the genome of *Clostridium sporogenes* strain CRG3817. A promoter-less copy of the *tcdR* gene (including its ribosome binding site, RBS) of *Clostridium difficile* strain 630 has been inserted into the genome using ACE technology immediately downstream of the *pyrE* gene (CS3235). Illustrated are the surrounding genes, and the position of the promoter responsible for expression of tcdR, and the position of the *tcdR* RBS. The illustrated terminator is the T1 transcriptional terminator of the *Clostridium pasteurianum* ferredoxin gene.

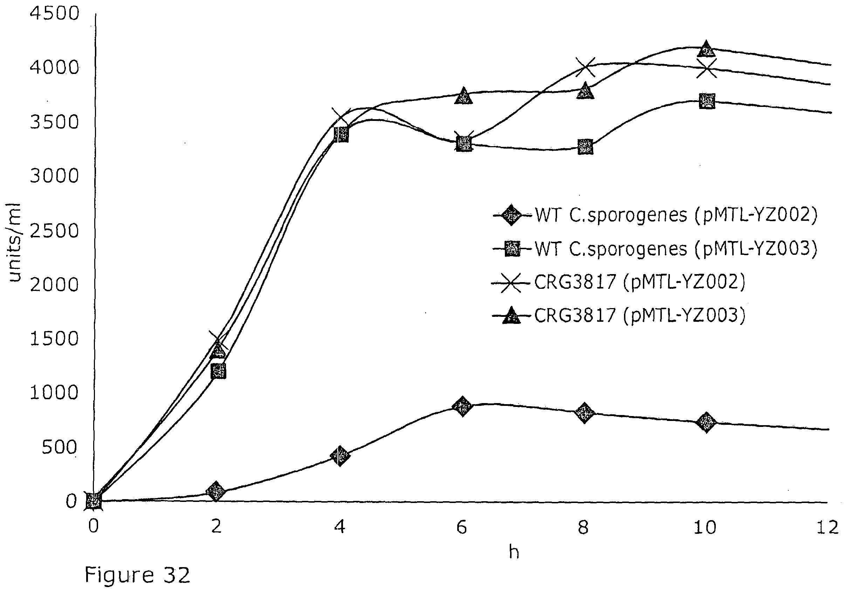

**Figure 32** - shows CAT activity of either *Clostridium sporogenes* NCIMB 10696 wildtype or strain CRG3817 (carrying *tcdR*) carrying plasmids pMTL-YZ002 (P*_{tcdB}*), and pMTL-YZ003 (P*_{fdx}*).

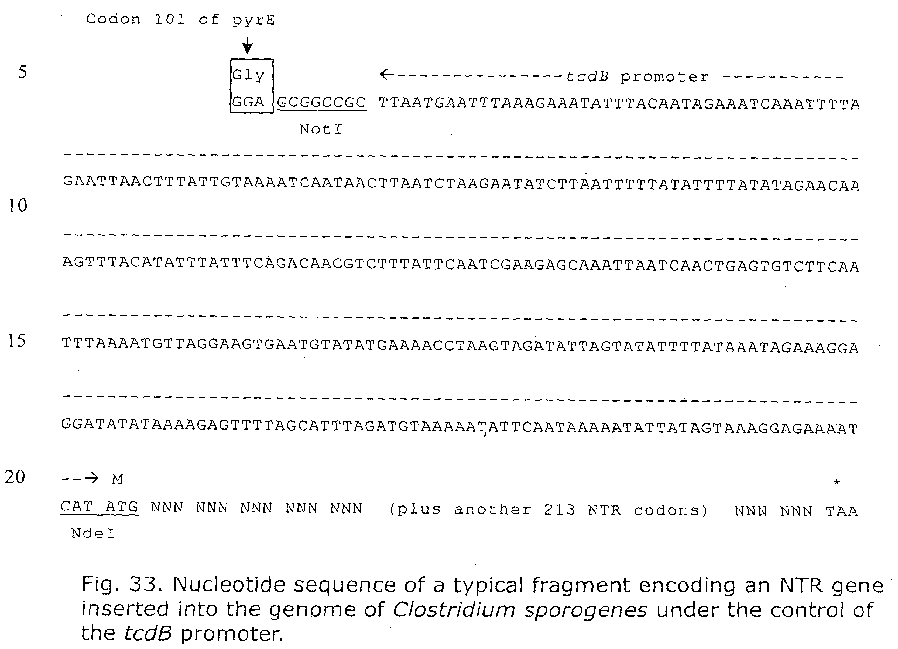

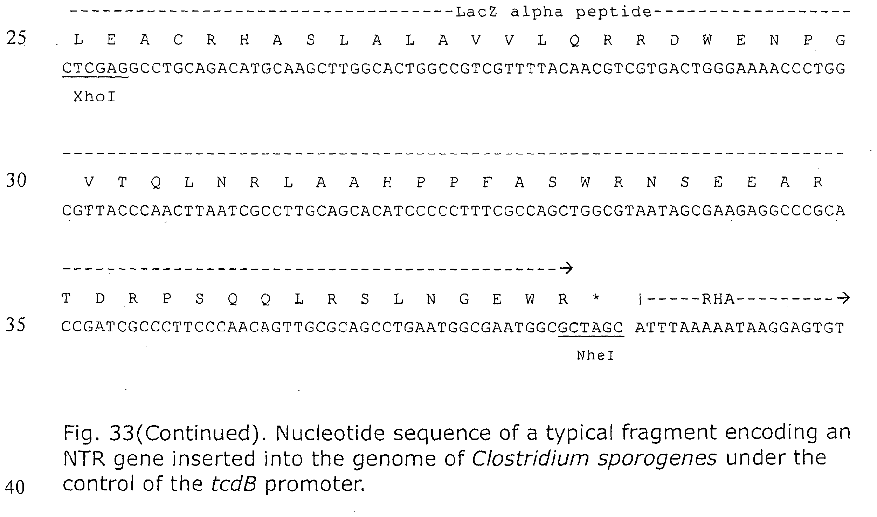

**Figure 33** - shows the nucleotide sequence of a typical fragment encoding an NTR gene inserted into the genome of *Clostridium sporogenes* under the control of the *tcdB* promoter.

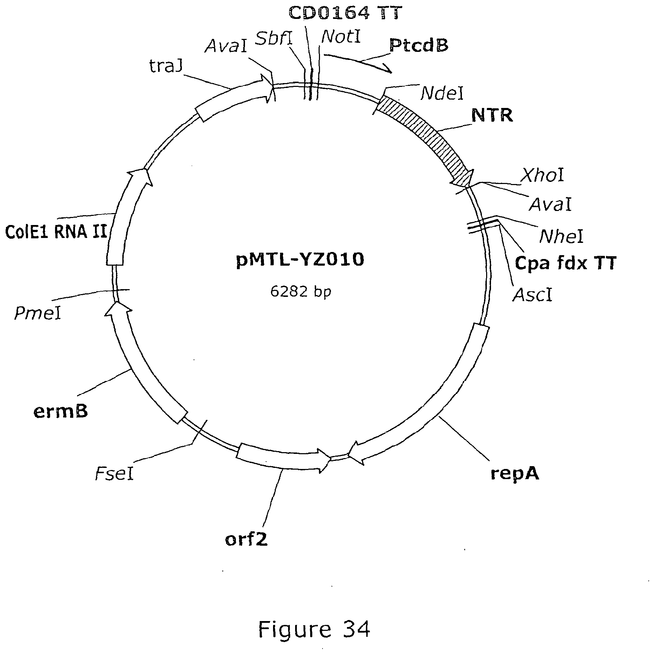

**Figure 34** - shows a schematic view of the plasmid pMTL-YZ010.
   Key: CD0164 TT, transcriptional terminator descented downstream of the *Clostridium difficile* strain 630 CD0164 gene; po, the promoter region of the *Clostridium difficile tcdB* gene; *NTR,* a bacterial nitroreductase; Cpa fdx TT, transcriptional terminator of the *Clostridium pasteurianum* ferredoxin gene; *repA* and orf2, replication region of the *Clostridium botulinum* plasmid pBP1; *ermB,* the macrolidelincosamide-streptogramin B antibiotic resistance gene of plasmid pAMβ1; ColE1, the replication origin of plasmid ColE1, and; *traJ,* transfer function of the RP4 *oriT* region.

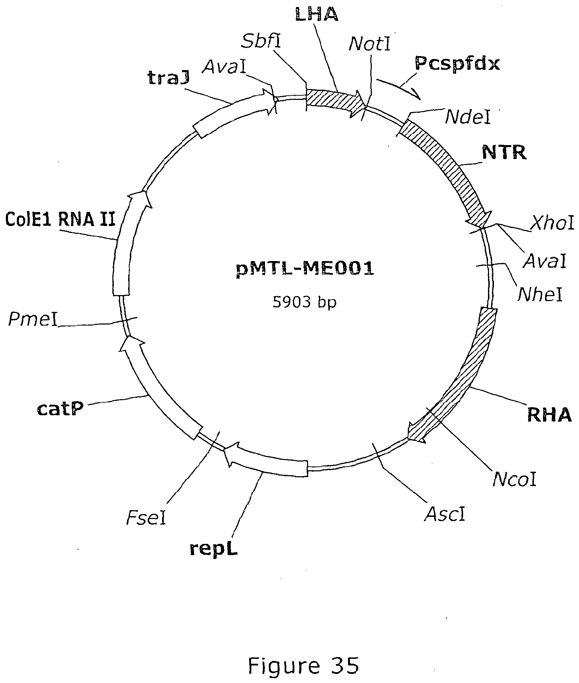

**Figure 35** - shows a schematic view of the plasmid PMTL-ME001.
   Key: LHA, left hand homology arm composting 300bp internal fragment of *Clostridium sporogenes* pyrE gene; Pfdx, the promoter region of the *Clostridium pasteurianum* ferredoxin gene, derivatised to include an operator sequence from the *E.coli lac* operon and designated fac; *NTR,* a bacterial nitroreductase; RHA, right hand homology arm composed of 1200 bp from immediately downstream of the *Clostridium sporogenes* pyrE gene, encompassing the CS3234 gene; RepL, the replication protein of plasmid pIM13; CatP, a *Clostridium perfringens-derived* gene encoding chloramphenicol acetyltransferase, and; ColE1, the replication origin of plasmid ColE1, and; *traJ,* transfer function of the RP4 *oriT* region.

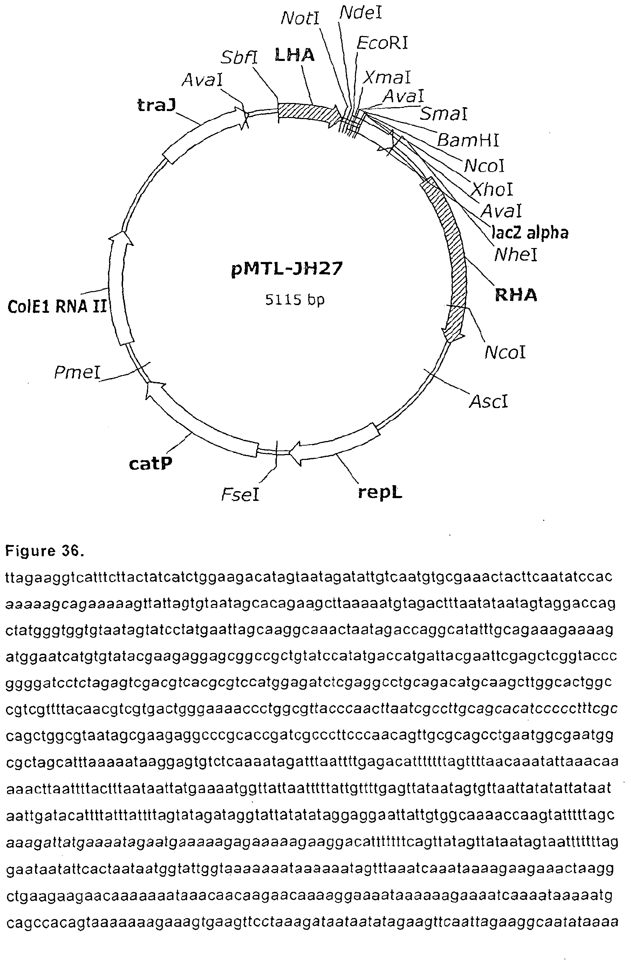

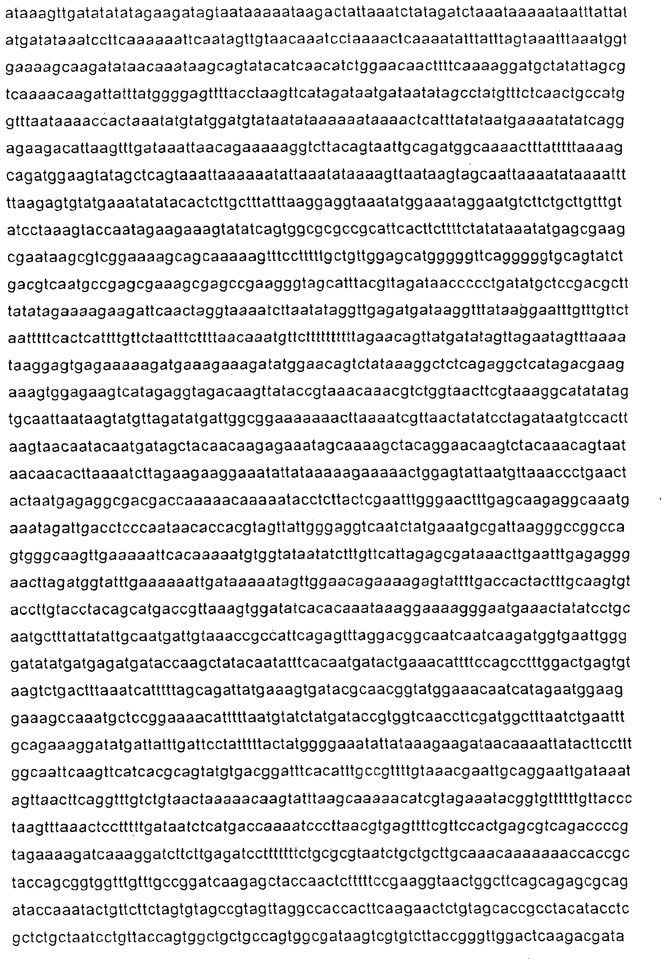

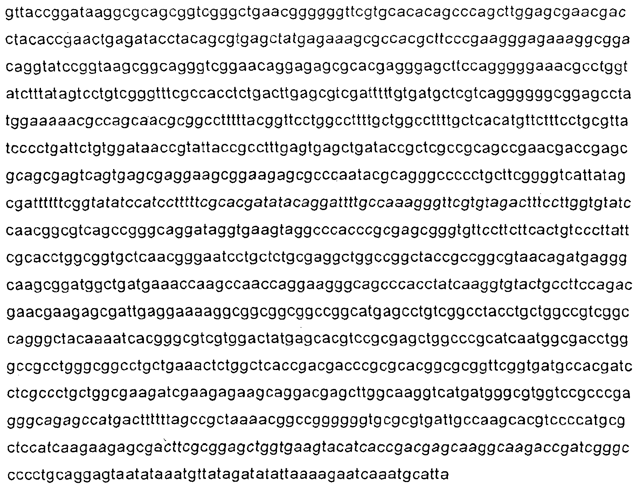

**Figure 36** - shows a schematic representation of the ACE vector PMTL-JH27.
   Key: LHA, left hand homology arm composting 300bp internal fragment of *Clostridium sporogenes* pyrE gene; LacZ', incorporating multiple cloning sites; RHA, right hand homology arm composed of 1200 bp from immediately downstream of the *Clostridium sporogenes* pyrE gene, encompassing the CS3234 gene; RepL, the replication protein of plasmid plM13; CatP, a *Clostridium perfringens-derived* gene encoding chloramphenicol acetyltransferase, and; ColE1, the replication origin of plasmid ColE1, and; *traJ,* transfer function of the RP4 *oriT* region.

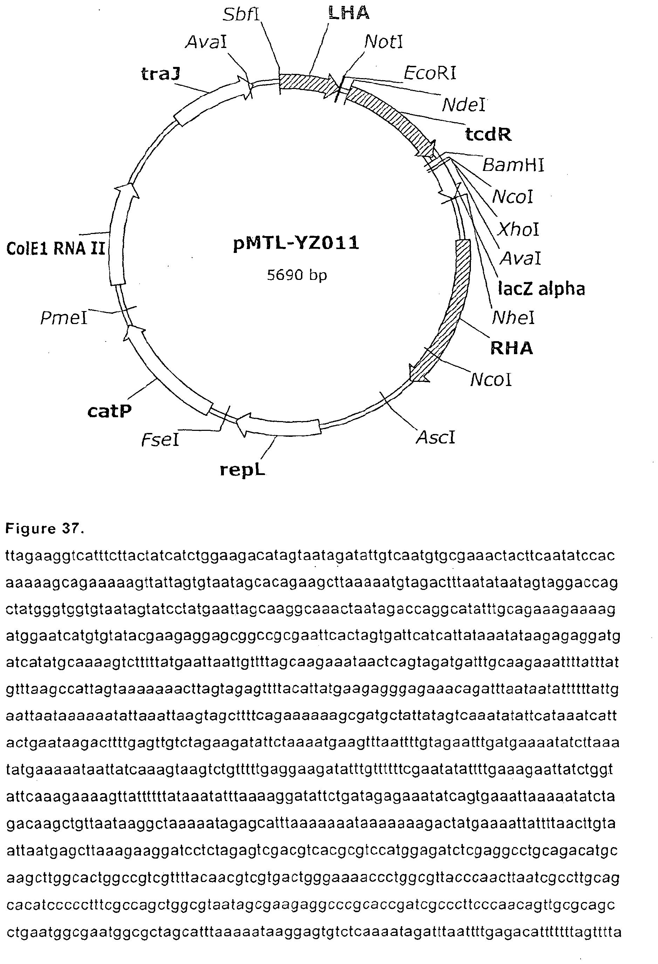

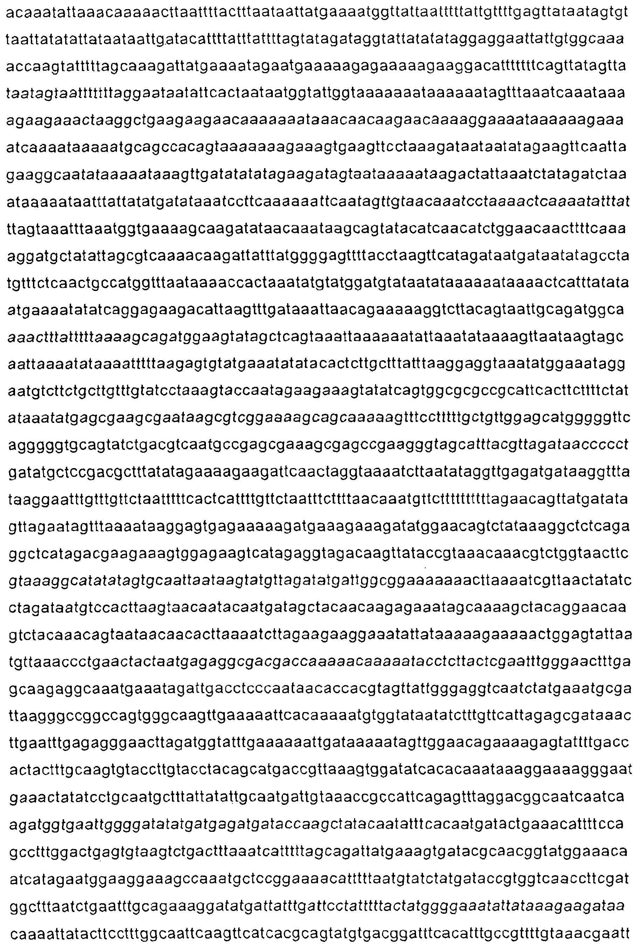

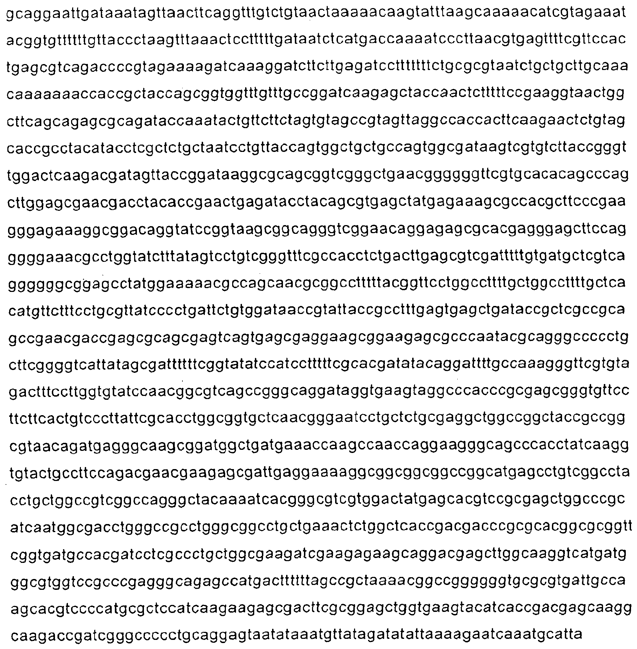

**Figure 37** - shows a schematic view of the plasmid PMTL-YZ011.
   Key: LHA, left hand homology arm composting 300bp internal fragment of *Clostridium sporogenes* pyrE gene; *tcdR* , derived from *Clostridium difficile* strain 630 and encoding an alternative RNA polymerase sigma-factor assigned to group 5 of the sigma (70) family; RHA, right hand homology arm composed of 1200 bp from immediately downstream of the *Clostridium sporogenes* pyrE gene, encompassing the CS3234 gene; RepL, the replication protein of plasmid plM13; CatP, a *Clostridium perfringens-derived* gene encoding chloramphenicol acetyltransferase, and; ColE1, the replication origin of plasmid ColE1, and; *traJ,* transfer function of the RP4 *oriT* region.

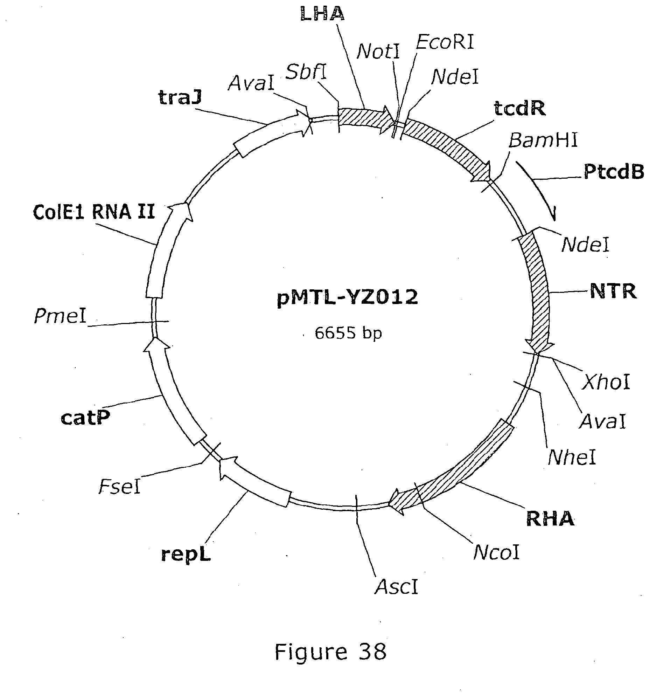

**Figure 38** - shows a schematic view of the plasmid PMTL-YZ012.
   Key: LHA, left hand homology arm composting 300bp internal fragment of *Clostridium sporogenes* pyrE gene; *tcdR* , derived from *Clostridium difficile* strain 630 and encoding an alternative RNA polymerase sigma-factor assigned to group 5 of the sigma (70) family; PtcdB, the promoter region of the *Clostridium difficile* toxinB gene; *NTR,* a bacterial nitroreductase; RHA, right hand homology arm composed of 1200 bp from immediately downstream of the *Clostridium sporogenes* pyrE gene, encompassing the CS3234 gene; RepL, the replication protein of plasmid plM13; CatP, a *Clostridium* perfringens-derived gene encoding chloramphenicol acetyltransferase, and; ColE1, the replication origin of plasmid ColE1, and; *traJ,* transfer function of the RP4 *oriT* region.

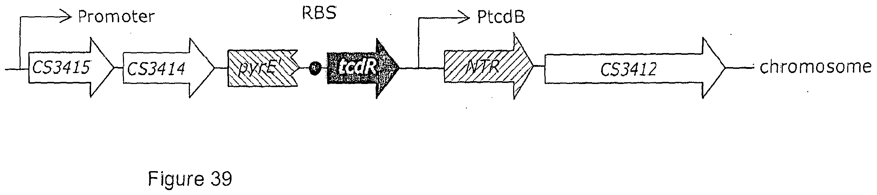

**Figure 39** - shows a schematic representation of the genome of *Clostridium sporogenes* strain CRG3844. A promoter-less copy of the tcdR gene (including its ribosome binding site, RBS) of *Clostridium difficile* strain 630 has been inserted into the genome using ACE technology immediately downstream of the disrupted *pyrE* gene (CS3413), followed by the NTR gene expressed by the *tcdB* promoter. Illustrated are the surrounding genes, and the position of the promoter responsible for expression of *tcdR,* and the position of the tcdR RBS. The illustrated terminator is the T1 transcriptional terminator of the *Clostridium pasteurianum* ferredoxin gene.

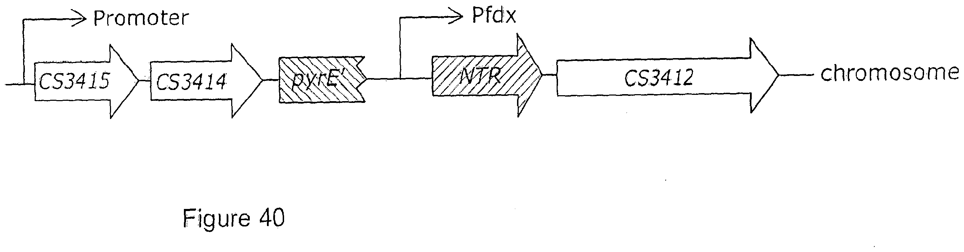

**Figure 40** - shows a schematic representation of the genome of *Clostridium sporogenes* strain CRG1650. An NTR gene expressed by the *fdx* has been inserted into the genome using ACE technology immediately downstream of the disrupted *pyrE* gene (CS3413). Illustrated are the surrounding genes, and the position of the promoter responsible for expression of *tcdR,* and the position of the *tcdR* RBS. The illustrated terminator is the T1 transcriptional terminator of the *Clostridium pasteurianum* ferredoxin gene.

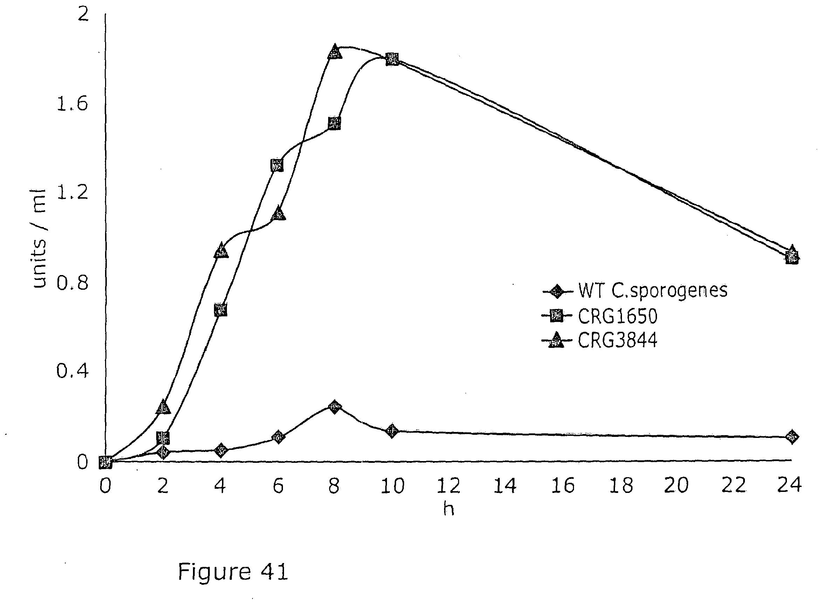

**Figure 41** - shows NTR activity of *Clostridium sporogenes* NCIMB 10696 wildtype or strain CRG3844 (*NTR* under the transcriptional control of P*_{fcdB}*), and CRG1650 (*NTR* under the transcriptional control of P*_{fdx}*).

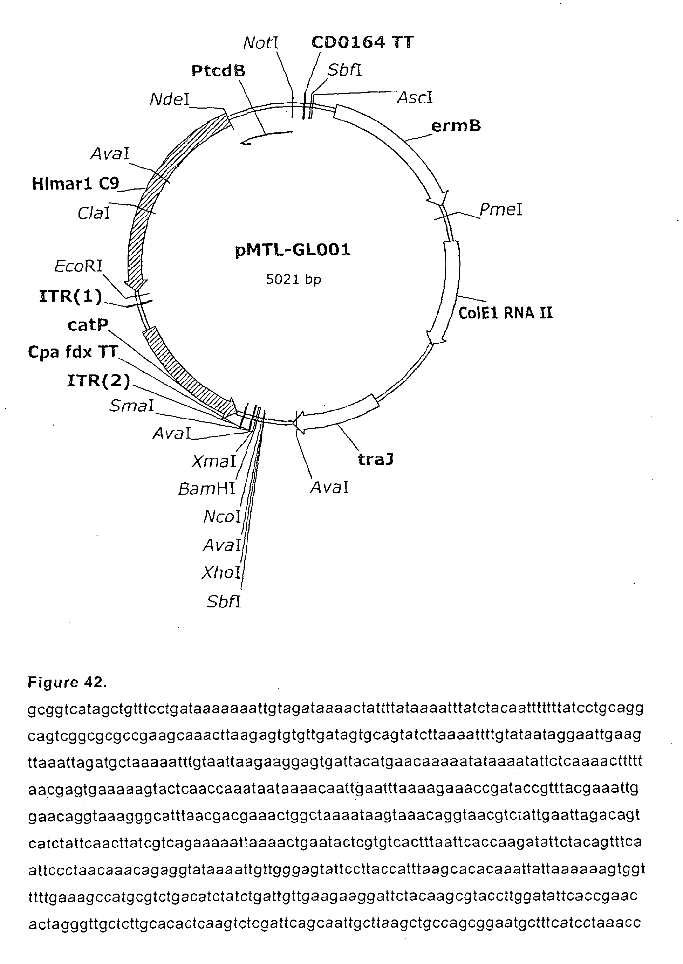

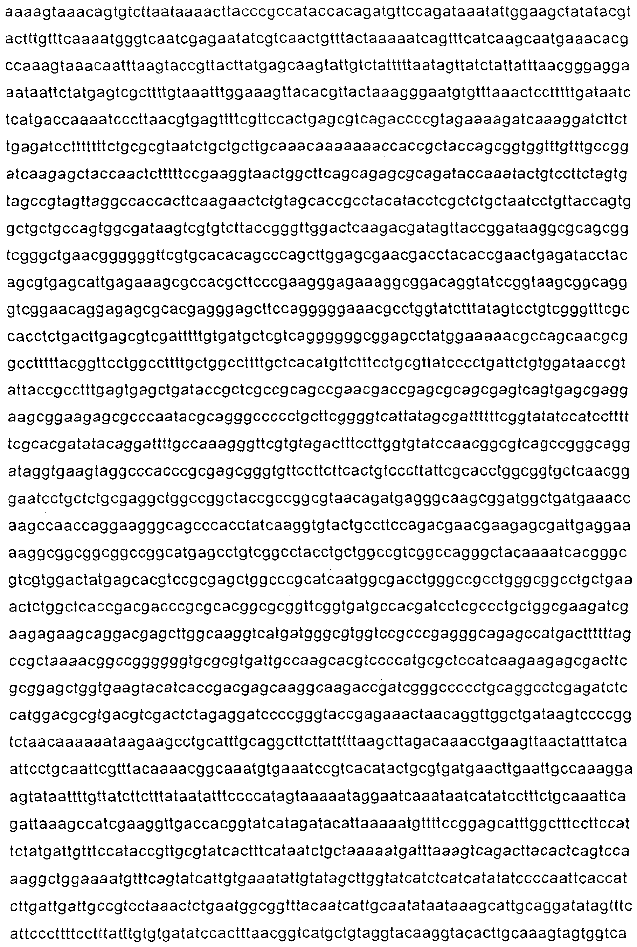

**Figure 42** - shows a schematic representation of the *mariner* transposon plasmid pMTL-GL001. Plasmid pMTL-GL001 was made by deleting the region between the AscI and Fsel sites of plasmid pMTL-SC1 which carries the pBP1 replicon.
   Key: CD0164 TT, a transcriptional terminator isolated from downstream of the *Clostridium difficile* strain 630 CD0164 gene; ermB, the macrolide-lincosamide-streptogramin B antibiotic resistance gene of plasmid pAMβ1; ColE1, the replication origin of plasmid ColE1; *traJ,* transfer function of the RP4 *oriT* region; IR1 & IR2, inverted repeat regions flanking the mini-transposon element; Cpa *fdx* TT the transcriptional terminator of the *Clostridium pasteurianum* ferredoxin gene; catP, a *Clostridium perfringens-derived* gene encoding chloramphenicol acetyltransferase, HImar1 C9, mariner transposase gene, and P*_{tcdB}*, the promoter region of the *Clostridium difficile tcdB* gene.

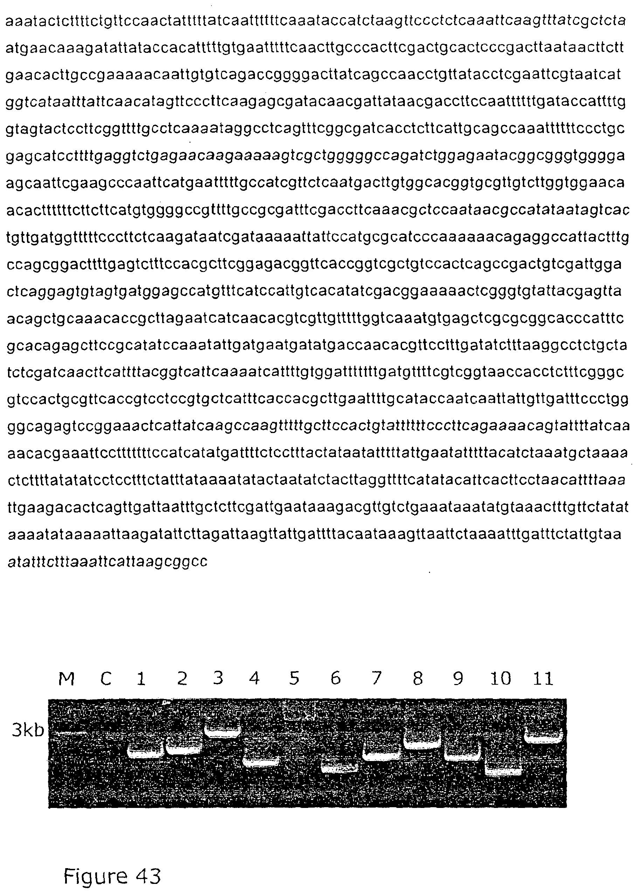

**Figure 43** - shows agarose gel electrophoresis of the inverse PCR DNA fragments generated from the chromsome of 11 randomly selected putative transposon mutants in *Clostridium acetobutylicum* strain CRG3011. Genomic DNA from 11 individual transposon mutants was isolated and digested with HindIII restriction endonuclease and the resultant DNA circularised by subsequent incubation with T4 DNA ligase. Lane M, Molecular Weight marker; Lane C, WT *Clostridium acetobutylicum* control; lanes 1 to 11, pMTL-GL001 derived Thiamphenicol resistant transposon mutant clones 1 to 11.

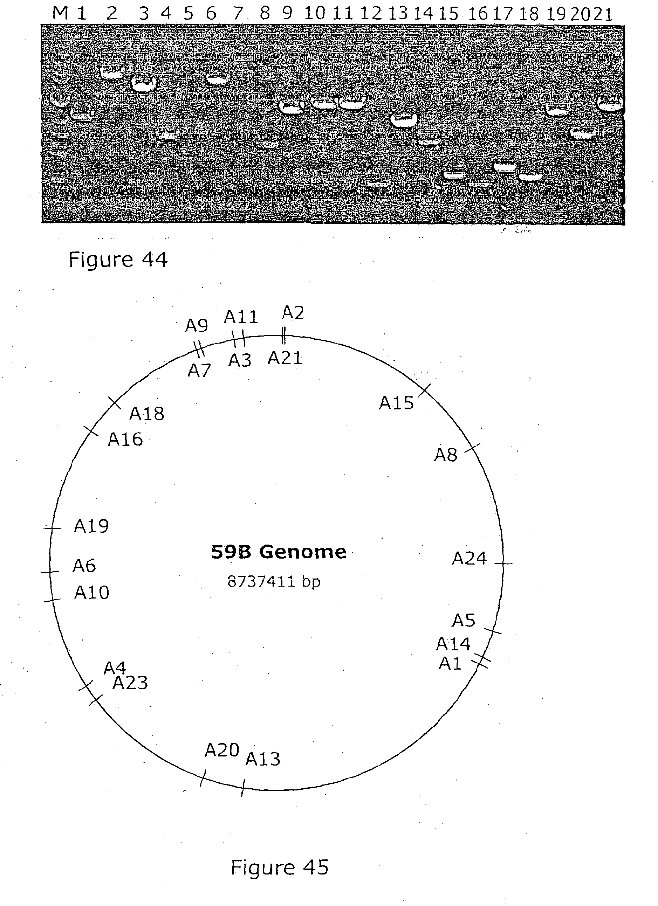

**Figure 44** - shows agarose gel electrophoresis of the inverse PCR DNA fragments generated from the chromsome of 21 randomly selected putative transposon mutants in *Clostridium beijerinckii* strain CRG3920 (59B with tcdR inserted at *pyrE* locus). Genomic DNA from 24 individual transposon mutants was isolated and digested with HindIII restriction endonuclease and the resultant DNA circularised by subsequent incubation with T4 DNA ligase. Lane M, Molecular Weight marker; lanes 1 to 21, pMTL-GL001 derived Thiamphenicol resistant transposon mutant clones 1 to 21.
**Figure 45** - shows the location of the different transposon insertions around the *Clostridium beijerinckii* stain 59B genome. Twenty-one independent transposon insertions were sequenced, and their relative positions on the genome is illustrated.

### Construction of a Clostridial strain producing TcdR

In order to generate a Clostridial strain producing functional TcdR protein from a chromosomally located gene, the following steps were undertaken:
A DNA fragment encompassing the structural gene encoding TcdR and its ribosome binding (RBS) site was PCR-amplified from the chromosome of the *Clostridium difficile* strain 630, and cloned into the vector pGEM t. This localised the *tcdR* gene and RBS to a NotI-BamHI fragment (SEQ ID NO: 12). This fragment was excised and inserted between the equivalent sites of the plasmid pMTL-ME6c (SEQ: ID NO: 13, Figure 1) to yield the plasmid pMTL-YZ001 as shown in Figure 2 (SEQ ID NO: 14). Plasmid pMTL-ME6c is essentially plasmid pMTL-JH14 (Heap JT et al., 2012, Nucleic Acids Research, 1-10, doi:10.1093/nar/gkr1321), but which lacks the *lacZ*'- encoding region residing between the shorter Left Homology Arm (LHA, encoding a *pyrE* allele) and the longer Right Homology Arm (RHA, encoding CAC0028) and in which a region of DNA encompassing the transcriptional terminator of the *Clostridium pasteurianum* ferredoxin gene has been inserted 3' to the *pyrE* allele, and preceding CAC0028.

To introduce the *tcdR* gene into the chromosome, the method, Allele-Coupled Exchange (ACE) was employed as described in (Heap JT et al., 2012, Nucleic Acids Research, 1-10, doi:10.1093/nar/gkr1321). *In vivo* methylated pMTL-YZ001 plasmid DNA was prepared from cells of *E. coli* XL1-Blue MR containing plasmid pAN2 (Heap et al., 2007, J. Microbiol. Methods, 70(3):452-64), and transformed into the ACE-generated *C*. *acetobutylicum pyrE* mutant described in Heap JT et al., 2012, Nucleic Acids Research, 1-10, doi:10.1093/nar/gkr1321. Transformed cells were plated onto CGM agar (Hartmanis MGN and Gatenbeck S, 1984, Appl. Environ. Microbiol. 47: 1277-1283) supplemented with 15 µg/ml thiamphenicol and 20 µg/ml uracil. After 24 hours, fast growing single colonies were picked and re-streaked twice onto CGM agar containing 15 µg/ml thiamphenicol and 20 µg/ml uracil. These cells represented those in which the plasmid had integrated into the genome by single cross-over recombination between the RHA. Thereafter, cells were streaked onto CBM agar (O'Brien RW and Morris JG, 1971, J. Gen. Microbiol. 68:307-318) to select for cells able to grow in the absence of exogenous uracil as a consequence of plasmid excision (through recombination between the duplicated LHA) and restoration of a functional *pyrE* allele. The final construct has the *tcdR* gene, together with its RBS, inserted immediately downstream of the *pyrE* gene, immediately upstream of the *Clostridium pasteurianum* transcriptional terminator. The *tcdR* gene is transcribed from the promoter upstream of CAC0025 (as demonstrated in Figure 3). The *Clostridium acetobutylicum* strain generated was designated CRG3011.

### Demonstration of TcdR functionality

To test the functionality of TcdR in the *Clostridium acetobutylicum* strain CRG3011, a plasmid (pMTL-YZ002, SEQ ID NO: 15) was introduced into the *C*. *acetobutylicum* cell in which the expression of a promoter-less copy of a *catP* gene was placed under the transcriptional control of the *tcdB* promoter. Plasmid pMTL-YZ002 (SEQ ID NO: 15) as shown in Figure 4, was constructed by excising a ca. 334 bp Notl/Ndel fragment from the plasmid pMTL-SC1 (Cartman and Minton, 2010, Applied Environ Microbiol, 76: 1103-9) and inserting it between the Notl and Ndel sites of plasmid pMTL82254 (Heap JT et al., 2009, J Microbiol Methods, 78: 79-85). For comparative purposes, a second plasmid was constructed identical to pMTL-YZ002, but in which the fragment encompassing the *tcdB* promoter was replaced with an equivalent Notl/Ndel fragment encompassing the *fdx* promoter (Figure 5). This plasmid was designated pMTL-YZ003 as illustrated in Figure 5 (SEQ ID NO: 16).

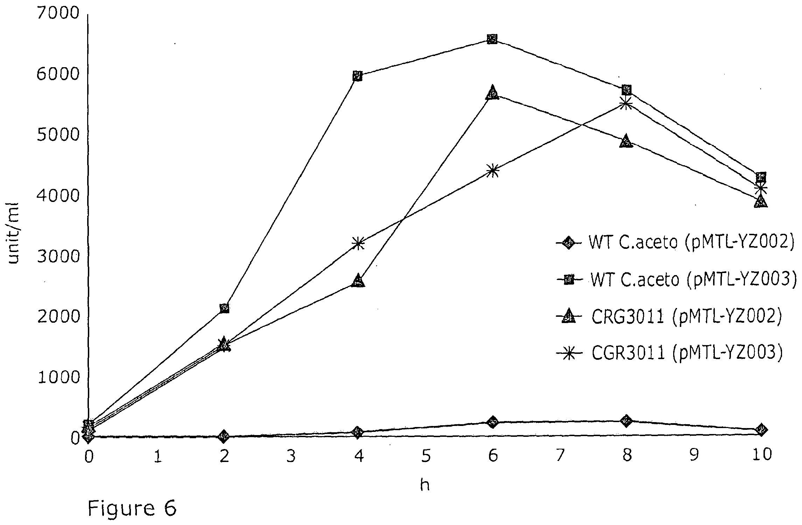

The two plasmids pMTL-YZ002 and pMTL-YZ003 were introduced into the wildtype ATCC 824 strain of *Clostridium acetobutylicum* and strain CRG3011 carrying the *tcdR* gene in its chromosome. Subsequently, the four cell lines were cultivated in CGM medium until an OD₆₀₀, of between 2.0 to 2.5 harvested by centrifugation, resuspended and disrupted by sonication. Lysates were then assayed for CAT (chloramphenciol acetyltransferase) activity using the assay of Shaw (Shaw WV, 1975, Methods Enzymol 43: 737-755). As demonstrated in Figure 6, the *tcdB* promoter is relatively inactive in the wild type strain in the absence of TcdR. Moreover, the level of TcdR-mediated CAT production from the *tcdB* promoter (pMTL-YZ002) is broadly equivalent to that of the strong *fdx* promoter (pMTL-YZ003).

### TcdR-mediated production of carbohydrate-active enzymes and binding proteins involved in the synthesis and degradation of complex carbohydrates, "CAZymes".

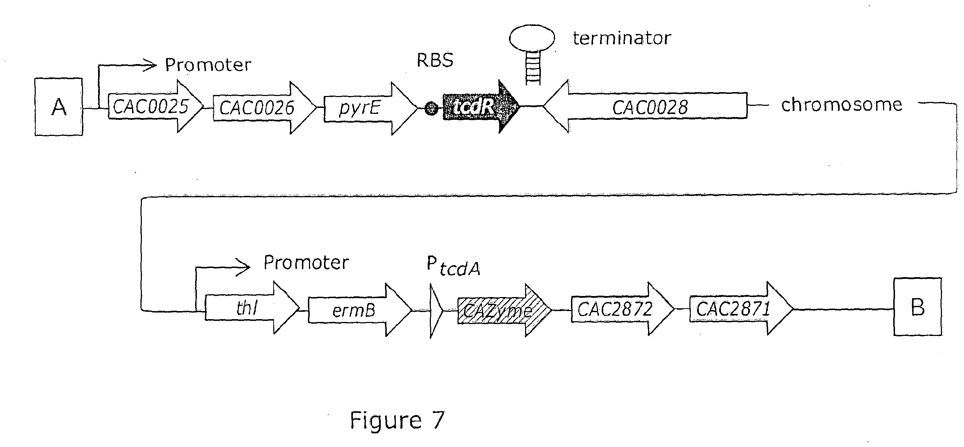

In order to bring about production of carbohydrate-active enzymes and binding proteins involved in the synthesis and degradation of complex carbohydrates (CAZymes), genes encoding the appropriate protein were inserted into the chromosome of the strain CRG3011 carrying the *tcdR* gene immediately downstream of the thiolase gene (thl) under the transcriptional control of the *tcdA* promoter (P*_{tcdA}*). A schematic of the resultant strains generated is illustrated in Figure 7. Additionally, in these examples, the natural stop codon of the gene was replaced with an extended sequence encoding a Flag-Tag protein (DYKDDDDK) and which itself ended in a suitable translational stop codon.

Each genetic element inserted, therefore, comprised the *tcdA* promoter, followed by a structural gene encoding a natural or chimeric CAZyme which had been derivatised to include a C-terminal Flag-Tag, comprising the amino acid sequence DYKDDDDK. Each gene was assembled from their 3 component parts in BioBrick-2 (BB-2) standard format (Figure 8). Thus, each component part is preceded by a prefix sequence (GAATTCGCGGCCGCACTAGT) encompassing the restriction enzyme sites EcoRI (GAATTC), Notl (GCGGCCGC) and Spel (ACTAGT) and followed by a suffix sequence (GCTAGCGCGGCCGCCTGCAG) encompassing the restriction enzyme sites Nhel (GCTAGC), Notl (GCGGCCGC) and Pstl (CTGCA).

By way of example, to construct a CAZyme expression unit which directed the production of a cellulosome associated, glycoside hydrolase of *Clostridium thermocellum* (that encoding CelA, a glycoside hydrolase family 8,GH8, enzyme), the BB-2 *tcdA* component part (SEQ ID NO: 17) was isolated as an EcoRI-NheI fragment, the BB-2 CelA structural gene (SEQ ID NO: 18) as an Spel-Nhel fragment and the BB-2 Flag-Tag component part (SEQ ID NO: 19) as a Spel-Pstl fragment. All three fragments ligated together yielded in a 1595 bp EcoRI-PstI BB-2 fragment which comprised two 6-nt sequences (GCTAGT) between the elements ligated together (*tcdA* and *celA, celA* and Flat-tag). These 6-nt 'SCAR' sequences were the result of the fusion of a Nhel and Spel restriction site as shown in Figure 9.

Similar CAZyme encoding elements were assembled in the same way, except that the CAZyme gene itself was generated out of two different BB-2 fragments encoding either a hydrolytic/catalytic domain or a cellulosome dockerin domain (Fig. 10). The hydrolytic domain was derived from *C*. *cellulolyticum* (SEQ ID NO: 20 [Cel48F] and SEQ ID NO: 21 [Xyn10A]), the dockerin domain from *C*. *thermocellum* (SEQ ID NO: 22) or *C*. *acetobutylicum* (SEQ ID NO: 23). The combinations generated including CelA are shown in Figure 11.

The final BB-2 CAZyme expressing units were cleaved with Notl and Nhel and inserted between the equivalent restriction sites of the ACE plasmid pMTL-JH16 (Figure 12, SEQ ID NO: 24). The plasmids made and their components are listed in Table 1.

**Table 1. List of plasmids generated to express native and chimeric CAZymes**

| **Plasmid** | **Vector Used** | **Promoter** | **Catalytic Domain** | **Dockerin Domain** | **Flag-Tag** |
|---|---|---|---|---|---|
| pMTL-KS001 | pMTL-JH16 | P*_{tcdA}* | Cel48F | Cel9C | variant 1 |
| pMTL-KS002 | pMTL-JH16 | P*_{tcdA}* | Xyn10A | CelS | variant 1 |
| pMTL-KS003 | pMTL-JH16 | P*_{tcdA}* | CelA | CelA | variant 1 |

The correct assembly of each plasmid was confirmed by restriction analysis and sequencing.

To introduce the CAZymes carried by each plasmid into the genome, the method, Allele-Coupled Exchange (ACE) was employed (Heap JT et al., 2012, Nucleic Acids Research, 1-10, doi:10.1093/nar/gkr1321). *In vivo* methylated pMTL-KS001, pMTL-KS002 and pMTL-KS003 plasmid DNA was prepared from cells of *E. coli* XL1-Blue MR containing plasmid pAN2 (Heap et al., 2007, J. Microbiol. Methods, 70(3):452-64), and transformed into *C.acetobutylicum* strain CRG3011 carrying the *tcdR* gene. Transformed cells were plated onto 2xYTG agar (Hartmanis MGN and Gatenbeck S, 1984, Appl. Environ. Microbiol.47: 1277-1283) supplemented with 15 µg/ml thiamphenicol. After 24 hours, fast growing single colonies were picked and re-streaked onto CBM agar containing 40 µg/ml erythromycin. These cells represented those in which the plasmid had first integrated into the genome by single cross-over recombination between the RHA, and thereafter and positioned the promoter-less *ermB* downstream of the *thl* gene (see Figure 8). To confirm loss of the plasmid, erythromycin resistant colonies were patch plated onto CBM containing 15 µg/ml thiamphenicol, where no growth occurred. The authenticity of the clones was determined by PCR and nucleotide sequencing of the DNA fragments generated.

For detection of the expression of the recombinant CAZymes, strains were grown for 12-16 hours in dilution series in 2xYTG medium (pH 5.7). Subsequently, exponentially grown cultures were used to inoculate the 25- to 50-ml 2x YTG medium containing 100 mM MES (2-morpholinoethanesulfonic acid) buffer) (pH 6.9 at inoculation) main culture to a final OD₆₀₀ of 0.15. After reaching an OD₆₀ of 1.2 to 1.8 and a pH of not less than 6.0, cells were harvested by centrifugation (8,000 x g, 15 min, 4°C). The culture supernatant was centrifuged a second time, before its protein content was precipitated using final concentrations of 0.03% (w/v) DOC and 10% (w/v) TCA (centrifugation at 10,000 x g, 30 min, 4°C). Protein pellets were washed with 10 ml of 10 mM Tris-HCl (pH 7.5), dried and resuspended in 1 M Tris-HCl (pH 7.5) and kept on ice. All samples were normalised to their OD₆₀₀ at the time of the harvest in the early stationary phase. Following, SDS-PAGE and Western-Blot analysis were carried out using NuPAGE® Novex 4-12% Bis-Tris gels, the XCell II™ Blot Module (Invitrogen) and the ProteoQwest™ FLAG® colorimetric Western Blotting Kit (Sigma Aldrich). All steps were carried out according to the manufacturer's manuals. An analysis of culture supernatants of native CelA and chimeric Cel48F and Xyn10A expressing strains is shown in Figure 13. All chosen enzymes showed detectable signals using the TcdR/*tcdA* expression system.

### Use of the expression system for expression of the transposase Himar1 C9

Transposon mutagenesis using the *mariner* transposon-based transposon vector pMTL-SC1 is reliant on TcdR-mediated expression of the mariner transposase. Accordingly, the introduction of this vector into the *C*. *acetobutylicum* strain CRG3011 should result in transposition of the mini-transposon carrying the *catP* gene.

To test this possibility, pMTL-SC1 was first modified by swopping its Gram positive replication region (based on the plasmid pBP1) with another replicon based on a segregationally unstable derivative of the pCB102 replication region. To achieve this, the pCB102 replicon was isolated from the modular plasmid pMTL83251 (Heap et al., 2009, J Microbiol. Methods. 78(1): 79-85) as a 1626bp AscI and Fsel fragment and inserted between the equivalent sites of pMTL-SC1, replacing the equivalent fragment encompassing the pBP1 replicon. The plasmid obtained was designated pMTL-YZ004 (SEQ ID NO: 25, Figure 13).

Thereafter, plasmid pMTL-YZ004 was linearised through cleavage at its unique Nsil site, which resides within the coding region of the putative replication protein of pCB102, RepH. The linearised DNA was treated with T4 polymerase and the resultant blunt-ended fragment self-ligated and transformed into *E.coli* Top10. A plasmid, pMTL-YZ005, was isolated which no longer cleaved with Nsil. The presence of the expected sequence was then confirmed by nucleotide sequencing. The resultant manipulation altered the sequence ATGCAT to AT. There consequence deletion of 4 nucleotides (TGCA) causes a frameshift in the RepH coding sequence, replacing the COOH-terminal region of RepH (CIKYYARSFKKAHVKKSKKKK) with LNIMGALKKLM. This replacement affects the segregational stability of the plasmid.

To determine whether transposition would occur in strain CGR3011, plasmid pMTL-YZ005 was transformed into *Clostridium acetobutylicum* CRG3011 and transformants selected on CGM plates containing 40 µg/ml erythromycin. Plates carrying greater than 10 isolated, transformant colonies were then incubated at 37°C for 72 hours. All of the colony growth was scraped from the plate using a sterile loop and the cells resuspended in CGM media containing +20% Glycerol. The cell suspension was then plated at serial dilutions onto CGM agar plates containing 15 µg/ml thiamphenicol. A total of 100 colonies were then patch plated onto CGM plates containing 15 µg/ml thiamphenicol and CGM plates containing 40 µg/ml erythromycin as a simple test to ascertain whether the plasmid pMTL-YZ005 was still present. All 100 colonies still retained resistance to erythromycin, indicating that under the conditions employed, the plasmids had not been lost from the population.

To establish whether transposition had occurred, inverse PCR was performed according to the procedure of Cartman and Minton (Cartman ST and Minton NP, 2010, Applied Environmental Microbiology, 76: 1103-1109). Genomic DNA was isolated from 8 individual thiamphenicol resistant clones and digested overnight with HindIII at a concentration of 200ng/µl. The HindIII restriction endonuclease was heat inactivated (65°C for 30 min), and DNA was diluted to a concentration of 5 ng/µl in a reaction with T4 DNA ligase to favor self-ligation (and thus circularization) of restriction fragments. Ligation reaction mixtures were incubated at ambient temperature for 1 h, and then the T4 ligase was heat inactivated (65°C for 30 min). Inverse PCRs were carried out in 50-µl volumes using the KOD Hot Start DNA polymerase Master Mix kit (Novagen), with 100 ng of ligated DNA and primers catP-INV-F1, SEQ ID NO: 26 (5'-TAAATCATTTTTAGCAGATTATGAAAGTGATACGCAACGGTATGG-3') and catP-INV-R1, SEQ ID NO: 27 (5'-TATTGTATAGCTTGGTATCATCTCATCATATATCCCCAATTCACC-3'), which face out from the transposon-based *catP* sequence. Inverse PCR products were run out on a 0.8% (wt/vol) agarose gel (Fig. 18), purified with the QIAquick gel purification kit (Qiagen), and sequenced using primer catP-INV-R2 SEQ ID NO: 28 (5'-TATTTGTGTGATATCCACTTTAACGGTCATGCTGTAGGTACAAGG-3'). To identify the genomic location of transposon insertions, sequence data were analyzed using GENtle.

These data revealed that in 6 of the 8 colonies tested, the transposon had inserted into 6 different locations within the *C*. *acetobutylicum* genome (Table 2). In one case (No. 5) no product was obtained, and in another (No. 5) the sequence corresponds to plasmid pMTL-YZ005. These data provide proof of principle that the presence of *tcdR* in the genome of CRG3011 allows transposition of the *mariner* transposon in *Clostridium acetobutylicum.*

**Table 2. Sequence analysis of the Inverse PCR products from eight randomly selected thiamphenicol resistant colonies carrying pMTL-YZ005**

| **Colony Number** | **Position of Insertion in the *C. acetobutylicum* ATCC 824 genome** | **Gene affected** | **Gene function** |
|---|---|---|---|
| 1 | 521363 (forward strand) | promoter region of CA_C0453 | ABC-transporter, ATP-binding protein |
| 2 | 3083171 (forward strand) | CA_C2948 coding region | ABC-transporter, ATPase |
| 3 | 3562958 (forward strand) | CA_C3385 | hypothetical protein |
| 4 | 286365 (reverse strand) | CA_C3457 | hypothetical protein |
| 5 | pMTL-YZ005 | NAP | NAP |
| 6 | No sequence | NAP | NAP |
| 7 | 2805189 (forward strand) | CA_C2685 | maltose phosphorylase |
| 8 | 1839066 (forward strand) | CA_C1688 | penicillin-binding protein |

### The use of the TcdR sigma factor to express butanol, isopropanol or acetone pathway genes in C.acetobutylicum

A derivative of *C*. *acetobutylicum* lacking pSOL1 was generated to serve as a host for the subsequent strain engineering. This was achieved using a variation of the ACE method in which the *repA* gene of pSOL1 was inactivated. Such a strain is effectively a 'blank canvas' for engineering solvent production, with the key genes involved in solvent production expressed only from introduced DNA, free from native regulation. This also provides a sensitive background in which to monitor gene expression using solvent production as we study different expression constructs. Genes have previously been introduced into pSOL1-free strains, but always using plasmids (which are unstable, and require antibiotic maintenance) and the host strains used were always degenerates obtained by extensive passaging (Sillers et al., 2008, Metabolic Engineering 10: 321-332; Lee et al., 2009, Biotechnol J, 4:1432-1440).

A series of synthetic BB-2 DNA constructs were assembled in which the genes encoding key enzymes in solvent production were cloned, either alone or in combination with other genes into the ACE vector pMTL-JH16 and then integrated using ACE into the chromosome of the pSOL1-free 'strain P' immediately downstream of the thiolase gene (*thl*)*.* In each case, the stop codon of each gene was replaced with the Biobrick encoding a Flag-Tag plus stop codon (SEQ ID NO: 29) as schematically shown in Figure 15. In some cases the promoter of the tcdA gene was also added as a BioBrick-2, as schematically shown in Figure 16.

In the initial series of strains created, the expression of the various inserted genes was designed to be reliant on the upstream thiolase gene promoter (*pthl,* Figure 17). Direct evidence that expression was occurring was obtained by performing ProteoQwest™ FLAG® colorimetric Western Blotting (Sigma Aldrich) on cell lysates using antibody directed against the Flag-Tag present at the COOH-terminus of every enzyme (Figure 18).

The three strains containing the complete operons for butanol (P28), acetone (P25) or isopropanol (P24) production were further characterised by 72-hours batch fermentations and GC analysis of products formed in comparison to the control pSOL1-free strain 'P' and wild-type. In each case the expected end products were detected, confirming functional expression of all the inserted genes. However, the concentration of the products was low (strain P28, 1.7 mM butanol; strain P24, 4.5 mM isopropanol; strain P25, 10.0 mM acetone) suggesting that low enzyme expression was likely the limiting flux in the inserted pathways.

To test this hypothesis and attempt to improve production, strain P36 was constructed (Figure 19) in which the strong promoter from the *ptb* gene was inserted in front of the butanol pathway genes (*ald* and *bdhB*), instead of relying upon the upstream promoter of the *thl* gene. The strain was characterised as before. Butanol production was found to have improved slightly (3.7 mM butanol after 72 hours fermentation). This butanol concentration was still low, suggesting that expression was still the limiting factor for butanol production.

As limiting expression might be a recurring problem with constructs maintained on the chromosome at a single copy, the 'orthogonal' expression system based on the sigma factor TcdR and the corresponding *tcdA* promoter, both components from *Clostridium difficile* was utilised Assembly of the single enzymes and the operon constructs containing *tcdR* and *tcdA* was carried out following the BB-2 standard. Different strains were constructed. In strain P43, after integration of the synthetic construct into the chromosome, expression of *tcdR* is directed by the upstream strong vegetative *thl* promoter (Figure 19). In turn, TcdR directs expression of the butanol pathway genes from the *tcdA* promoter. TcdR should not recognise any other promoter in *C*. *acetobutylicum* other than the introduced *tcdA* promoter, which would result in high level expression of the butanol pathway. Indeed, when the p45 strain was characterised, 18.1 mM butanol was present in the batch fermentation after 72 hours, a considerable improvement over the previous strains.

To improve the yield of valuable products in the fermentation, a strain was sought in which the butanol pathway genes (*ald* and *bdhB*) were highly expressed, while the isopropanol pathway genes (*ctfA, ctfB, adh* and adc) were expressed at a low level. The aim was for butyric acid and acetic acid re-uptake to occur, without flux to acetone and isopropanol competing too strongly with flux to butanol. To this end, a hybrid system was designed and constructed in which the isopropanol pathway genes (*ctfA, ctfB, adh* and adc) were expressed from the native *thl* promoter, along with tcdR, while the butanol pathway genes (*ald* and *bdhB*) were expressed from the heterologous *tcdA* promoter as before (strain P45; Figure 19).

Strain P45 produced almost as much butanol as strain P43, and also produced 8.1 mM lower butyrate and 9.9 mM lower acetate concentrations. Isopropanol was produced to a concentration of 9.7 mM, and a small amount (1.4 mM) of acetone was also measured, which is an intermediate in the isopropanol pathway. To see if the undesired residual acetone could be eliminated, strain P51 was constructed and designed, in which the gene which reduces acetone to isopropanol (ald) was moved from the operon under relatively low-level expression from the *thl* promoter to the operon under high-level from the *tcdA* promoter. Acetone production was indeed eliminated, and strain P51 also produced slightly more isopropanol.

**Table 3. Levels of Organic Acid and Solvents in the various clones of C.acetobutylicum**

| | **Concentration of Organic Acids and Solvents (mM)** | | | | | | | | |
|---|---|---|---|---|---|---|---|---|---|
| STRAIN | Acetate | Acetoin | Acetone | Butanol | Butyrate | Ethanol | Propanol | Lactate | Glucose |
| WT | 2.5 | 1.1 | 63.0 | 89.2 | 6.4 | 9.3 | 0.1 | 7.4 | 93.8 |
| P | 5.8 | 0.8 | 0.0 | 0.0 | 32.8 | 1.7 | 0.0 | 8.7 | 219.6 |
| P28 | 7.0 | 0.8 | 0.0 | 1.7 | 30.5 | 2.2 | 0.0 | 9.8 | 198.4 |
| P24 | 4.3 | 0.4 | 1.9 | 0.0 | 35.3 | 2.0 | 4.5 | 8.8 | 188.0 |
| P25 | 3.3 | 0.8 | 10.0 | 0.0 | 36.5 | 1.7 | 0.0 | 14.8 | 174.7 |
| P36 | 8.0 | 0.8 | 0.0 | 3.7 | 31.8 | 2.2 | 0.0 | 8.8 | 208.7 |
| P43 | 14.6 | 1.0 | 0.0 | 18.1 | 33.9 | 2.7 | 0.0 | 8.5 | 170.7 |
| P45 | 4.7 | 0.1 | 1.4 | 17.3 | 25.8 | 1.4 | 9.7 | 6.2 | 194.0 |
| P51 | 3.6 | 0.1 | 0.0 | 17.6 | 23.0 | 1.6 | 11.4 | 6.8 | 188.2 |

### TcdR-mediated production of carbohydrate-active enzymes and binding proteins involved in the synthesis and degradation of complex carbohydrates, "CAZymes"

In order to demonstrate the effectiveness of the orthogonal expression system in the production of carbohydrate-active enzymes and binding proteins (CAZymes) involved in the synthesis and degradation of complex carbohydrates, two sets of ACE plasmids were generated as described in Table 1 and 2. Genes encoding the appropriate protein were inserted into the chromosome of the wild type *C*. *acetobutylicum* ATCC 824 and the strain CRG3011 carrying the *tcdR* gene, immediately downstream of the thiolase gene (*thl*) with or without the transcriptional control of the *tcdB* promoter (P*_{tcdB}*).

**Table 1. List of ACE plasmid generated to express native and chimeric CAZymes under the transcriptional control of the thl promoter when integrated into the genome.**

| **Plasmid** | **Vector Used** | **Promoter** | **Catalytic Domain** | **Dockerin Domain** | **Flag-Tag** |
|---|---|---|---|---|---|
| pMTL-KS004 | pMTL-JH16 | *Pₜₕₗ* | Cel48F | CelS | variant 1 |
| pMTL-KS005 | pMTL-JH16 | *Pₜₕₗ* | Cel48F | Cel9C | variant 1 |
| pMTL-KS006 | pMTL-JH16 | *Pₜₕₗ* | Cel9G | CelS | variant 1 |
| pMTL-KS007 | pMTL-JH16 | *Pₜₕₗ* | Cel9E | CelS | variant 1 |
| pMTL-KS008 | pMTL-JH16 | *Pₜₕₗ* | Xyn10A | CelS | variant 1 |
| pMTL-KS009 | pMTL-JH16 | *Pₜₕₗ* | CelA | CelA | variant 1 |

**Table 2. List of plasmid generated to express native and chimeric CAZymes under the transcriptional control of the tcdB promoter when integrated into the genome.**

| **Plasmid** | **Vector Used** | **Promoter** | **Catalytic Domain** | **Dockerin Domain** | **Flag-Tag** |
|---|---|---|---|---|---|
| pMTL-KS010 | pMTL-JH16 | P*_{tcdB}* | Cel48F | CelS | variant 1 |
| pMTL-KS011 | pMTL-JH16 | P*_{tcdB}* | Cel48F | Cel9C | variant 1 |
| pMTL-KS012 | pMTL-JH16 | P*_{tcdB}* | Cel9G | CelS | variant 1 |
| pMTL-KS013 | pMTL-JH16 | P*_{tcdB}* | Cel9E | CelS | variant 1 |
| pMTL-KS014 | pMTL-JH16 | P*_{tcdB}* | Xyn10A | CelS | variant 1 |
| pMTL-KS015 | pMTL-JH16 | P*_{tcdB}* | CelA | CelA | variant 1 |

To introduce the CAZymes carried by each plasmid into the genome, the method, Allele-Coupled Exchange (ACE) was employed (Heap JT et al., Nucleic Acids Research, 2012 40(8):e59). In brief, *in vivo* methylated ACE plasmids DNA was prepared from cells of *E. coli* XL1-Blue MR containing plasmid pAN2 (Heap et al.J. Microbiol. Methods 2007 70(3):452-64), and transformed into *C.acetobutylicum* strains. Transformed cells were plated onto 2xYTG agar (Hartmanis MGN and Gatenbeck S, Appl. Environ. Microbiol 1984 47: 1277-1283) supplemented with 15 µg/ml thiamphenicol. After 24 hours, fast growing single colonies were picked and re-streaked onto CBM agar containing 40µg/ml erythromycin. These cells represented those in which the plasmid had first integrated into the genome by single cross-over recombination between the RHA, and thereafter excised through excision via recombination at the LHA resulting in the integration of the promoter-less *ermB* gene, together with the CAZymes-encoding gene under the control of the *tcdB* promoter, downstream of the *thl* gene (see Fig. 11). To confirm loss of the plasmid, erythromycin resistant colonies were patch plated onto CBM containing 15 µg/ml thiamphenicol, where no growth occurred. The authenticity of the clones was determined by PCR and nucleotide sequencing of the DNA fragments generated.

For detection of the expression of the recombinant CAZymes, strains were grown for 12-16 hours in dilution series in 2xYTG medium (pH 5.7). Subsequently, exponentially grown cultures were used to inoculate the 25- to 50-ml 2x YTG medium (pH 6.9 at inoculation) main culture to a final OD₆₀₀ of 0.15. After reaching an OD₆₀₀ of 1.2 to 1.8 and a pH of not less than 6.0, cells were harvested by centrifugation (8,000 x g, 15 min, 4°C). The culture supernatant was centrifuged a second time, before its protein content wwas precipitated using final concentrations of 0.03% (w/v) DOC and 10% (w/v) TCA (centrifugation at 10,000 x g, 30 min, 4°C). Protein pellets were washed with 10 ml of 10 mM Tris-HCI (pH 7.5), dried and resuspended in 1 M Tris-HCI (pH 7.5) and kept on ice. All samples were normalised to their OD₆₀₀ at the time of the harvest in the early stationary phase. Following, SDS-PAGE and Western-Blot analysis were carried out using NuPAGE® Novex 4-12% Bis-Tris gels and the XCell II™ Blot Module (Invitrogen) and the ProteoQwest™ FLAG® colorimetric Western Blotitng Kit (Sigma Aldrich). All steps were carried out according to the manufacturer's manuals. An analysis of culture supernatants of native and chimeric CAZymes expressing strains is shown in Fig. 20. All chosen enzymes were produced at visably higher levels when then gene was under the control of the TcdR/tcdB expression system, compared to when under the transcriptional control of the *thl* promoter (see Fig. 20).

### Generation of Clostridium acetobutylicum strains producing BotR

To demonstrate that all members of the group 5 RNA polymerase sigma factors may be used in a similar way to TcdR and its cognate promoters, we tested the effectiveness of the equivalent system form *Clostridium botulinum,* BotR (CntR) and its two cognate promoters, *Pₙₜₙₕ* and P*ₕₐ₃₄*, which reside immediately upstream of the *ntnh-botA* operon and the *ha34-ha17-ha70* operons, respectively. To test the system, the *botR* gene was integrated into the *C.acetobutylicum* genome at the *pyrE* locus using ACE, and the two cognate promoters (P*ₙₜₙₕ* and P*ₕₐ₃₄*) placed immediately 5' to a promoter-less copy of the *C*. *perfringens catP* and inserted into an autonomous plasmid, pMTL82254.

The *botR* gene was integrated into the genome at the *pyrE* locus using two different strategies: (i) a DNA fragment encompassing only the structural gene encoding BotR and its ribosome binding (RBS) site, and; (ii) a DNA fragment encompassing the structural gene encoding BotR and its ribosome binding (RBS) site and its promoter. Both fragments were PCR-amplified from the chromosome of the *Clostridium botulinum* strain ATCC 3502, NotI-BamHI fragments (SEQ ID NO: 26 and 27), and cloned into the vector pMTL-ME6c (SEQ ID NO: 13 Fig. 1) to yield the plasmid pMTL-YZ005 and pMTL-YZ006 (SEQ ID NO: 28 and 29, Fig. 21 and 22). Plasmid pMTL-ME6c is essentially plasmid pMTL-JH14 (Heap et al.J. Microbiol. Methods 2007 70(3):452-64), but has an additional transcriptional terminator (that of the ferredoxin gene of *Clostridium pasteurianum,* T1) inserted between the *lacZ* and the right hand homology arm (RHA, encoding CAC0028).
SEQ ID NO: 26: (rbs-botR) 
gcggccgcaaataatatgtatattATGGAAGGTAGTGGTAAATATGAATAAATTGTTTTACA
AATTAAAATGTTAAAAAATGACAATAGGGAGTTTCAAGAAATTTTTAAGCATTTTGA
AAAAACTATAAATATATTTACTAGAAAATATAATATATATGATAATTACAATGATATTT
TGTACCATTTATGGTATACACTTAAAAAGTTGATTTGAGCAATTTCAATACACAAA
ATGATTTAGAGAGATATATTAGTAGGACTTTAAAAAGATATTGCTTAGATATTTGCA
ATAAAAGAAAGATTGATAAGAAAATAATATATAATTCAGAAATTGTAGATAAGAAAT
TAAGCTTAATAGCAAATAGTTATTCAAGTTATTTAGAATTTGAATTTAATGATTTAAT
ATCCATATTACCTGATGATCAAAAGAAAATTATATATATGAAATTTGTTGAAGATATT
AAGGAGATAGATATAGCTAAAAAACTTAATATAAGTCGTCAATCTGTATATAAAAAT
AAAATAATGGCTTTAGAGAGATTAGAACCCATATTGAAAAAATTAATTAATATGTAGt
ttggatcc
SEQ ID NO: 27: (pro-botR)

gcggccgcataattgattatggatatttcgtaaaaatggcttattaaaatttaaaggcaattagtttatttatagta
taataaaaaaataatatgtatattatggaagggtagtggtaaatATGAATAAATTGTTTTACAAA
TTAAAATGTTAAAAAATGACAATAGGGAGTTTCAAGAAATTTTTAAGCATTTTG
AAAAAACTATAAATATATTTACTAGAAAATATAATATATATGATAATTACAATG
ATATTTTGTACCATTTATGGTATACACTTAAAAAAGTTGATTTGAGCAATTTCA
ATACACAAAATGATTTAGAGAGATATATTAGTAGGACTTTAAAAAGATATTGC
TTAGATATTTGCAATAAAAGAAAGATTGATAAGAAAATAATATATAATTCAGAA
ATTGTAGATAAGAAATTAAGCTTAATAGCAAATAGTTATTCAAGTTATTTAGAA
TTTGAATTTAATGATTTAATATCCATATTACCTGATGATCAAAAGAAAATTATA
TATATGAAATTTGTTGAAGATATTAAGGAGATAGATATAGCTAAAAACTTAAT
ATAAGTCGTCAATCTGTATATAAAAATAAAATAATGGCTTTAGAGAGATTAGA
ACCCATATTGAAAAAATTAATTAATATGTAGtttggatcc

To introduce the *botR* gene into the chromosome, the method, Allele-Coupled Exchange (ACE) was employed (Heap et al.J. Microbiol. Methods 2007 70(3):452-64). In brief, *in vivo* methylated pMTL-YZ005 and pMTL-YZ006 plasmid DNA were prepared from cells of *E. coli* XL1-Blue MR containing plasmid pAN2 (Heap et al. J. Microbiol. Methods 2007 70(3):452-64), and transformed into the ACE-generated *C*. *acetobutylicum pyrE* mutant described in Heap et al.J. Microbiol. Methods 2007 70(3):452-64. Transformed cells were plated onto CGM agar (Hartmanis MGN and Gatenbeck S, Appl. Environ. Microbiol 1984 47: 1277-1283) supplemented with 15µg/ml thiamphenicol and 20µg/ml uracil. After 24 hours, fast growing single colonies were picked and re-streaked twice onto CGM agar containing 15 µg/ml thiamphenicol and 20µg/ml uracil. These cells represented those in which the plasmid had integrated into the genome by single cross-over recombination between the RHA. Thereafter, cells were streaked onto CBM agar (O'Brien RW and Morris JG, J. Gen. Microbiol 1971 68:307-318) to select for cells able to grow in the absence of exogenous uracil as a consequence of plasmid excision (through recombination between the duplicated LHA) and restoration of a functional *pyrE* allele.

In the case of strategy (i), using plasmid pMTL-YZ005, the final construct has the *botR* gene, together with its RBS, inserted immediately downstream of the *pyrE* gene, and immediately upstream of the *Clostridium pasteurianum* transcriptional terminator. The *botR* gene is transcribed from the promoter upstream of CAC0025 (see Fig. 23 and 24). The strain generated was designated CRG3755. In the case of strategy (ii), using plasmid pMTL-YZ006, the final strain CRG3756 is essentially the same as CRG3755, except the *botR* gene is under the transcriptional control of the native botR promoter.

### Demonstration of BotR functionality

To test the functionality of BotR in the *Clostridium acetobutylicum* strains CRG3755 and CRG3756, plasmids (pMTL-YZ007 and pMTL-YZ008) were introduced into the *C. acetobutylicum* cell in which the expression of a promoter-less copy of a *catP* gene was placed under the transcriptional control of either P*ₙₜₙₕ* (pMTL-YZ007) or P*ₕₐ₃₄* (pMTL-YZ008). Plasmid pMTL-YZ007 (SEQ ID NO: 30 Fig. 25) was constructed by PCR amplifying the *ntnH* promoter, a ca. 158 bp NotI/NdeI fragment from chromosome of the *Clostridium botulinum* strain ATCC 3502 and inserting it between the NotI and Ndel sites of plasmid pMTL82254 (Heap JT et al., 2009, J Microbiol Methods, 78: 79-85).

Plasmid pMTL-YZ008 (SEQ ID NO: 31 Fig. 26) was constructed by PCR amplifying the *ha33* promoter, a ca. 197 bp Notl/Ndel fragment from chromosome of the *Clostridium botulinum* strain ATCC 3502 and inserting it between the Notl and Ndel sites of plasmid pMTL82254.

The three plasmids pMTL-YZ007, pMTL-YZ008 and pMTL-YZ003 (SEQ ID NO:16 Fig. 5). were introduced into the wildtype ATCC 824 strain of *Clostridium acetobutylicum* and strain CRG3755 and CRG3756 carrying the *botR* gene in its chromosome. Subsequently, the cell lines were cultivated in CGM media until an OD₆₀₀, harvested by centrifugation, resuspended and disrupted by sonication. Lysates were then assayed for CAT (chloramphenciol acetyltransferase) activity using the assay of Shaw (Shaw WV Methods Enzymol 1975 43: 737-755). The results are shown in Fig. 27 and 28.

These results demonstrated that the *ntnH* promoter and ha33 promoter are relatively inactive in the wild type strain in the absence of BotR. Moreover, the level of BotR-mediated CAT production from the ha33 promoter (pMTL-YZ008) is much greater to than that of the *fdx* promoter (pMTL-YZ003), Fig 28.

### Generation of a Clostridium sporogenes strain producing TcdR

To generate a strain producing functional TcdR protein from a chromosomally located gene, the following steps were undertaken.

A DNA fragment encompassing the structural gene encoding TcdR and its ribosome binding (RBS) site was excised as a NotI-BamHI fragment (SEQ ID NO: 12) from plasmid pMTL-YZ001 (SEQ ID NO: 14. Fig. 2), and inserted between the equivalent sites of the plasmid pMTL-JH29 (SEQ ID NO: 32, Fig. 29) to yield the plasmid pMTL-YZ009 (SEQ ID NO: 33 Fig. 30).

To introduce the *tcdR* gene into the chromosome of *Clostridium sporogenes* NCIMB 10696, the method, Allele-Coupled Exchange (ACE) was employed (Heap JT et al., Nucleic Acids Research, 2012 40(8):e59)**.** In brief, pMTL-YZ009 plasmid DNA was transformed into the ACE-generated *C*. *sporogenes pyrE* mutant described in Heap JT et al. Nucleic Acids Research, 2012 40(8):e59. Transformed cells were plated onto TYG agar (Purdy D et al. Mol. Microbiol 2002 46:439-452) supplemented with 15µg/ml thiamphenicol. After 24 hours, fast growing single colonies were picked and re-streaked twice onto TYG agar containing 15µg/ml thiamphenicol. These cells represented those in which the plasmid had integrated into the genome by single cross-over recombination between the RHA. Thereafter, cells were streaked onto Minimal Medium agar (MM) (Dixon NM et al. J. Appl. Bacteriol 1987 63:171-182) to select for cells able to grow in the absence of exogenous uracil as a consequence of plasmid excision (through recombination between the duplicated LHA) and restoration of a functional *pyrE* allele. The final construct has the *tcdR* gene, together with its RBS, inserted immediately downstream of the *pyrE* gene. The *tcdR* gene is transcribed from the promoter upstream of CS3415 (see Fig. 31). The strain generated was designated CRG3817.

### Demonstration of TcdR functionality in Clostridium sporogenes

To test the functionality of TcdR in the *Clostridium sporogenes* strain CRG3817, plasmid pMTL-YZ002 (Fig. 4 SEQ ID NO: 15), was introduced into the cell in which the expression of a promoter-less copy of a *catP* gene was placed under the transcriptional control of the *tcdB* promoter. For comparative purposes, plasmid pMTL-YZ003 was also constructed identical to pMTL-YZ002, but in which the fragment encompassing the *tcdB* promoter was replaced with an equivalent Notl/Ndel fragment encompassing the *fdx* promoter (Fig. 5). This plasmid was designated pMTL-YZ003 (SEQ ID NO: 16).

The two plasmids pMTL-YZ002 and pMTL-YZ003 were introduced into the wildtype NCIMB 10696 strain of *Clostridium sporogenes* and strain CRG3817 carrying the *tcdR* gene in its chromosome. The four cell lines were then cultivated in TYG media until an OD₆₀₀, cells were harvested by centrifugation, and the resuspended cells disrupted by sonication. Lysates were then assayed for CAT (chloramphenciol acetyltransferase) activity using the assay of Shaw (Shaw WV, Methods Enzymol 1975 43:737-755). The results are shown in Fig 32.

These results demonstrate that even in the absence of TcdR (that is when pMTL-YZ002 is present in wild-type *C*. *sporogenes*), the *tcdB* promoter shows some activity, as evidenced by CAT activity. However, this activity is significantly lower than when the *tcdR* gene is present, ie., when pMTL-YZ002 is present in strain CRG3817. Moreover, the level of TcdR-mediated CAT production from the *tcdB* promoter (pMTL-YZ002) is broadly equivalent to that of the strong *fdx* promoter (pMTL-YZ003).

### TcdR-mediated production of a prodrug converting enzyme (PCE)

To test establish that the TcdR system could be used to express a gene encoding a prodrug-converting enzyme (PCE), pMTL-YZ002 was first modified by swapping its *catP* gene (Fig. 4) with a gene encoding a bacterial nitroreductase. Nitroreductase enzymes are entirely innocuous and ubiquitous to all bacteria (Anlezark GM et al., Microbiology 2002, 148: 297-306). Most bacterial species invariably carry more than one form. They are involved in oxidation-reduction process - metabolic process that results in the removal or addition of one or more electrons to or from a substance, with or without the concomitant removal or addition of a proton or protons. The gene used here was comprised of 222 codons, specifying an enzyme of some 24 kDa in size.

To clone the NTR gene, the gene was amplified by PCR using primers that created an *Nde*I site (CATATG) over the start codon (that is effectively introducing CAT before the ATG start codon), and introduced an *Xho*I site (CTCGAG) immediately after the TAA stop codon. This 678 bp *Nde*I*-Xho*I fragment (Fig. 33) was inserted into plasmid pMTL-YZ002 in place of the *catP* gene, yielding plasmid pMTL-YZ010 (Fig 34). The NTR gene was excised out from plasmid pMTL-ME001 (Fig. 35). A DNA fragment encompassing the structural gene encoding TcdR and its ribosome binding (RBS) site was excised as a *Not*I-*Bam*HI fragment (SEQ ID NO: 12) from plasmid pMTL-YZ001 (SEQ ID NO: 14. Fig. 2), and inserted between the equivalent sites of the plasmid pMTL-JH27 (SEQ ID NO: 34 Fig. 36) to yield the plasmid pMTL-YZ011 (Fig. 37). Then a *Not*I*-Bam*HI fragment containing the native *tcdB* promoter and the NTR gene was restriction digested from plasmid pMTL-YZ010, then cloned into the equivalent sites of plasmid pMTL-YZ011, resulting in the plasmid pMTL-YZ012 (Fig. 38). This plasmid was used to insert the fragment of *tcdR* gene and NTR gene expressed from the *tcdB* promoter into the chromosome using the method, Allele-Coupled Exchange (ACE) (Heap JT et al., Nucleic Acids Research, 2012 40(8):e59).

Plasmid pMTL-YZ012 plasmid DNA was then transformed into the *C*. *sporogenes* NCIMB 10696 strain described in Heap JT et al., Nucleic Acids Research, 2012 40(8):e59. Transformed cells were plated onto TYG agar (Purdy D et al. Mol. Microbiol 2002 46:439-452) supplemented with 15µg/ml thiamphenicol. After 24 hours, fast growing single colonies were picked and re-streaked twice onto TYG agar containing 15µg/ml thiamphenicol. These cells represented those in which the plasmid had integrated into the genome by single cross-over recombination between the RHA. Thereafter, cells were streaked onto Minimal Medium agar (MM) (Dixon NM et al. J. Appl. Bacteriol 1987 63:171-182) supplied with 40µg/ml uracil and 3mg/ml Fluoro-orotic acid (FOA) to select for cells able to grow in the presence of FOA as a consequence of plasmid excision (through recombination between the duplicated LHA) and the disruption of the *pyrE* gene. The final construct has the *tcdR* gene, together with its RBS, inserted immediately downstream of the disrupted *pyrE* gene, then followed by theNTR gene expressed by the *tcdB* promoter. (see Fig. 39) The strain generated was designated CRG3844.

To determine the level of NTR expression in strain CGR3844, WT *C*. *sporogenes* and strain CRG1650 were used as the negative and positive controls. Strain CRG1650 was generated using plasmid pMTL-ME001 (Fig. 35) by the method ACE, which is chromosome integrated NTR gene expressed from the *fdx* promoter, inserted immediately downstream of the disrupted *pyrE* gene (Fig. 40) The three cell lines were then cultivated in TYG media until an OD₆₀₀, cells were harvested by centrifugation, and the resuspended cells disrupted by sonication. Lysates were then assayed for NTR (nitroreductase) activity using the assay of Menadione assay (Knox, R.J., et al. Biochem Pharmacol 1988 37(24):4671-4677.). The results are shown in Fig 41.

These results demonstrate that the level of TcdR-mediated NTR production from the *tcdB* promoter (CGR3844) is broadly equivalent to that of the strong *fdx* promoter (CRG1650), and that the system may be used to express genes encoding PCEs in *C*. *sporogenes..*

### Use of the expression system for expression of the transposase Himarl C9 using suicide plasmid delivery in Clostridium acetobutylicum

By modifying electroporation parameters, we were able to achieve transformation frequencies of 1 to 4*10⁶ transformants per µg DNA. This transformation efficiency is suitable for transposon mutagenesis using the *mariner* transposon-based transposon vector pMTL-GL001, a suicide plasmid, (SEQ ID NO: 36, Fig 42).

Plamid pMTL-SC1 was modified by deleting its Gram-positive replication region (based on the plasmid pBP1). To achieve this, the pBP1 replicon was deleted from the plasmid pMTL-SC1 (Cartman ST and Minton NP, 2010, Applied Environmental Microbiology 2010 76:1103-1109**)** as a 2403bp AscI and FseI fragment, the rest of the plasmid, 5017bp fragment was treated with T4 polymerase and the resultant blunt-ended fragment self-ligated. The plasmid obtained was designated pMTL-GL001 (SEQ ID NO: 36, Figure 42).

To make high transformation efficiency competent cells, *Clostridium acetobutylicum* CRG3011 was inoculated with a heavy loop in 10 ml 2xYTG liquid medium, then serially diluted into 10⁻¹, 10⁻² and 10⁻³ 10 ml cultures overnight. The next day, use all 10ml of the most dilute overnight culture that still shows good growth to inoculate 310ml of 2xYTG. Incubate at 37°C until OD₆₀₀ = 0.2- 0.25 (usually about 3-4 hours). Put 500ml of EPB on ice inside the anaerobic cabinet air locker. Pour 40ml of culture into each of eight 50ml Falcon tubes and centrifuge at 4°C at 4000 x rpm for 10 minutes.Place tubes on ice and carefully transfer into the anaerobic cabinet.Carefully pour off the supernatants and re-suspend each pellet in 20ml EPB by shaking or gentle pipetting. Return to ice, then combine all eight tubes into four 40ml. Place the tubes on ice, then transfer out of the anaerobic cabinet and centrifuge as before. Carefully pour off the supernatants and re-suspend each pellet in 40ml EPB by shaking. Return to ice. Carefully pour off the supernatant and re-suspend each pellet in the rest of EPB by gentle pipetting. Transfer to one tube and return to ice (normally around 1.5 ml at this stage). The cells are now ready to electroporate, and should be kept on ice and used promptly or aliquot the rest of competent cell in glass vials and store in -80°C (adding 10% DMSO before freeze). There are enough cells for 3 transformations. Aliquot the competent cells in glass vials, use immediately or store in -80°C.

Add 20µL of miniprepped methylated plasmid pMTL-GL001 DNA (10 µg) to each chilled 0.4cm gap electroporation cuvette on ice. Label the cuvettes and transfer into the anaerobic cabinet. Gently add 590µL of competent cells to each cuvette, and incubate on ice for 2 minutes. Dry the outside of the cuvette with tissue, and place in the electroporation chamber. Electroporate immediately using 2.0kV, 25µF and ∞Ω. Immediately add 1ml warm anaerobic 2xYTG from a recovery tube to the cuvette and mix gently. Transfer the entire transformation mixture back into the recovery tube and allow at least 4 hrs recovery. Spin the cells at room temperature and discard supernatant. Re-suspend the pellet in 0.5ml 2xYTG and plate transformants onto CGM agar plates containing 15µg/ml thiamphenicol.

All colonies that arise should be transposon mutants and normally there should be around 10³ per transformation. To establish whether transposition had occurred, inverse PCR was performed according to the procedure of Cartman and Minton (Cartman ST and Minton NP Applied Environmental Microbiology 2010, 76:1103-1109**).** Genomic DNA was isolated from 11 individual thiamphenicol resistant clones and digested overnight with Hindlll at a concentration of 200ng/µl. The Hindlll restriction endonuclease was heat inactivated (65°C for 30 min), and DNA was diluted to a concentration of 5 ng/µl in a reaction with T4 DNA ligase to favor self-ligation (and thus circularization) of restriction fragments. The ligation reaction mixtures were incubated at ambient temperature for 1 h, and then the T4 ligase was heat inactivated (65°C for 30 min). Inverse PCRs were carried out in 50µl volumes using the KOD Hot Start DNA polymerase Master Mix kit (Novagen), with 100 ng of ligated DNA and primers catP-INV-F1, SEQ ID NO: 26 (5'-TAAATCATTTTTAGCAGATTATGAAAGTGATACGCAACGGTATGG-3') and catP-INV-R1, SEQ ID NO: 27 (5'-TATTGTATAGCTTGGTATCATCTCATCATATATCCCCAATTCACC-3'), which face out from the transposon-based *catP* sequence. Inverse PCR products were run out on a 0.8% (wt/vol) agarose gel (Fig. 43), purified with the QIAquick gel purification kit (Qiagen), and sequenced using primer catP-INV-R2 SEQ ID NO: 28 (5'-TATTTGTGTGATATCCACTTTAACGGTCATGCTGTAGGTACAAGG-3').

To identify the genomic location of transposon insertions, sequence data were analyzed using GENtle. These data revealed that in all the colonies tested, the transposon had inserted into 11 different locations within the *C*. *acetobutylicum* genome (Table 3). These data provide proof of principle that the presence of *tcdR* in the genome of CRG3011 allows transposition of the *mariner* transposon in *Clostridium acetobutylicum.*

**Table 3. Sequence analysis of the Inverse PCR products from eight randomly selected thiamphenicol resistant colonies carrying pMTL-YZ005**

| **Colony Number** | **Position of Insertion in the C.** | **Gene** | **Gene function** |
|---|---|---|---|
| 1 | reverse 3727867 | CA_C 0187 | nagB |
| 2 | forward 3033632 | CA_C 2899 | LysM repeat-comtainging |
| 3 | forward 549399 | CA_C0475 | HD-GYP domain containing |
| 4 | forward 768217 | N/A | promoter region of CA_C0661 |
| 5 | reverse 1200532 | CA_C 2631 | hypothetical protein |
| 6 | forward 328924 | CA_C 0289 | response regulator |
| 7 | forward 776062 | CA_C0667 | sugar-binding periplasmic |
| 8 | reverse 3896561 | CA_C0033 | ABCI family protein kinase |
| 9 | forward 3926957 | CA_C3720 | hypothetical protein |
| 10 | reverse 1180389 | N/A | promoter region of CA_C2646 |
| 11 | forward 716024 | CA_C0611 | hypothetical protein |

### Demonstration of the expression system for expression of the transposase Himar1 C9 using suicide plasmid delivery in Clostridium beijerinckii

*Clostridium beijerinckii* strain CRG3920 was engineered in a similar manner as described before in *Clostridium acetobutylicum* and *Clostridium sporogenes.* The *tcdR* gene was inserted into the chromosome of *Clostridium beijerinckii* strain 59B, using the method, Allele-Coupled Exchange (ACE) (Heap JT et al., Nucleic Acids Research, 2012 40(8):e59)**.** The resulting strain of *Clostridium beijerinckii* (strain CRG3920) has the *tcdR* gene, together with its RBS, inserted immediately downstream of the *pyrE* gene. The *tcdR* gene is transcribed from the promoter upstream of the *pyrE* operon.

280ul frozen aliquot of *Clostridium beijerinckii* strain CRG3920 was transformed with 20µl pMTL-GL001 plasmid (176.6ng/µl) as described before, followed by a recovery period of at least 4 hrs in 3 ml of 2xYTG media. The cells were spun down, then the supernatant was discarded, cells resuspended in 950µl 2xYTG and 100µl dilutions plated out on CBM + thiamphenicol (15µg/ml) plates. (Colony counts: 10⁻²: 215, 199, 223, 10⁻³: 13, 19, 18, 10⁻⁴: 3, 2, 1.) 100 colonies were picked from 10⁻² plate and stabbed first into CBM + Erythromycin (15µg/ml) plate then into CBM only plate, to confirm plasmid loss. No colonies grew on CBM+Em plate, all grew on CBM only plate, indicating the suicide plasmid indeed cannot replicate within the cell. 24 single colonies were picked from the CBM only plate with cocktail sticks and used to inoculate 1 ml of CBM + thiamphenicol (15µg/ml) broth, 21 out of 24 were grown overnight. Genomic DNA were extracted from these overnight culture and inverse PCR were performed as described before. PCR products were visualised on a 0.8% agarose gel (Fig. 44) and bands cut from the gel, before being sent for sequencing. To identify the genomic location of transposon insertions, sequence data were analyzed using GENtle open source software (http://gentle.magnusmanske.de/).

The data revealed that each transposon insertion had taken place at a different position around the genome as illustrated in Figure 45.

## Claims

1. A bacterial expression system for expressing a nucleic acid in a non-pathogenic bacterial species, the expression system comprising:
a) DNA encoding a group 5 RNA polymerase sigma factor; and
b) an expression cassette comprising a promoter recognised by the group 5 RNA polymerase sigma factor operably linked to a heterologous nucleic acid;
wherein (a) and (b) are located on the same expression vector, separate expression vectors or are integrated into the non-pathogenic bacterial host genome, and
wherein the heterologous nucleic acid is a CAZyme-encoding nucleic acid.

2. A bacterial expression system according to claim 1,
wherein the group 5 RNA polymerase sigma factor is selected from BotR, TetR, TcdR or UviA, and optionally or preferably wherein the group 5 RNA polymerase sigma factor is TcdR; and/or
wherein the group 5 RNA polymerase sigma factor has a sequence identity of at least 60%, 65%, 70%, 75%, 80%, 85%, 90%, 95%, 98%, 99% or is identical to one or more of SEQ ID NO: 1, SEQ ID NO: 2, SEQ ID NO: 3 or SEQ ID NO: 4.

3. A bacterial expression system according to any one of the preceding claims, wherein the promoter recognised by the group 5 RNA polymerase sigma factor is selected from *ntnh, ha33, tetX, tcdA, tcdB* and/or *bcn* promoter; and optionally or preferably wherein the promoter recognised by the group 5 RNA polymerase sigma factor is *tcdA* and/or *tcdB.*

4. A bacterial expression system according to any one of the preceding claims, wherein the CAZyme-encoding nucleic acid encodes CeI48F, CeIA or Xyn10A.

5. A method for expressing a nucleic acid in a non-pathogenic bacterial cell comprising:
(1) introducing a bacterial expression system according to any one of claims 1 to 4 into the bacterial host; wherein the bacterial expression system comprises:
(a) DNA encoding a group 5 RNA polymerase sigma factor; and
(b) an expression cassette comprising a promoter recognised by the group 5 RNA polymerase sigma factor operably linked to a heterologous nucleic acid encoding a CAZyme;
wherein (a) and (b) are located on the same expression vector, separate expression vectors or are integrated into the non-pathogenic bacterial host genome.

6. A method according to claim 5, wherein (a) is integrated into the bacterial genome; or wherein (b) is integrated into the bacterial genome; or wherein (a) and (b) are integrated into the non-pathogenic bacterial genome in a single event or in two separate events.

7. An expression vector which comprises an expression cassette comprising a promoter recognised by the group 5 RNA polymerase sigma factor operably linked to a heterologous nucleic acid encoding a CAZyme.

8. An expression vector according to claim 7, wherein the group 5 RNA polymerase sigma factor is selected from BotR, TetR, TcdR or UviA; and optionally or preferably wherein the group 5 RNA polymerase sigma factor is TcdR.

9. An expression vector according to claim 8, wherein the group 5 RNA polymerase sigma factor has a sequence identity of at least 60%, 65%, 70%, 75%, 80%, 85%, 90%, 95%, 98%, 99% or is identical to one or more of SEQ ID NO: 1, SEQ ID NO: 2, SEQ ID NO: 3 or SEQ ID NO: 4.

10. An expression vector according to any one of claims 7 to 9, wherein the promoter recognised by the group 5 RNA polymerase sigma factor is selected from *ntnh, ha33, tetX, tcdA, tcdB* and/or *bcn;* and optionally or preferably wherein the promoter recognised by the group 5 RNA polymerase sigma factor is tcdA and/or tcdB.

11. A non-pathogenic bacterial cell transformed with the expression system of any one of claims 1 to 4 or the expression vector of claims 7 to 10; and optionally or preferably wherein the non-pathogenic bacterial cell is *C*. *acetobutylicum,* , *C. beijerinckii, C. Ijungdahlii, C. kluyveri,* , *C*. *autoethanogenum, C.pasteurianum, C. saccharobutylicum, C. carboxidovorans, C. sporogenes, C. phytofermentans, C. ragsdalei, C. tyrobutyricum,* , *C. butyricum, C. cellulolyticum, C. formicaceticum, C. novyi, C. scatologenes, C. septicum, C. sordellii,, C. sticklandii,* , *C*. *thermocellum, C. thermosaccharolyticum, C.paprosolvens, C. scindens,* or *C*. *bifermentans.*

## Patentansprüche

1. Bakterielles Expressionssystem zum Exprimieren einer Nukleinsäure in einer nicht-pathogenen Bakterienart, wobei das Expressionssystem Folgendes umfasst:
(a) DNA, die einen Gruppe-5-RNA-Polymerase-Sigma-Faktor codiert; und
(b) eine Expressionskassette, die einen Promotor umfasst, der von dem Gruppe-5-RNA-Polymerase-Sigma-Faktor, der funktionell mit einer heterologen Nukleinsäure verbunden ist, erkannt wird;
wobei sich (a) und (b) auf demselben Expressionsvektor befinden, sich auf getrennten Expressionsvektoren befinden oder in das Genom des nicht-pathogenen Bakterienwirts integriert sind und
wobei es sich bei der heterologen Nukleinsäure um eine CAZyme codierende Nukleinsäure handelt.

2. Bakterielles Expressionssystem nach Anspruch 1,
wobei der Gruppe-5-RNA-Polymerase-Sigma-Faktor aus BotR, TetR, TcdR oder UviA ausgewählt ist und wobei es sich bei dem Gruppe-5-RNA-Polymerase-Sigma-Faktor wahlweise oder vorzugsweise um TcdR handelt;
und/oder
wobei der Gruppe-5-RNA-Polymerase-Sigma-Faktor eine Sequenzidentität von mindestens 60 %, 65 %, 70 %, 75 %, 80 %, 85 %, 90 %, 95 %, 98 %, 99 % mit einer oder mehreren von SEQ ID Nr. 1, SEQ ID Nr. 2, SEQ ID Nr. 3 oder SEQ ID Nr. 4 aufweist oder mit einer oder mehreren davon identisch ist.

3. Bakterielles Expressionssystem nach einem der vorhergehenden Ansprüche, wobei der von dem Gruppe-5-RNA-Polymerase-Sigma-Faktor erkannte Promotor aus dem *ntnh-, ha33-, tetX-, tcdA-, tcdB-* und/oder *bcn*-Promotor ausgewählt ist; und wobei es sich bei dem von dem Gruppe-5-RNA-Polymerase-Sigma-Faktor erkannten Promotor wahlweise oder vorzugsweise um *tedA* und/oder *tedB* handelt.

4. Bakterielles Expressionssystem nach einem der vorhergehenden Ansprüche, wobei die CAZyme codierende Nukleinsäure CeI48F, CeIA oder Xyn10A codiert.

5. Verfahren zum Exprimieren einer Nukleinsäure in einer nicht-pathogenen Bakterienzelle, umfassend:
(1) Einführen eines bakteriellen Expressionssystems nach einem der Ansprüche 1 bis 4 in den bakteriellen Wirt; wobei das bakterielle Expressionssystem Folgendes umfasst:
(a) DNA, die einen Gruppe-5-RNA-Polymerase-Sigma-Faktor 5 codiert; und
(b) eine Expressionskassette, die einen Promotor umfasst, der von dem Gruppe-5-RNA-Polymerase-Sigma-Faktor erkannt wird, der funktionell mit einer ein CAZyme codierenden, heterologen Nukleinsäure verbunden ist;
wobei sich (a) und (b) auf demselben Expressionsvektor befinden, sich auf getrennten Expressionsvektoren befinden oder in das Genom des nicht-pathogenen Bakterienwirts integriert sind.

6. Verfahren nach Anspruch 5, wobei (a) in das bakterielle Genom integriert ist oder wobei (b) in das bakterielle Genom integriert ist oder wobei (a) und (b) in einem einzigen Ereignis oder in zwei getrennten Ereignissen in das bakterielle Genom integriert sind.

7. Expressionsvektor, der eine Expressionskassette umfasst, die einen Promotor umfasst, der von dem Gruppe-5-RNA-Polymerase-Sigma-Faktor erkannt wird, der funktionell mit einer ein CAZyme codierenden, heterologen Nukleinsäure verbunden ist.

8. Expressionsvektor nach Anspruch 7, wobei der Gruppe-5-RNA-Polymerase-Sigma-Faktor aus BotR, TetR, TcdR oder UviA ausgewählt ist und wobei es sich bei dem Gruppe-5-RNA-Polymerase-Sigma-Faktor wahlweise oder vorzugsweise um TcdR handelt.

9. Expressionsvektor nach Anspruch 8, wobei der Gruppe-5-RNA-Polymerase-Sigma-Faktor eine Sequenzidentität von mindestens 60 %, 65 %, 70 %, 75 %, 80 %, 85 %, 90 %, 95 %, 98 %, 99 % mit einer oder mehreren von SEQ ID Nr. 1, SEQ ID Nr. 2, SEQ ID Nr. 3 oder SEQ ID Nr. 4 aufweist oder mit einer oder mehreren davon identisch ist.

10. Expressionsvektor nach einem der Ansprüche 7 bis 9, wobei der von dem Gruppe-5-RNA-Polymerase-Sigma-Faktor erkannte Promotor aus *ntnh, ha33, tetX, tcdA, tcdB* und/oder *bcn* ausgewählt ist; und wobei es sich bei dem von dem Gruppe-5-RNA-Polymerase-Sigma-Faktor erkannten Promotor wahlweise oder vorzugsweise um *tcdA* und/oder *tcdB* handelt.

11. Nicht-pathogene Bakterienzelle, die mit dem Expressionssystem nach einem der Ansprüche 1 bis 4 oder dem Expressionsvektor nach Anspruch 7 bis 10 transformiert ist, und wobei es sich bei der nicht-pathogenen Bakterienzelle wahlweise oder vorzugsweise um *C*. *acetobutylicum, C. beijerinckii, C. Ijungdahlii, C. kluyveri, C. autoethanogenum, C. pasteurianum, C. saccharobutylicum, C. carboxidovorans, C. sporogenes, C*. *phytofermentans, C. ragsdalei, C. tyrobutyricum, C. butyricum, C. cellulolyticum, C*. *formicaceticum, C. novyi, C. scatologenes, C. septicum, C. sordellii, C. sticklandii, C*. *thermocellum, C. thermosaccharolyticum, C. paprosolvens, C. scindens* oder *C*. *bifermentans* handelt.

## Revendications

1. Système bactérien d'expression servant à exprimer un acide nucléique dans une espèce bactérienne non pathogène, le système d'expression comprenant :
a) un ADN codant un facteur sigma d'ARN polymérase de groupe 5 ; et
b) une cassette d'expression comprenant un promoteur reconnu par le facteur sigma d'ARN polymérase de groupe 5 lié de manière opérationnelle à un acide nucléique hétérologue ;
dans lequel (a) et (b) sont situés sur le même vecteur d'expression, sur des vecteurs d'expression séparés ou sont intégrés au génome hôte bactérien non pathogène, et
dans lequel l'acide nucléique hétérologue est un acide nucléique codant une CAZyme.

2. Système bactérien d'expression selon la revendication 1,
dans lequel le facteur sigma d'ARN polymérase de groupe 5 est sélectionné parmi BotR, TetR, TcdR ou UviA, et facultativement ou de préférence dans lequel le facteur sigma d'ARN polymérase de groupe 5 est TcdR ; et/ou
dans lequel le facteur sigma d'ARN polymérase de groupe 5 a une identité de séquence d'au moins 60 %, 65 %, 70 %, 75 %, 80 %, 85 %, 90 %, 95 %, 98 %, 99 % ou est identique à l'une ou plusieurs de SEQ ID NO : 1, SEQ ID NO : 2, SEQ ID NO : 3 ou SEQ ID NO : 4.

3. Système bactérien d'expression selon l'une quelconque des revendications précédentes, dans lequel le promoteur reconnu par le facteur sigma d'ARN polymérase de groupe 5 est sélectionné parmi le promoteur *ntnh, ha33, tetX, tcdA, tcdB* et/ou *bcn* ; et facultativement ou de préférence dans lequel le promoteur reconnu par le facteur sigma d'ARN polymérase de groupe 5 est *tcdA* et/ou *tcdB.*

4. Système bactérien d'expression selon l'une quelconque des revendications précédentes, dans lequel l'acide nucléique codant une CAZyme code CeI48F, CelA ou Xyn10A.

5. Procédé d'expression d'un acide nucléique dans une cellule bactérienne non pathogène comprenant :
(1) l'introduction d'un système bactérien d'expression selon l'une quelconque des revendications 1 à 4 dans l'hôte bactérien ; dans lequel le système bactérien d'expression comprend :
a) un ADN codant un facteur sigma d'ARN polymérase de groupe 5 ; et
b) une cassette d'expression comprenant un promoteur reconnu par le facteur sigma d'ARN polymérase de groupe 5 lié de manière opérationnelle à un acide nucléique hétérologue codant une CAZyme ;
dans lequel (a) et (b) sont situés sur le même vecteur d'expression, sur des vecteurs d'expression séparés ou sont intégrés au génome hôte bactérien non pathogène.

6. Procédé selon la revendication 5, dans lequel (a) est intégré au génome bactérien ; ou dans lequel (b) est intégré au génome bactérien ; ou dans lequel (a) et (b) sont intégrés au génome bactérien non pathogène en un seul événement ou en deux événements séparés.

7. Vecteur d'expression qui comprend une cassette d'expression comprenant un promoteur reconnu par le facteur sigma d'ARN polymérase de groupe 5 lié de manière opérationnelle à un acide nucléique hétérologue codant une CAZyme.

8. Vecteur d'expression selon la revendication 7, dans lequel le facteur sigma d'ARN polymérase de groupe 5 est sélectionné parmi BotR, TetR, TcdR ou UviA; et facultativement ou de préférence dans lequel le facteur sigma d'ARN polymérase de groupe 5 est TcdR.

9. Vecteur d'expression selon la revendication 8, dans lequel le facteur sigma d'ARN polymérase de groupe 5 a une identité de séquence d'au moins 60 %, 65 %, 70 %, 75 %, 80 %, 85 %, 90 %, 95 %, 98 %, 99 % ou est identique à l'une ou plusieurs de SEQ ID NO : 1, SEQ ID NO : 2, SEQ ID NO : 3 ou SEQ ID NO : 4.

10. Vecteur d'expression selon l'une quelconque des revendications 7 à 9, dans lequel le promoteur reconnu par le facteur sigma d'ARN polymérase de groupe 5 est sélectionné parmi *ntnh, ha33, tetX, tcdA, tcdB* et/ou *bcn ;* et facultativement ou de préférence dans lequel le promoteur reconnu par le facteur sigma d'ARN polymérase de groupe 5 est *tcdA* et/ou *tcdB.*

11. Cellule bactérienne non pathogène transformée avec le système d'expression selon l'une quelconque des revendications 1 à 4 ou le vecteur d'expression selon les revendications 7 à 10 ; et facultativement ou de préférence dans laquelle la cellule bactérienne non pathogène est *C*. *acetobutylicum, C. beijerinckii, C. Ijungdahlii, C. kluyveri, C. autoethanogenum, C. pasteurianum, C. saccharobutylicum, C. carboxidovorans, C. sporogenes, C. phytofermentans, C. ragsdalei, C. tyrobutyricum, C. butyricum, C. cellulolyticum, C. formicaceticum, C. novyi, C. scatologenes, C. septicum, C. sordellii, C. sticklandii, C. thermocellum, C. thermosaccharolyticum, C. paprosolvens, C. scindens, ou C. bifermentans.*
